# EUROPÄISCHE PATENTSCHRIFT

(11) **EP 2 079 705 B1**
(45) Veröffentlichungstag und Bekanntmachung des Hinweises auf die Patenterteilung: **15.09.2010**
(21) Anmeldenummer: 07821046.5
(22) Anmeldetag: 09.10.2007
(51) Int. Cl.: C07D 233/54

(54) **VERFAHREN ZUR HERSTELLUNG QUARTERNÄRER CARBONATE**
METHOD FOR PRODUCING QUATERNARY CARBONATES
PROCÉDÉ DE PRODUCTION DE CARBONATES QUATERNAIRES

(30) Priorität: 10.10.2006 AT 16882006
(43) Veröffentlichungstag der Anmeldung: 22.07.2009
(73) Patentinhaber: proionic Production of Ionic Substances GmbH & Co KG, 8074 Grambach (AT)
(72) Erfinder: KALB, Roland, A-8700 Leoben (AT)
(74) Vertreter: Vinazzer, Edith
(86) Internationale Anmeldenummer: PCT/EP2007/060674
(87) Internationale Veröffentlichungsnummer: WO 2008/052860

(56) Entgegenhaltungen:
- EP-A- 0 291 074
- EP-A- 0 668 271
- WO-A-2005/021484
- DE-A1- 19 836 477
- DE-A1- 19 919 494
- US-A- 5 854 360
- US-A- 5 856 513
- ZHI-BIN ZHOU, MASAYUKI TAKEDA, MAKOTO UE: "New hydrophobic ionic liquids based on perfluoroalkyltrifluoroborate anions" JOURNAL OF FLOURINE CHEMISTRY, Bd. 125, 2004, Seiten 471-476, XP002473943
- SIMA T ET AL: "The syntheses of carbamates from reactions of primary and secondary aliphatic amines with dimethyl carbonate in ionic liquids" TETRAHEDRON LETTERS, ELSEVIER, AMSTERDAM, NL, Bd. 43, Nr. 45, 4. November 2002 (2002-11-04), Seiten 8145-8147, XP004387209 ISSN: 0040-4039

## Beschreibung

Die Erfindung betrifft ein Verfahren zur Herstellung von quaternären Ammonium, Phosphonium, Sulfonium, und Heteroarylium Alkylcarbonaten und Arylcarbonaten.

Ein Verfahren zur Herstellung quaternärer Salze ist aus der EP-A-0 291 074 bekannt. In einem ersten Verfahrensschritt wird durch Reaktion eines Phosphines mit einem Carbonsäurediester das korrespondierende quaternäre Carbonat hergestellt. In einem zweiten Verfahrensschritt wird das gebildete quaternäre Carbonat mit einer organischen Säure gemischt, während das erzeugte Kohlendioxid aus dem System entfernt wird, um das Carbonat in das korrespondierende Säuresalz durch Anionenaustausch umzuwandeln.

Ionische Verbindungen - Verbindungen, welche ionische Flüssigkeiten oder ionische Feststoffe oder Gemische derselben sind - weisen äußerst interessante Eigenschaften auf, wie beispielsweise das Fehlen eines (meßbaren) Dampfdrucks (mit wenigen Ausnahmen: siehe Martyn J. Earle, José M.S.S. Esperanc, Manuela A. Gilea1, José N. Canongia Lopes, Luis P.N. Rebelo, Joseph W. Magee, Kenneth R. Seddon & Jason A. Widegren, Nature 2006, Vol.439, 831-834), einen sehr großen Liquidusbereich, gute elektrische Leitfähigkeit und ungewöhnliche Solvatations-Eigenschaften. Diese Eigenschaften prädestinieren sie für den Einsatz in verschiedenen Bereichen technischer Anwendungen. So können sie beispielsweise als Lösungsmittel (bei organischer und anorganischer Synthese im Allgemeinen, bei der Übergangsmetallkatalyse, der Biokatalyse, der PhasentransferKatalyse, bei Mehrphasen-Reaktionen, in der Photochemie, in der Polymersynthese und der Nanotechnologie), als Extraktionsmittel (bei der flüssig-flüssig- und der flüssig-gasförmig Extraktion im Allgemeinen, der Entschwefelung von Rohöl, der Entfernung von Schwermetallen aus Abwässern, der Flüssigmembranextraktion), als Elektrolyte (in Batterien, Brennstoffzellen, Kondensatoren, Solarzellen, Sensoren, in der Elektrochromie, der Galvanotechnik, in der elektrochemischen Metallbearbeitung, in der elektrochemischen Synthese im Allgemeinen, bei der elektroorganischen Synthese, der Nanotechnologie), als Schmierstoffe, als Thermofluide, als Gele, als Reagenzien zur organischen Synthese, in der "Green Chemistry" (Ersatz für Volatile Organic Compounds), als Antistatika, in Spezialanwendungen der Analytik (Gaschromatographie, Massenspektroskopie, Kapillarzonenelektrophorese), als Flüssigkristalle, etc. eingesetzt werden. Diesbezüglich wird beispielsweise auf "Rogers, Robin D.; Seddon, Kenneth R. (Eds.); Ionic Liquids - Industrial Applications to Green Chemistry, ACS Symposium Series 818, 2002; ISBN 0841237891" und "Wasserscheid, Peter; Welton, Tom (Eds.); Ionic Liquids in Synthesis, Verlag Wiley-VCH 2003; ISBN 3527305157" verwiesen.

Die Optimierung der Eigenschaften für die jeweilige Anwendung kann in weiten Grenzen durch eine Variation der Struktur von Anion und Kation bzw. eine Variation ihrer Kombination erfolgen, was den ionischen Flüssigkeiten die Bezeichnung "Designer Solvents" (siehe beispielsweise Freemantle, M.; Chem. Eng. News, 78, 2000, 37) eingebracht hat. In diesem Sinne wurden viele unsystematische Einzelsynthesen für die Herstellung ionischer Verbindungen im Labor- und Technikumsmaßstab entwickelt. Dementsprechend sind die Kosten der Produktion dieser Verbindungen extrem hoch und die Umsetzbarkeit im Industriemaßstab kaum realisierbar.

Ausgangsverbindungen der Synthesen solcher ionischer Verbindungen sind im Allgemeinen alkyl- oder arylsubstituierte organische Stickstoff-, Phosphor- oder Schwefelnucleophile (Amine, Phosphine, Sulfide, Heteroaromaten). Die derzeit bekannten Synthesemethoden werden am Beispiel der Synthese quaternärer Ammoniumverbindungen (siehe nachstehende Abbildung 1) hier kurz erläutert:

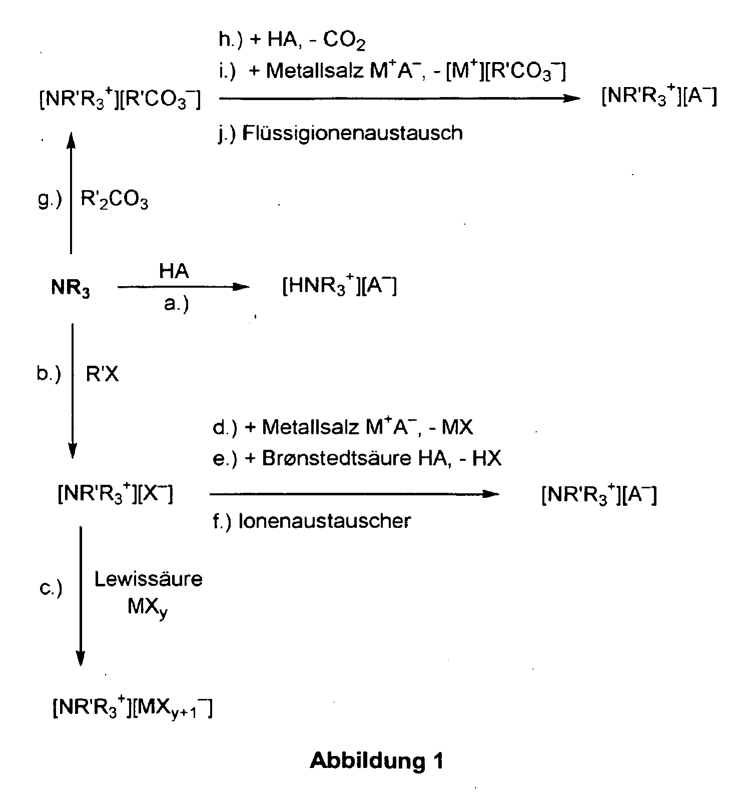

### a. Bronsted-Säure/Basenreaktion

Ein Amin NR₃ reagiert mit einer Protonensäure HA unter Bildung des Salzes [HNR₃⁺][A⁻]. Diese Darstellung ionischer Flüssigkeiten ist die älteste bekannte (Walden, P., Bull. Acad. Imper. Sci. (St. Petersburg), 1914, 1800) und führt beispielsweise bei der Reaktion von Ethylamin mit Salpetersäure zur Bildung von Ethylammoniumnitrat mit einem Schmelzpunkt von Fₚ = 13°C. In diesem Fall ist das Kation nicht quaterniert sondern nur protoniert. Diese organischen Salze kommen für typische Anwendungsgebiete ionischer Flüssigkeiten meist nicht in Frage, weil sie thermisch und chemisch instabil sind.

### b. Alkylierung bzw. Arylierung zu einem quaternären Salz

Das Amin NR₃ wird mit einem Alkylierungs- oder Arylierungsreagens R'X in einer nucleophilen Substitution unter Bildung eines quaternierten Ammoniumsalzes [NR'R₃⁺][X⁻] umgesetzt (Wilkes, J.S.; Zaworotko, M.J., J. Chem. Soc., Chem. Commun., 13, 1992, 965). Als typische Alkylierungsmittel kommen dabei Halogenalkane wie z.B. 1-Chlorbutan, 1-Bromethan, Methyliodid oder Dialkylsulfate wie beispielsweise Dimethylsulfat oder Diethylsulfat zur Anwendung. Diese Alkylierungsmittel sind zwar reaktiv und bilden rasch das gewünschte Produkt, sie sind aber wie alle starken Alkylierungsreagenzien relativ toxisch, zum Teil cancerogen und im Falle der Halogenalkane ggf. atmosphärenschädigend. Die gebildete ionische Verbindung [NR'R₃⁺][X⁻] kann wiederum selbst Ausgangsmaterial für weitere Umsetzungen gemäß den Reaktionen c - f sein.

### c. Reaktion mit einer Lewis-Säure

Zur Darstellung von ionischen Flüssigkeiten auf der Basis von Halogenmetallat-Anionen (siehe z.B. Wilkes, J.S.; Levisky, J.A.; Wilson, R.A.; Hussey, C.L., Inorg. Chem. 1982, 21, 1263) kann das quaternäre Ammoniumsalz [NR'R₃⁺][X⁻] direkt mit einer entsprechenden Lewis-Säure MX_{y} zur gewünschten Verbindung [NR'R₃⁺][MX_{y+1}⁻ ] umgesetzt werden. Im Falle des Tetrachloroaluminat-Anions [AlCl₄]⁻ wird z.B. ein entsprechendes Ammoniumchlorid [NR'R₃⁺][Cl⁻] direkt mit Aluminiumchlorid AlCl₃ versetzt.

Auf Halogenmetallat basierende ionische Flüssigkeiten sind extrem wasser- und sauerstoff-empfindlich, sehr korrosiv und werden nur für ganz spezielle Zwecke eingesetzt (beispielsweise Olefin-Oligomerisierung, Friedel-Crafts Reaktionen). Bereits geringste Spuren Wasser führen zur Hydrolyse unter Bildung von HCl, HBr oder HI. Ihre Synthese gestaltet sich dementsprechend schwierig, nicht zuletzt auch aufgrund der Tatsache, dass das Ausgangsmaterial [NR'R₃⁺][X⁻] stark hygroskopisch ist.

### d. Anionenaustausch via Metathese

Hierbei wird das Anion eines quaternären Ammoniumsalzes, das beispielsweise durch Alkylierung bzw. Arylierung gebildete quaternäre Ammoniumsalz [NR'R₃⁺]X⁻] wird gegen das gewünschte Anion [A⁻] ausgetauscht. Hierzu wird das Salz [NR'R₃⁺][X⁻] in einem trockenen, organischen Lösungsmittel (z.B. Aceton, Acetonitril, Dichlormethan) mit einer stöchiometrischen Menge eines Metallsalzes (meist ein entsprechendes Alkali- oder Erdalkalisalz) [M⁺][A⁻] versetzt, welches das gewünschte Anion [A⁻] aufweist.

Der Reaktionsansatz wird unter Ausschluß von Luftfeuchtigkeit typischerweise für einige Tage bis Wochen kräftig gerührt, wobei die gewünschte ionische Flüssigkeit in dem gewählten Lösungsmittel löslich ist und das Anion [X⁻] als unlösliches, festes Metallsalz [M⁺][X⁻] ausfällt und durch Filtration entfernt wird. Neben der sehr langen Reaktionszeit und den großen Mengen von Abfall [M⁺][X⁻] ist bei diesem Verfahren von Nachteil, dass man trockene Lösungsmittel verwenden muss, um einen kompletten Umsatz zu erzielen; ansonsten löst sich das zu entfernende Salz [M⁺][X⁻] in größeren Mengen im Lösungsmittel, wobei diese Halogenidspuren den späteren Einsatz der ionischen Flüssigkeit limitieren (Halogenide sind beispielsweise Katalysatorgifte; Entsorgung durch Verbrennen ist problematisch).

Das wasserfreie Arbeiten ist insbesondere im Industriemaßstab problematisch, da das Edukt [NR'R₃⁺][X⁻] im Allgemeinen sehr hygroskopisch ist und der Produktionsprozess daher unter Ausschluss jeglicher Luftfeuchtigkeit erfolgen muss. Die genannten Nachteile könnten zwar durch den Einsatz von wässrigen Lösungsmittelsystemen und Silbersalzen [Ag⁺][A⁻] behoben werden (wobei die auch in Wasser sehr schwerlöslichen Silberhalogenide ausfallen), dies ist allerdings im Industriemaßstab aus ökonomischen Gründen völlig ausgeschlossen.

Ein kürzlich zum Patent angemeldetes einstufiges Produktionsverfahren (Wasserscheid, Peter; Hilgers, Claus; Boesmann, Andreas; EP1182197 A1 (Solvent Innovation GmbH, Germany), 2002), welches die Reaktionen b und d vereint, kann zwar das Problem des Ausschlusses von Luftfeuchtigkeit zum Teil entschärfen (keine Isolation der hygroskopischen Zwischenstufe [NR'R₃⁺][X⁻] notwendig), allerdings bleiben die Reaktionszeiten nach wie vor äußerst lang, es fällt weiterhin Abfall an und die potentielle Gefahr der Verunreinigung des Endproduktes mit Halogenid-Ionen ist allgegenwärtig; darüber hinaus lassen sich Kondensationsprodukte und Verunreinigungen, die bei der Darstellung der hier nicht isolierten Zwischenstufe entstehen, nur nachträglich unter erheblich größerem Aufwand aus dem fertigen Produkt entfernen.

Allen Anionenaustauschverfahren via Metathese ist aber insbesondere gemeinsam, dass sie nicht allgemein anwendbar sind und nur für ganz bestimmte Kombinationen aus Kationen und Anionen zufriedenstellend funktionieren. Nur wenn die gewünschte ionische Flüssigkeit im Lösungsmittel ausreichend löslich ist (kann oft problematisch sein, zum Teil große Mengen Lösungsmittel nötig) und/oder das eingesetzte Metallsalz [M⁺][A⁻] im Lösungsmittel wesentlich besser löslich ist als das zu entfernende, kann mit Erfolg gerechnet werden. Des weiteren kann das ausgefällte Salz [M⁺][X⁻] zum Teil in der ionischen Flüssigkeit selbst löslich sein.

### e. Anionenaustausch via Bronsted-Säure

Eine weitere Variante des Anionenaustausches stellt die Reaktion von [NR'R₃⁺][X⁻] mit einer Brønstedsäure HA dar. In diesem Fall muss die zugehörige freie Säure des gewünschten Anions stärker sein als die entsprechende Säure HX, sodass auch diese Reaktion nur für wenige Anionen [A⁻] in Frage kommt. Ist HX - wie im Falle der Halogenwasserstoffsäuren - eine flüchtige Säure, kann diese verhältnismäßig leicht im Vakuum entfernt werden (ionische Flüssigkeiten besitzen im Allgemeinen keinen messbaren Dampfdruck); ist die ionische Flüssigkeit hydrophob, kann die Säure HX mit Wasser extraktiv entfernt werden. In allen anderen Fällen gestaltet sich die Isolation des Produktes schwierig.

### f. Anionenaustausch an einem Ionentauscherharz

Der Austausch des Ions [X⁻] gegen das gewünschte Ion [A⁻] kann auch in einem allgemein bekannten Verfahren an einem herkömmlichen Anionentauscherharz erfolgen; aus ökonomischer Sicht ist dieses Verfahren im Industriemaßstab aber kaum anwendbar, da die maximale Beladung eines Harzes sehr beschränkt ist.

### g. Alkylierung bzw. Arylierung zu einem quaternären Carbonat

Hier wird nun das Amin NR₃ mit dem Alkylierungs- oder Arylierungsmittel Dialkyl- oder Diarylcarbonat R'₂CO₃ umgesetzt, wobei ein quaternäres Alkyl- oder Arylcarbonat [NR'R₃⁺][R'CO₃⁻] gebildet wird. Dieses kann selbst bereits die gewünschte ionische Verbindung sein oder als Edukt zur Synthese der Verbindung [NR'R₃⁺][A⁻] dienen, wie in den nun folgenden Punkten h - j beschrieben. Weiterhin sind aufgrund des Herstellungsprozesses die so erhaltenen Verbindungen frei von (korrosiven) Halogeniden.

### h. Anionenaustausch durch Reaktion mit einer Brønsted-Säure

Das gewünschte Anion [A⁻] wird nun bevorzugt durch Reaktion des quaternären Carbonates [NR'R₃⁺][R'CO₃⁻] mit der zum Anion [A⁻] konjugierten Brønsted-Säure HA eingeführt, wobei Kohlendioxid freigesetzt wird. Diese Reaktion ist äußerst vielseitig, schnell, einfach und effizient und erzeugt keinen festen Abfall.

### i. Anionenaustausch via Metathese

Alternativ zu der bevorzugten Reaktion mit einer Brønsted-Säure HA, kann auch eine Metathese analog zu d. durchgeführt werden, wobei nun das quaternäre Carbonat [NR'R₃⁺][R'CO₃⁻] mit einem Metallsalz M⁺A⁻ umgesetzt wird, wobei das Kation des Metallsalzes M⁺ mit dem Carbonat [R'CO₃⁻] schwerlösliches [M⁺][R'CO₃⁻] bildet, welches ausfällt und entfernt wird, sodaß die gewünschte ionische Verbindung [NR'R₃⁺][A⁻] zurückbleibt und isoliert werden kann. Typische Metalle M⁺ sind z.B. Calcium, Magnesium, Zink, Mangan etc. Diese Reaktion verläuft nahezu quantitativ in wäßriger Lösung und benötigt im Gegensatz zu d. kein Lösungsmittel.

### j. Anionenaustausch mittels Flüssigionenaustausch

Eine weitere Möglichkeit besteht in der Reaktion eines langkettigen Carbonats [NR'R₃⁺][R'CO₃⁻], welches im Allgemeinen wegen der hohen Polarität des Anions [R'CO₃⁻] wasserlöslich ist, mit einem Metallsalz M⁺A⁻, wobei hier das Kation M⁺ keine schwerlöslichen Carbonate bildet; es kommt zu einer Ionenaustauschreaktion und wasserunlösliches [NR'R₃⁺][A⁻] scheidet sich ab, während [M+][R'CO₃⁻] in Lösung bleibt. Typischerweise gelingt diese Reaktion mit einer Vielzahl von Anionen A⁻, welche in der Regel immer wesentlich unpolarer sind als das Anion [R'CO₃⁻], sodaß also das Ionenpaar [NR'R₃⁺][A⁻] nicht mehr wassermischbar ist und sich abscheidet.

Die beschriebenen Verfahren zur Herstellung Ionischer Verbindungen, also ionischer Flüssigkeiten oder ionischer Feststoffe, weisen alle ihre typischen Nachteile auf.

Das Verfahren über quaternäre Alkyl- bzw. Arylcarbonate ist im Allgemeinen für eine breite Palette von quaternären Kationen geeignet (unter anderem Ammonium, Phosphonium, Sulfonium, Imidazolium, Piperidinium, Pyrrolidinium, Morpholinium usw.), allerdings nimmt die Reaktionsgeschwindigkeit der Alkylierung bzw. Arylierung von Aminen, Phosphinen, Sulfiden und Heteroarylen mit Alkyl- und/oder Arylcarbonaten mit wachsender Kohlenstoffzahl der Alkyl- bzw. Arylreste dramatisch ab, bzw. die Ausbeute der gewünschten Produkte.

Der Erfindung liegt also die Aufgabe zugrunde, ein Verfahren zur Herstellung quaternärer Carbonate zur Verfügung zu stellen, welches die beschriebenen Nachteile nicht aufweist.

Gelöst wird die gestellte Aufgabe dadurch, daß ein quaternäres Carbonat der Formel I

[A]⁺ [R^{2'}OCO₂]⁻ (I),

in der
[A]⁺ für ein quaternäres Ammonium-Kation [R^{1'}R¹R²R³N]⁺, ein Phosphonium-Kation [R^{1'}R¹R²R³P]⁺, ein Sulfonium-Kation [R^{1'}R¹R²S]⁺ oder ein heteroaromatisches Kation steht,
R¹, R², R³ unabhängig voneinander für Wasserstoff, ggf. sub. Alkyl, Alkenyl, Alkinyl, Cycloalkyl, Cycloalkenyl, Aryl oder Heteroaryl stehen; oder
zwei der Reste R¹, R², R³ bilden gemeinsam mit dem Heteroatom, an welchem sie gebunden sind einen Ring aus, wobei dieser gesättigt oder ungesättigt, unsubstituiert oder substituiert ist und wobei diese Kette durch ein oder mehrere Heteroatome ausgewählt aus der Gruppe O, S, NH oder N-C₁-C₄-Alkyl unterbrochen sein kann;
R^{1'}, R^{2'} unabhängig voneinander für ggf. sub. Alkyl, Alkenyl, Alkinyl, Cycloalkyl, Cycloalkenyl, Aryl oder Heteroaryl stehen; oder
R^{1'} und R²' bilden gemeinsam eine -CHR^{a}-CHR^{b}-Kette, -CHR^{a}-CH₂-CHR^{b}-Kette oder ggf. substuierten -C₆H₄-1,2-diyl-Rest stehen, wobei R^{a} und R^{b} unabhängig voneinander für Wasserstoff oder C₁-C₆-Alkyl; wobei in diesem Fall eine zwitterionische Verbindung der Formel I erhalten wird, bei der R^{1'} und [R^{2'}OCO₂]⁻ den Rest -OCO-CHR^{a}-CHR^{b}-, -OCO-CHR^{a}-CH₂-CHR^{b}- oder ggf. sub. -OCO-1-C₆H₄-2-yl- ausbilden (Verbindung I');
hergestellt wird, indem man ein Amin R¹R²R³N der Formel IIa , Phosphin R¹R²R³P der Formel IIb, Sulfid R¹R²S der Formel IIc oder ein Heteroaromat der Formel IId, wobei die Reste R¹, R² und R³ die oben genannte Bedeutung haben,
mit einem Alkyl- und/oder Arylcarbonat der Formel III

R^{1'}O(CO)OR^{2'} (III), wobei

R^{1'}, R²' unabhängig voneinander für ein ggf. sub. Alkyl, Alkenyl, Alkinyl, Cycloalkyl, Cycloalkenyl, Aryl oder Heteroaryl stehen; oder
R^{1'} und R²' gemeinsam eine -CHR^{a}-CHR^{b}-Kette, -CHR^{a}-CH₂-CHR^{b}-Kette oder ggf. substuierten -C₆H₄-1,2-diyl-Rest bilden, wobei R^{a} und R^{b} unabhängig voneinander für Wasserstoff oder C₁-C₆-Alkyl stehen; wobei in diesem Fall eine zwitterionische Verbindung der Formel I' erhalten wird;

in Gegenwart katalytischer Mengen an einer Lewis-Säure umsetzt.

Der Heteroaromat der Formel IIb ist üblicherweise ein 5- oder 6-gliedriger Heteroaromat, der mindestens ein Stickstoffatom sowie gegebenenfalls ein Sauerstoff- oder Schwefelatom aufweist, und der unsubstituiert oder substituiert ist und/oder anneliert ist, vorzugsweise ist der Heteroaromat der Formel IIb ausgewählt aus der Grupppe: 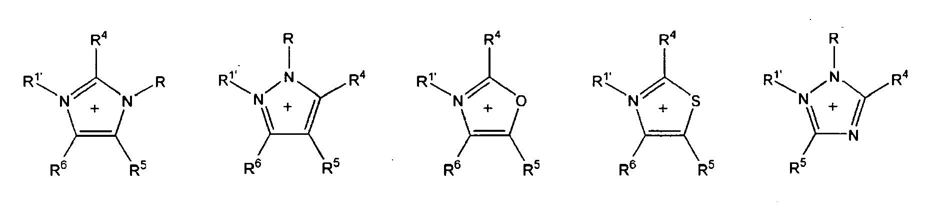 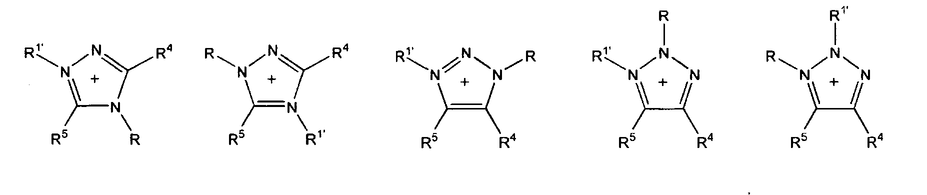 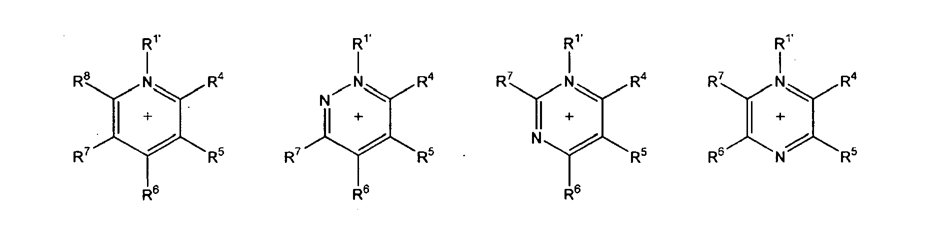 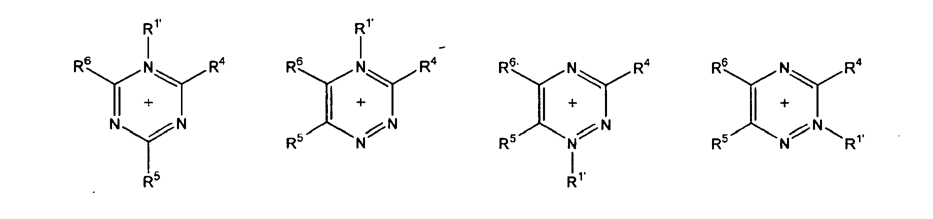 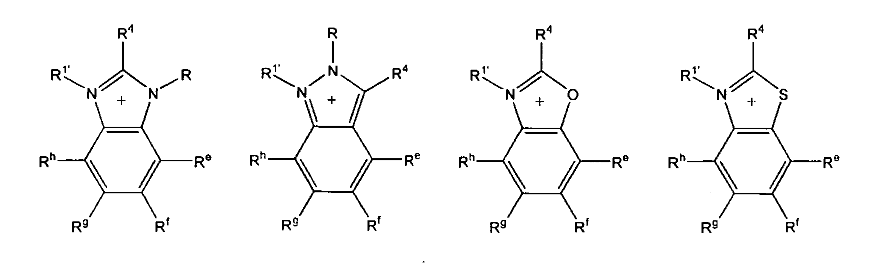 wobei die Reste folgende Bedeutung haben:
R Wasserstoff, ggf. sub. Alkyl, Alkenyl, Alkinyl, Cycloalkyl, Cycloalkenyl, Aryl oder Heteroaryl;
R¹, R², R³ unabhängig voneinander für Wasserstoff, ggf. sub. Alkyl, Alkenyl, Alkinyl, Cycloalkyl, Cycloalkenyl, Aryl oder Heteroaryl stehen; oder
zwei der Reste R¹, R², R³ bilden gemeinsam mit dem Heteroatom, an welchem sie gebunden sind einen Ring aus, wobei dieser gesättigt oder ungesättigt, unsubstituiert oder substituiert ist und wobei diese Kette durch ein oder mehrere Heteroatome ausgewählt aus der Gruppe O, S, NH oder N-C₁-C₄-Alkyl unterbrochen sein kann;
R⁴, R⁵, R⁶, R⁷, R⁸ unabhängig voneinander Wasserstoff, Halogen, Nitro, Cyano, OR^{c}, SR^{c}, NR^{c}R^{d}, COR^{c}, COOR^{c}, CO-NR^{c}R^{d}, C₁-C₃₀-Alkyl, C₃-C₁₂-Cycloalkyl, C₂-C₃₀-Alkenyl, C₃-C₁₂-Cycloalkenyl, Aryl oder Heteroaryl, wobei die 6 letztgenannten Reste ein oder mehrere Halogenreste tragen können und/oder 1 bis 3 Reste ausgewählt aus der Gruppe C₁-C₆-Alkyl, Aryl, Heteroaryl, C₃-C₇-Cycloalkyl, Halogen, OR^{c}, SR^{c}, NR^{c}R^{d}, COR^{c}, COOR^{c}, CO-NR^{c}R^{d} , wobei R^{c} und R^{d} unabhängig voneinander für Wasserstoff, C₁-C₆-Alkyl, C₁-C₆-Halogenalkyl, Cyclopentyl, Cyclohexyl, Phenyl, Tolyl oder Benzyl stehen; oder
zwei der Reste R, R⁴, R⁵, R⁶, R⁷, R⁸, welche benachbart sind, bilden gemeinsam mit dem Atom, an welchem sie gebunden sind einen Ring aus, wobei dieser ungesättigt oder aromatisch, unsubstituiert oder substituiert ist und wobei die durch die betreffenden Reste gebildetete Kette durch ein oder mehrere Heteroatome ausgewählt aus der Gruppe O, S, N, NH oder N-C₁-C₄-Alkyl unterbrochen sein kann.

In einer besonderen Ausgestaltungsform werden nach dem erfindungsgemäßen Verfahren quaternäre Carbonate der Formel I hergestellt, wobei:
[A]⁺ für ein quaternäres Ammonium-Kation [R^{1'}R¹R²R³N]⁺, ein Phosphonium-Kation [R^{1'}R¹R²R³P]⁺, ein Sulfonium-Kation [R^{1'}R¹R²S]⁺ oder ein heteroaromatisches Kation steht, das ausgewählt ist aus der Gruppe 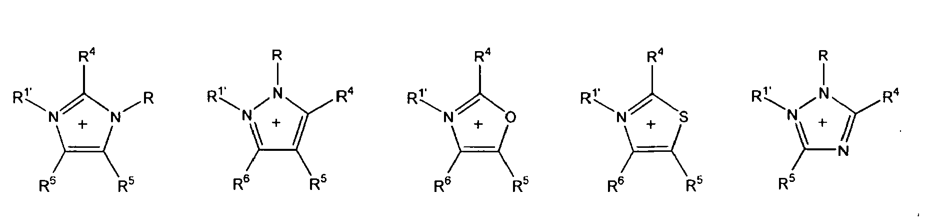 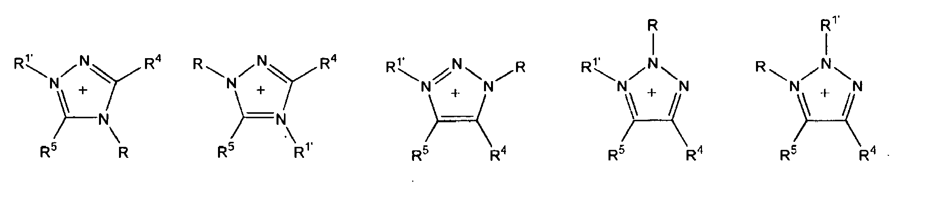 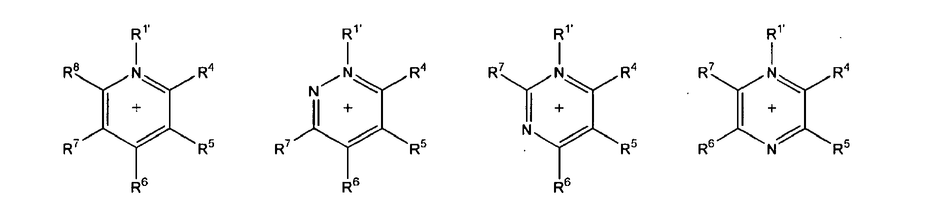 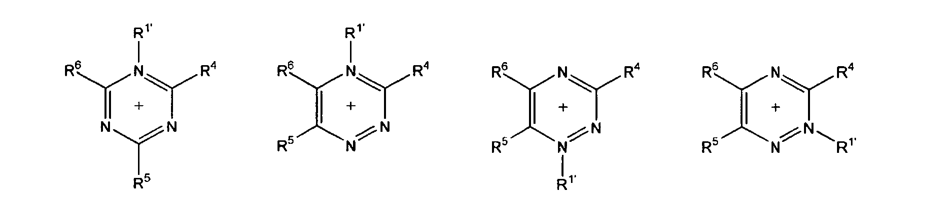 wobei die Reste folgende Bedeutung haben:
   R¹, R², R³ unabhängig voneinander für Wasserstoff, ggf. sub. Alkyl, Alkenyl, Alkinyl, Cycloalkyl, Cycloalkenyl, Aryl oder Heteroaryl stehen; oder
   zwei der Reste R¹, R², R³ bilden gemeinsam mit dem Heteroatom, an welchem sie gebunden sind einen Ring aus, wobei dieser gesättigt oder ungesättigt, unsubstituiert oder substituiert ist und wobei diese Kette durch ein oder mehrere Heteroatome ausgewählt aus der Gruppe O, S, NH oder N-C₁-C₄-Alkyl unterbrochen sein kann;
   R⁴, R⁵, R⁶, R⁷, R⁸ unabhängig voneinander Wasserstoff, Halogen, Nitro, Cyano, OR^{c}, SR^{c}, NR^{c}R^{d}, COR^{c}, COOR^{c}, CO-NR^{c}R^{d}, C₁-C₃₀-Alkyl, C₃-C₁₂-Cycloalkyl, C₂-C₃₀-Alkenyl, C₃-C₁₂-Cycloalkenyl, Aryl oder Heteroaryl, wobei die 6 letztgenannten Reste ein oder mehrere Halogenreste tragen können und/oder 1 bis 3 Reste ausgewählt aus der Gruppe C₁-C₆-Alkyl, Aryl, Heteroaryl, C₃-C₇-Cycloalkyl, Halogen, OR^{c}, SR^{c}, NR^{c}R^{d}, COR^{c}, COOR^{c}, CO-NR^{c}R^{d}, wobei R^{c} und R^{d} unabhängig voneinander für Wasserstoff, C₁-C₆-Alkyl, C₁-C₆-Halogenalkyl, Cyclopentyl, Cyclohexyl, Phenyl, Tolyl oder Benzyl stehen; oder
   zwei der Reste R, R⁴, R⁵, R⁶, R⁷, R⁸, welche benachbart sind, bilden gemeinsam mit dem Atom, an welchem sie gebunden sind einen Ring aus, wobei dieser ungesättigt oder aromatisch, unsubstituiert oder substituiert ist und wobei die durch die betreffenden Reste gebildetete Kette durch ein oder mehrere Heteroatome ausgewählt aus der Gruppe O, S, N, NH oder N-C₁-C₄-Alkyl unterbrochen sein kann;
   R Wasserstoff, ggf. sub. Alkyl, Alkenyl, Alkinyl, Cycloalkyl, Cycloalkenyl, Aryl oder Heteroaryl;
   R^{1'}, R^{2'} unabhängig voneinander ggf. sub. Alkyl, Alkenyl, Alkinyl, Cycloalkyl, Cycloalkenyl, Aryl oder Heteroaryl; oder
   R^{1'} und R^{2'} gemeinsam eine -CHR^{a}-CHR^{b}-Kette, -CHR^{a}-CH₂-CHR^{b}-Kette oder ggf. substuierten -C₆H₄-1,2-diyl-Rest bilden, wobei R^{a} und R^{b} unabhängig voneinander für Wasserstoff oder C₁-C₆-Alkyl stehen; wobei in diesem Fall eine zwitterionische Verbindung der Formel I' erhalten wird.

In einer bevorzugten Ausgestaltungsform werden nach dem erfindungsgemäßen Verfahren quaternäre Carbonate der Formel I hergestellt, wobei:
R¹, R², R³ unabhängig voneinander für Wasserstoff, C₁-C₃₀-Alkyl, C₃-C₁₂-Cycloalkyl, C₂-C₃₀-Alkenyl, C₃-C₁₂-Cycloalkenyl, C₂-C₃₀-Alkinyl, Aryl oder Heteroaryl, wobei die 7 letztgenannten Reste ein oder mehrere Halogenreste tragen können und/oder 1 bis 3 Reste ausgewählt aus der Gruppe C₁-C₆-Alkyl, Aryl, Heteroaryl, C₃-C₇-Cycloalkyl, Halogen, OR^{c}, SR^{c}, NR^{c}R^{d}, COR^{c}, COOR^{c}, CO-NR^{c}R^{d}, und wobei R^{c} und R^{d} für Wasserstoff, C₁-C₆-Alkyl, C₁-C₆-Halogenalkyl, Cyclopentyl, Cyclohexyl, Phenyl, Tolyl
   oder Benzyl stehen; oder
   zwei der Reste R¹, R², R³ gemeinsam mit dem Heteroatom, an welchem sie gebunden sind einen Ring ausbilden, wobei dieser gesättigt oder ungesättigt, unsubstituiert oder substituiert ist und wobei diese Kette durch ein oder mehrere Heteroatome ausgewählt aus der Gruppe O, S, NH oder N-C₁-C₄-Alkyl unterbrochen sein kann;
R⁴, R⁵, R⁶, R⁷, R⁸ unabhängig voneinander für Wasserstoff, Halogen, Nitro, Cyano, OR^{c}, SR^{c}, NR^{c}R^{d}, COR^{c}, COOR^{c}, CO-NR^{c}R^{d}, C₁-C₃₀-Alkyl, C₃-C₁₂-Cycloalkyl, C₂-C₃₀-Alkenyl, C₃-C₁₂-Cycloalkenyl, Aryl oder Heteroaryl, wobei die 6 letztgenannten Reste ein oder mehrere Halogenreste tragen können und/oder 1 bis 3 Reste ausgewählt aus der Gruppe C₁-C₆-Alkyl, Aryl, Heteroaryl; C₃-C₇-Cycloalkyl, Halogen, OR^{c}, SR^{c}, NR^{c}R^{d}, COR^{c}, COOR^{c}, CO-NR^{c}R^{d}, und wobei R^{c} und R^{d} für Wasserstoff, C₁-C₆-Alkyl, C₁-C₆-Halogenalkyl, Cyclopentyl, Cyclohexyl, Phenyl, Tolyl oder Benzyl stehen; oder
zwei der Reste R, R⁴, R⁵, R⁶, R⁷, R⁸, welche benachbart sind, bilden gemeinsam mit dem Atom, an welchem sie gebunden sind einen Ring aus, wobei dieser ungesättigt oder aromatisch, unsubstituiert oder substituiert ist und wobei die durch die betreffenden Reste gebildetete Kette durch ein oder mehrere Heteroatome ausgewählt aus der Gruppe O, S, N, NH oder N-C₁-C₄-Alkyl unterbrochen sein kann; oder
R für Wasserstoff, C₁-C₃₀-Alkyl, C₃-C₁₂-Cycloalkyl, C₂-C₃₀-Alkenyl, C₃-C₁₂-Cycloalkenyl, C₂-C₃₀-Alkinyl, Aryl oder Heteroaryl, wobei die 7 letztgenannten Reste ein oder mehrere Halogenreste tragen können und/oder 1 bis 3 Reste ausgewählt aus der Gruppe C₁-C₆-Alkyl, Aryl, Heteroaryl, C₃-C₇-Cycloalkyl, Halogen, OR^{c}, SR^{c}, NR^{c}R^{d}, COR^{c}, COOR^{c}, CO-NR^{c}R^{d}, wobei R^{c} und R^{d} für Wasserstoff, C₁-C₆-Alkyl, C₁-C₆-Halogenalkyl, Cyclopentyl, Cyclohexyl, Phenyl, Tolyl oder Benzyl steht;
R^{1'}, R²' unabhängig voneinander für ein C₁-C₃₀-Alkyl, C₃-C₁₂-Cycloalkyl, C₂-C₃₀-Alkenyl, C₃-C₁₂-Cycloalkenyl, C₂-C₃₀-Alkinyl, Aryl oder Heteroaryl, wobei die 7 letztgenannten Reste ein oder mehrere Halogenreste tragen können und/oder 1 bis 3 Reste ausgewählt aus der Gruppe C₁-C₆-Alkyl, Aryl, Heteroaryl, C₃-C₇-Cycloalkyl, Halogen, OR^{c}, SR^{c}, NR^{c}R^{d}, COR^{c}, COOR^{c}, CO-NR^{c}R^{d}, wobei R^{c} und R^{d} für Wasserstoff, C₁-C₆-Alkyl, C₁-C₆-Halogenalkyl, Cyclopentyl, Cyclohexyl, Phenyl, Tolyl oder Benzyl stehen; oder
R^{1'} und R²' gemeinsam eine -CHR^{a}-CHR^{b}-Kette, -CHR^{a}-CH₂-CHR^{b}-Kette oder ggf. substuierten -C₆H₄-1,2-diyl-Rest bilden, wobei R^{a} und R^{b} unabhängig voneinander für Wasserstoff oder C₁-C₆-Alkyl stehen;

In einer weiteren bevorzugten Ausgestaltungsform werden nach dem erfindungsgemäßen Verfahren quaternäre Carbonate der Formel I hergestellt, wobei die Reste unabhängig als auch im Kombination folgende Bedeutungen haben:
R¹, R², R³ unabhängig voneinander für Wasserstoff, C₁-C₃₀-Alkyl, C₃-C₁₂-Cycloalkyl,
   C₂-C₃₀-Alkenyl, C₃-C₁₂-Cycloalkenyl, Aryl oder Heteroaryl, wobei die 6 letztgenannten Reste ein oder mehrere Halogenreste tragen können und/oder 1 bis 3 Reste ausgewählt aus der Gruppe C₁-C₆-Alkyl, Aryl, Heteroaryl, C₃-C₇-Cycloalkyl, Halogen, OR^{c}, SR^{c}; NR^{c}R^{d}, COR^{c}, COOR^{c}, CO-NR^{c}R^{d}, und wobei R^{c} und R^{d} für Wasserstoff, C₁-C₆-Alkyl, C₁-C₆-Halogenalkyl, Cyclopentyl, Cyclohexyl, Phenyl, Tolyl oder Benzyl stehen;
   vorzugsweise für
   Wasserstoff;
   C₁-C₃₀-Alkyl, insbesondere Methyl, Ethyl, 1-Propyl, 2-Propyl, 1-Butyl, 2-Butyl, 2-Methyl-1-propyl (Isobutyl), 2-Methyl-2-propyl (tert.-Butyl), 1-Pentyl, 2-Pentyl, 3-Pentyl, 2-Methyl-1-butyl, 3-Methyl-1-butyl, 2-Methyl-2-butyl, 3-Methyl-2-butyl, 2,2-Dimethyl-1-propyl, 1-Hexyl, 2-Hexyl, 3-Hexyl, 2-Methyl-1-pentyl, 3-Methyl-1-pentyl, 4-Methyl-1-pentyl, 2-Methyl-2-pentyl, 3-Methyl-2-pentyl, 4-Methyl-2-pentyl, 2-Methyl-3-pentyl, 3-Methyl-3-pentyl, 2,2-Dimethyl-1-butyl, 2,3-Dimethyl-1-butyl, 3,3-Dimethyl-1-butyl, 2-Ethyl-1-butyl, 2,3-Dimethyl-2-butyl, 3,3-Dimethyl-2-butyl, Heptyl, Octyl, Nonyl, Decyl, Undecyl, Dodecyl, Tridecyl, Tetradecyl, Pentadecyl, Hexadecyl, Heptadecyl, Octadecyl, Nonadecyl, Icosyl, Henicosyl, Docosyl, Tricosyl, Tetracosyl, Pentacosyl, Hexacosyl, Heptacosy, Octacosyl, Nonacosyl oder Triacontyl; C₁-C₆-Alkyl, welches durch Phenyl oder C₅-C₇-Cycloalkyl substituiert ist, insbesondere Phenylmethyl (Benzyl), Diphenylmethyl, Triphenylmethyl, 2-Phenylethyl, 3-Phenylpropyl, Cyclopentylmethyl, 2-Cyclopentylethyl, 3-Cyclopentylpropyl, Cyclohexylmethyl, 2-Cyclohexylethyl oder 3-Cyclohexylpropyl; CₙF_{2(n-a)-(1-b)}H_{2a-b} mit n ≤30, 0 ≤ a ≤ n und b = 0 oder 1, insbesondere CF₃, C₂F₅, C₆F₁₃, C₈F₁₇, C₁₀F₂₁ oder C₁₂F₂₅;
   C₃-C₁₂-Cycloalkyl, insbesondere Cyclopentyl oder Cyclohexyl,; C₃-C₁₂-Cycloalkyl, welches durch C₁-C₆-Alkyl substituiert ist, insbesondere 2-Methyl-1-cyclopentyl, 3-Methyl-1-cyclopentyl, 2-Methyl-1-cyclohexyl, 3-Methyl-1-cyclohexyl oder 4-Methyl-1-cyclohexyl; CₙF_{2(n-a)-(1-b)}H_{2a-b} mit n ≤30, 0 ≤ a ≤ n und b = 0 oder 1;
   C₂-C₃₀-Alkenyl, insbesondere 2-Propenyl, 3-Butenyl, cis-2-Butenyl oder trans-2-Butenyl; CₙF_{2(n-a)-(1-b)}H_{2a-b} mit n ≤30, 0 ≤ a ≤ n und b = 0 oder 1; C₃-C₁₂-Cycloalkenyl, insbesondere 3-Cyclopentenyl, 2-Cyclohexenyl, 3-Cyclohexenyl oder 2,5-Cyclohexadienyl; CₙF_{2(n-a)-(1-b)}H_{2a-b} mit n ≤30, 0 ≤ a ≤ n und b = 0 oder 1; Aryl oder Heteroaryl mit 2 bis 30 Kohlenstoffatomen, insbesondere Phenyl, 1-Naphthyl, 2-Naphthyl, 1-Pyrrolyl, 2-Pyrrolyl, 3-Pyrrolyl, 2-Pyridinyl, 3-Pyridinyl oder 4-Pyridinyl; Aryl oder Heteroaryl mit 6 bis 30 Kohlenstoffatomen, welche durch ein bis drei C₁-C₆-Alkyl- oder Phenyl-Reste substituiert sind, insbesondere 2-Methyl-phenyl (2-Tolyl), 3-Methyl-phenyl (3-Tolyl), 4-Methyl-phenyl, 2-Ethyl-phenyl, 3-Ethyl-phenyl, 4-Ethyl-phenyl, 2,3-Dimethyl-phenyl, 2,4-Dimethyl-phenyl, 2,5-Dimethyl-phenyl, 2,6-Dimethyl-phenyl, 3,4-Dimethyl-phenyl, 3,5-Dimethyl-phenyl oder 4-Phenyl-phenyl; oder C₆F₍₅₋ₐ₎Hₐ mit 0≤ a ≤ 5; stehen;
   oder
   zwei der Reste R¹, R², R³ bilden gemeinsam mit dem Heteroatom, an das sie gebunden sind eine -(CH₂)ₙ-Kette, wobei n 4, 5, 6 oder 7 ist, und wobei diese Kette durch 1 bis maximal drei C₁-C₄-Alkylreste substituiert sein kann und/oder durch O, S, NH oder N-C₁-C₄-Alkyl unterbrochen sein kann;
R⁴, R⁵, R⁶, R⁷, R⁸ unabhängig voneinander für Wasserstoff, Halogen, OR^{c}, COR^{c}, C₁-C₃₀-Alkyl, C₃-C₁₂-Cycloalkyl, C₂-C₃₀-Alkenyl, C₃-C₁₂-Cycloalkenyl, Aryl oder Heteroaryl, wobei die 6 letztgenannten Reste ein oder mehrere Halogenreste tragen können und/oder 1 bis 3 Reste ausgewählt aus der Gruppe C₁-C₆-Alkyl, Aryl, Heteroaryl, C₃-C₇-Cycloalkyl, Halogen, OR^{c}, SR^{c}, NR^{c}R^{d}, COR^{c}, COOR^{c}, CO-NR^{c}R^{d}, und wobei R^{c} und R^{d} für Wasserstoff, C₁-C₆-Alkyl, C₁-C₆-Halogenalkyl, Cyclopentyl, Cyclohexyl, Phenyl, Tolyl oder Benzyl stehen;
   vorzugsweise für
   Wasserstoff;
   Halogen,
   C₁-C₃₀-Alkyl, insbesondere Methyl, Ethyl, 1-Propyl, 2-Propyl, 1-Butyl, 2-Butyl, 2-Methyl-1-propyl (Isobutyl), 2-Methyl-2-propyl (tert.-Butyl), 1-Pentyl, 2-Pentyl, 3-Pentyl, 2-Methyl-1-butyl, 3-Methyl-1-butyl, 2-Methyl-2-butyl, 3-Methyl-2-butyl, 2,2-Dimethyl-1-propyl, 1-Hexyl, 2-Hexyl, 3-Hexyl, 2-Methyl-1-pentyl, 3-Methyl-1-pentyl, 4-Methyl-1-pentyl, 2-Methyl-2-pentyl, 3-Methyl-2-pentyl, 4-Methyl-2-pentyl, 2-Methyl-3-pentyl, 3-Methyl-3-pentyl, 2,2-Dimethyl-1-butyl, 2,3-Dimethyl-1-butyl, 3,3-Dimethyl-1-butyl, 2-Ethyl-1-butyl, 2,3-Dimethyl-2-butyl, 3,3-Dimethyl-2-butyl, Heptyl, Octyl, Nonyl, Decyl, Undecyl, Dodecyl, Tridecyl, Tetradecyl, Pentadecyl, Hexadecyl, Heptadecyl, Octadecyl, Nonadecyl, Icosyl, Henicosyl, Docosyl, Tricosyl, Tetracosyl, Pentacosyl, Hexacosyl, Heptacosy, Octacosyl, Nonacosyl oder Triacontyl; C₁-C₆-Alkyl, welches durch Phenyl oder C₅-C₇-Cycloalkyl substituiert ist, insbesondere Phenylmethyl (Benzyl), Diphenylmethyl, Triphenylmethyl, 2-Phenylethyl, 3-Phenylpropyl, Cyclopentylmethyl, 2-Cyclopentylethyl, 3-Cyclopentylpropyl, Cyclohexylmethyl, 2-Cyclohexylethyl oder 3-Cyclohexylpropyl; CₙF_{2(n-a)-(1-b})H_{2a-b} mit n ≤30, 0 ≤ a ≤ n und b = 0 oder 1, insbesondere CF₃, C₂F₅, C₆F₁₃, C₈F₁₇, C₁₀F₂₁ oder C₁₂F₂₅; OR^{a}, insbesondere Methoxy, Ethoxy; oder COR^{a}, insbesondere Formyl oder Acetyl; C₃-C₁₂-Cycloalkyl, insbesondere Cyclopentyl oder Cyclohexyl; C₃-C₁₂-Cycloalkyl, welches durch C₁-C₆-alkyl substituiert ist, insbesondere 2-Methyl-1-cyclopentyl, 3-Methyl-1-cyclopentyl, 2-Methyl-1-cyclohexyl, 3-Methyl-1-cyclohexyl oder 4-Methyl-1-cyclohexyl;
   CₙF_{2(n-a)-(1-b)}H_{2a-b} mit n ≤30, 0 ≤ a ≤ n und b = 0 oder 1;
   C₂-C₃₀-Alkenyl, insbesondere 2-Propenyl, 3-Butenyl, cis-2-Butenyl oder trans-2-Butenyl; CₙF_{2(n-a)-(1-b)}H_{2a-b} mit n ≤30, 0 ≤ a ≤ n und b = 0 oder 1;
   C₃-C₁₂-Cycloalkenyl, insbesondere 3-Cyclopentenyl, 2-Cyclohexenyl, 3-Cyclohexenyl oder 2,5-Cyclohexadienyl;
   CₙF_{2(n-a)-(1-b})H_{2a-b} mit n ≤30, 0 ≤ a ≤ n und b = 0 oder 1;
   Aryl oder Heteroaryl mit 2 bis 30 Kohlenstoffatomen, insbesondere Phenyl, 1-Naphthyl, 2-Naphthyl, 1-Pyrrolyl, 2-Pyrrolyl, 3-Pyrrolyl, 2-Pyridinyl, 3-Pyridinyl oder 4-Pyridinyl;
   Aryl oder Heteroaryl mit 6 bis 30 Kohlenstoffatomen, welche durch ein bis drei C₁-C₆-Alkyl- oder Phenyl-Reste substituiert sind, insbesondere 2-Methyl-phenyl (2-Tolyl), 3-Methyl-phenyl (3-Tolyl), 4-Methyl-phenyl, 2-Ethyl-phenyl, 3-Ethyl-phenyl, 4-Ethyl-phenyl, 2,3-Dimethyl-phenyl, 2,4-Dimethyl-phenyl, 2,5-Dimethyl-phenyl, 2,6-Dimethyl-phenyl, 3,4-Dimethyl-phenyl, 3,5-Dimethyl-phenyl oder 4-Phenyl-phenyl; oder C₆F₍₅₋ₐ₎Hₐ mit 0≤ a ≤ 5; stehen; oder
   zwei der Reste R, R⁴, R⁵, R⁶, R⁷, R⁸, welche benachbart sind, bilden gemeinsam mit dem Atom, an welchem sie gebunden sind ein 5- oder 6 gliedriges Ringsystem oder ein 5-oder 6-gliedriges aromatisches Ringsystem, welches unsubstituiert ist, oder durch 1 bis 4 Reste aus der Gruppe C₁-C₆-Alkyl, Aryl-, Heteroaryl-, C₃-C₇-Cycloalkyl, Halogen, OR^{a}, SR^{a}, NR^{a}R^{b}, COOR^{a}, CO-NR^{a}R^{b} oder COR^{a} substituiert ist, wobei R^{a}, R^{b} unabhängig voneinander für Wasserstoff, C₁-C₆-Alkyl, C₁-C₆-Halogenalkyl, Cyclopentyl, Cyclohexyl, Phenyl, Tolyl oder Benzyl stehen;
   vorzugsweise
   zwei der Reste R, R⁴, R⁵, R⁶, R⁷, R⁸, welche benachbart sind, bilden gemeinsam mit dem Atom, an welchem sie gebunden sind ein 5- oder 6 gliedriges Ringsystem oder ein 5- oder 6-gliedriges aromatisches Ringsystem, welches unsubstituiert ist, oder durch 1 bis 4 Reste aus der Gruppe C₁-C₆-Alkyl, Aryl-, Heteroaryl-, C₃-C₇-Cycloalkyl, Halogen, OR^{a}, SR^{a}, NR^{a}R^{b}, COOR^{a}, CO-NR^{a}R^{b} oder COR^{a} substituiert ist ,wobei R^{a}, R^{b} unabhängig voneinander für Wasserstoff, C₁-C₆-Alkyl, C₁-C₆-Halogenalkyl, Cyclopentyl, Cyclohexyl, Phenyl, Tolyl oder Benzyl stehen;
R für Wasserstoff, C₁-C₃₀-Alkyl, C₃-C₁₂-Cycloalkyl, C₂-C₃₀-Alkenyl, C₃-C₁₂-Cycloalkenyl, Aryl oder Heteroaryl, wobei die 6 letztgenannten Reste ein oder mehrere Halogenreste tragen können und/oder 1 bis 3 Reste ausgewählt aus der Gruppe C₁-C₆-Alkyl, Aryl, Heteroaryl, C₃-C₇-Cycloalkyl, Halogen, OR^{c}, SR^{c}, NR^{c}R^{d}, COR^{c}, COOR^{c}, CO-NR^{c}R^{d}, wobei R^{c} und R^{d} für Wasserstoff, C₁-C₆-Alkyl, C₁-C₆-Halogenalkyl, Cyclopentyl, Cyclohexyl, Phenyl, Tolyl oder Benzyl steht; vorzugsweise für
   Wasserstoff;
   C₁-C₃₀-Alkyl, insbesondere Methyl, Ethyl, 1-Propyl, 2-Propyl, 1-Butyl, 2-Butyl, 2-Methyl-1-propyl (Isobutyl), 2-Methyl-2-propyl (tert.-Butyl), 1-Pentyl, 2-Pentyl, 3-Pentyl, 2-Methyl-1-butyl, 3-Methyl-1-butyl, 2-Methyl-2-butyl, 3-Methyl-2-butyl, 2,2-Dimethyl-1-propyl, 1-Hexyl, 2-Hexyl, 3-Hexyl, 2-Methyl-1-pentyl, 3-Methyl-1-pentyl, 4-Methyl-1-pentyl, 2-Methyl-2-pentyl, 3-Methyl-2-pentyl, 4-Methyl-2-pentyl, 2-Methyl-3-pentyl, 3-Methyl-3-pentyl, 2,2-Dimethyl-1-butyl, 2,3-Dimethyl-1-butyl, 3,3-Dimethyl-1-butyl, 2-Ethyl-1-butyl, 2,3-Dimethyl-2-butyl, 3,3-Dimethyl-2-butyl, Heptyl, Octyl, Nonyl, Decyl, Undecyl, Dodecyl, Tridecyl, Tetradecyl, Pentadecyl, Hexadecyl, Heptadecyl, Octadecyl, Nonadecyl, Icosyl, Henicosyl, Docosyl, Tricosyl, Tetracosyl, Pentacosyl, Hexacosyl, Heptacosy, Octacosyl, Nonacosyl oder Triacontyl;
   C₁-C₆-Alkyl, welches durch Phenyl oder C₅-C₇-Cycloalkyl substituiert ist, insbesondere Phenylmethyl (Benzyl), Diphenylmethyl, Triphenylmethyl, 2-Phenylethyl, 3-Phenylpropyl, Cyclopentylmethyl, 2-Cyclopentylethyl, 3-Cyclopentylpropyl, Cyclohexylmethyl, 2-Cyclohexylethyl oder 3-Cyclohexylpropyl;
   CₙF_{2(n-a)-(1-b)}H_{2a-b} mit n ≤30, 0 ≤ a ≤ n und b = 0 oder 1, insbesondere CF₃, C₂F₅, C₆F₁₃, C₈F₁₇, C₁₀F₂₁ oder C₁₂F₂₅;
   C₃-C₁₂-Cycloalkyl, insbesondere Cyclopentyl oder Cyclohexyl;
   C₃-C₁₂-Cycloalkyl, welches durch C₁-C₆-Alkyl substituiert ist, insbesondere 2-Methyl-1-cyclopentyl, 3-Methyl-1-cyclopentyl, 2-Methyl-1-cyclohexyl, 3-Methyl-1-cyclohexyl oder 4-Methyl-1-cyclohexyl; CₙF_{2(n-a)-(1-b)}H_{2a-b} mit n ≤30, 0 ≤ a ≤ n und b = 0 oder 1;
   C₂-C₃₀-Alkenyl, insbesondere 2-Propenyl, 3-Butenyl, cis-2-Butenyl oder trans-2-Butenyl; CₙF_{2(n-a)-(1-b)}H_{2a-b} mit n ≤30, 0 ≤ a ≤ n und b = 0 oder 1;
   C₃-C₁₂-Cycloalkenyl, insbesondere 3-Cyclopentenyl, 2-Cyclohexenyl, 3-Cyclohexenyl oder 2,5-Cyclohexadienyl;
   CₙF_{2(n-a)-(1-b)}H_{2a-b} mit n ≤30, 0 ≤ a ≤ n und b = 0 oder 1;
   Aryl oder Heteroaryl mit 2 bis 30 Kohlenstoffatomen, insbesondere Phenyl, 1-Naphthyl, 2-Naphthyl, 1-Pyrrolyl, 2-Pyrrolyl, 3-Pyrrolyl, 2-Pyridinyl, 3-Pyridinyl oder 4-Pyridinyl; Aryl oder Heteroaryl mit 6 bis 30 Kohlenstoffatomen, welche durch ein bis drei C₁-C₆-Alkyl- oder Phenyl-Reste substituiert sind, insbesondere 2-Methyl-phenyl (2-Tolyl), 3-Methyl-phenyl (3-Tolyl), 4-Methyl-phenyl, 2-Ethyl-phenyl, 3-Ethyl-phenyl, 4-Ethyl-phenyl, 2,3-Dimethyl-phenyl, 2,4-Dimethyl-phenyl, 2,5-Dimethyl-phenyl, 2,6-Dimethyl-phenyl, 3,4-Dimethyl-phenyl, 3,5-Dimethyl-phenyl oder 4-Phenyl-phenyl; oder C₆F₍₅₋ₐ₎Hₐ mit 0≤ a ≤ 5; stehen;
R^{1'}, R^{2'} unabhängig voneinander für ein C₁-C₃₀-Alkyl, C₃-C₁₂-Cycloalkyl, C₂-C₃₀-Alkenyl, C₃-C₁₂-Cycloalkenyl, C₂-C₃₀-Alkinyl, Aryl oder Heteroaryl, wobei die 7 letztgenannten Reste ein oder mehrere Halogenreste tragen können und/oder 1 bis 3 Reste ausgewählt aus der Gruppe C₁-C₆-Alkyl, Aryl, Heteroaryl, C₃-C₇-Cycloalkyl, Halogen, OR^{c} SR^{c}, NR^{c}R^{d}, COR^{c}, COOR^{c}, CO-NR^{c}R^{d}, wobei R^{c} und R^{d} für Wasserstoff, C₁-C₆-Alkyl, C₁-C₆-Halogenalkyl, Cyclopentyl, Cyclohexyl, Phenyl, Tolyl oder Benzyl stehen;
   vorzugsweise für
   C₁-C₃₀-Alkyl, insbesondere Methyl, Ethyl, 1-Propyl, 2-Propyl, 1-Butyl, 2-Butyl, 2-Methyl-1-propyl (Isobutyl), 2-Methyl-2-propyl (tert.-Butyl), 1-Pentyl, 2-Pentyl, 3-Pentyl, 2-Methyl-1-butyl, 3-Methyl-1-butyl, 2-Methyl-2-butyl, 3-Methyl-2-butyl, 2,2-Dimethyl-1-propyl, 1-Hexyl, 2-Hexyl, 3-Hexyl, 2-Methyl-1-pentyl, 3-Methyl-1-pentyl, 4-Methyl-1-pentyl, 2-Methyl-2-pentyl, 3-Methyl-2-pentyl, 4-Methyl-2-pentyl, 2-Methyl-3-pentyl, 3-Methyl-3-pentyl, 2,2-Dimethyl-1-butyl, 2,3-Dimethyl-1-butyl, 3,3-Dimethyl-1-butyl, 2-Ethyl-1-butyl, 2,3-Dimethyl-2-butyl, 3,3-Dimethyl-2-butyl, Heptyl, Octyl, Nonyl, Decyl, Undecyl, Dodecyl, Tridecyl, Tetradecyl, Pentadecyl, Hexadecyl, Heptadecyl, Octadecyl, Nonadecyl, Icosyl, Henicosyl, Docosyl, Tricosyl, Tetracosyl, Pentacosyl, Hexacosyl, Heptacosy, Octacosyl, Nonacosyl oder Triacontyl;
   C₁-C₆-Alkyl, welches durch Phenyl oder C₅-C₇-Cycloalkyl substituiert ist, insbesondere Phenylmethyl (Benzyl), Diphenylmethyl, Triphenylmethyl, 2-Phenylethyl, 3-Phenylpropyl, Cyclopentylmethyl, 2-Cyclopentylethyl, 3-Cyclopentylpropyl, Cyclohexylmethyl, 2-Cyclohexylethyl oder 3-Cyclohexylpropyl;
   CₙF_{2(n-a)-(1-b)}H_{2a-b} mit n ≤30, 0 ≤ a ≤ n und b = 0 oder 1, insbesondere CF₃, C₂F₅, C₆F₁₃, C₈F₁₇, C₁₀F₂₁ oder C₁₂F₂₅;
   C₃-C₁₂-Cycloalkyl, insbesondere Cyclopentyl oder Cyclohexyl;
   C₃-C₁₂-Cycloalkyl, welches durch C₁-C₆-Alkyl substituiert ist, insbesondere 2-Methyl-1-cyclopentyl, 3-Methyl-1-cyclopentyl, 2-Methyl-1-cyclohexyl, 3-Methyl-1-cyclohexyl oder 4-Methyl-1-cyclohexyl;
   CₙF_{2(n-a)-(1-b})H_{2a-b} mit n ≤30, 0 ≤ a ≤ n und b = 0 oder 1;
   C₂-C₃₀-Alkenyl, insbesondere 2-Propenyl, 3-Butenyl, cis-2-Butenyl oder trans-2-Butenyl;
   CₙF_{2(n-a)-(1-b})H_{2a-b}mit n ≤30, 0 ≤ a ≤ n und b = 0 oder 1;
   C₃-C₁₂-Cycloalkenyl, insbesondere 3-Cyclopentenyl, 2-Cyclohexenyl, 3-Cyclohexenyl oder 2,5-Cyclohexadienyl;
   CₙF_{2(n-a)-(1-b)}H_{2a-b} mit n ≤30, 0 ≤ a ≤ n und b = 0 oder 1;
   Aryl oder Heteroaryl mit 2 bis 30 Kohlenstoffatomen, insbesondere Phenyl, 1-Naphthyl, 2-Naphthyl, 1-Pyrrolyl, 2-Pyrrolyl, 3-Pyrrolyl, 2-Pyridinyl, 3-Pyridinyl oder 4-Pyridinyl;
   Aryl oder Heteroaryl mit 6 bis 30 Kohlenstoffatomen, welche durch ein bis drei C₁-C₆-Alkyl- oder Phenyl-Reste substituiert sind, insbesondere 2-Methyl-phenyl (2-Tolyl), 3-Methyl-phenyl (3-Tolyl), 4-Methyl-phenyl, 2-Ethyl-phenyl, 3-Ethyl-phenyl, 4-Ethyl-phenyl, 2,3-Dimethyl-phenyl, 2,4-Dimethyl-phenyl, 2,5-Dimethyl-phenyl, 2,6-Dimethyl-phenyl, 3,4-Dimethyl-phenyl, 3,5-Dimethyl-phenyl oder 4-Phenyl-phenyl; oder
   C₆F₍₅₋ₐ₎Hₐ mit 0≤ a ≤ 5; stehen; oder
R^{1'} und R²' gemeinsam eine -CHR^{a}-CHR^{b}-Kette, -CHR^{a}-CH₂-CHR^{b}-Kette oder einen -C₆H₄-1,2-diyl-Rest bilden, wobei R^{a} und R^{b} unabhängig voneinander für Wasserstoff oder C₁-C₆-Alkyl stehen;
   vorzugsweise für
   -CH₂-CH₂-, -CH₂-CH₂-CH₂-, -CH(CH₃)-CH₂-,-CH₂-CH(CH₃)-, -CH(CH₃)-CH(CH₃)- oder -C₆H₄-1,2-diyl-;

In einer weiteren bevorzugten Ausgestaltungsform werden nach dem erfindungsgemäßen Verfahren quaternäre Carbonate der Formel I hergestellt, wobei:
R^{1'}, R²' unabhängig voneinander für ein C₁-C₃₀-Alkyl, C₃-C₁₂-Cycloalkyl, C₂-C₃₀-Alkenyl, C₃- C₁₂-Cycloalkenyl, C₂-C₃₀-Alkinyl, Aryl oder Heteroaryl, wobei die 7 letztgenannten Reste ein oder mehrere Halogenreste tragen können und/oder 1 bis 3 Reste ausgewählt aus der Gruppe C₁-C₆-Alkyl, Aryl, Heteroaryl, C₃-C₇-Cycloalkyl, Halogen, OR^{c}, SR^{c}, NR^{c}R^{d}, COR^{c}, COOR^{c}, CO-NR^{c}R^{d}, wobei R^{c} und R^{d} für Wasserstoff, C₁-C₆-Alkyl, C₁-C₆-Halogenalkyl, Cyclopentyl, Cyclohexyl, Phenyl, Tolyl oder Benzyl stehen;
   vorzugsweise für
   C₁-C₃₀-Alkyl, insbesondere Methyl, Ethyl, 1-Propyl, 2-Propyl, 1-Butyl, 2-Butyl, 2-Methyl-1-propyl (Isobutyl), 2-Methyl-2-propyl (tert.-Butyl), 1-Pentyl, 2-Pentyl, 3-Pentyl, 2-Methyl-1-butyl, 3-Methyl-1-butyl, 2-Methyl-2-butyl, 3-Methyl-2-butyl, 2,2-Dimethyl-1-propyl, 1-Hexyl, 2-Hexyl, 3-Hexyl, 2-Methyl-1-pentyl, 3-Methyl-1-pentyl, 4-Methyl-1-pentyl, 2-Methyl-2-pentyl, 3-Methyl-2-pentyl, 4-Methyl-2-pentyl, 2-Methyl-3-pentyl, 3-Methyl-3-pentyl, 2,2-Dimethyl-1-butyl, 2,3-Dimethyl-1-butyl, 3,3-Dimethyl-1-butyl, 2-Ethyl-1-butyl, 2,3-Dimethyl-2-butyl, 3,3-Dimethyl-2-butyl, Heptyl, Octyl, Nonyl, Decyl, Undecyl, Dodecyl, Tridecyl, Tetradecyl, Pentadecyl, Hexadecyl, Heptadecyl, Octadecyl, Nonadecyl, Icosyl, Henicosyl, Docosyl, Tricosyl, Tetracosyl, Pentacosyl, Hexacosyl, Heptacosy, Octacosyl, Nonacosyl oder Triacontyl; C₁-C₆-Alkyl, welches durch Phenyl oder C₅-C₇-Cycloalkyl substituiert ist, insbesondere Phenylmethyl (Benzyl), Diphenylmethyl, Triphenylmethyl, 2-Phenylethyl, 3-Phenylpropyl, Cyclopentylmethyl, 2-Cyclopentylethyl, 3-Cyclopentylpropyl, Cyclohexylmethyl, 2-Cyclohexylethyl oder 3-Cyclohexylpropyl; CₙF_{2(n-a)-(1-b)}H_{2a-b} mit n ≤30, 0 ≤ a ≤ n und b = 0 oder 1, insbesondere CF₃, C₂F₅, C₆F₁₃, C₈F₁₇, C₁₀F₂₁ oder C₁₂F₂₅; C₃-C₁₂-Cycloalkyl, insbesondere Cyclopentyl oder Cyclohexyl;
   C₃-C₁₂-Cycloalkyl, welches durch C₁-C₆-Alkyl substituiert ist, insbesondere 2-Methyl-1-cyclopentyl, 3-Methyl-1-cyclopentyl, 2-Methyl-1-cyclohexyl, 3-Methyl-1-cyclohexyl oder 4-Methyl-1-cyclohexyl; CₙF_{2(n-a)-(1-b})H_{2a-b} mit n ≤30, 0 ≤ a ≤ n und b = 0 oder 1; C₂-C₃₀-Alkenyl, insbesondere 2-Propenyl, 3-Butenyl, cis-2-Butenyl oder trans-2-Butenyl; CₙF_{2(n-a)-(1-b})H_{2a-b} mit n ≤30, 0 ≤ a ≤ n und b = 0 oder 1; C₃-C₁₂-Cycloalkenyl, insbesondere 3-Cyclopentenyl, 2-Cyclohexenyl, 3-Cyclohexenyl oder 2,5-Cyclohexadienyl; CₙF_{2(n-a)-(1-b)}H_{2a-b} mit n ≤30, 0 ≤ a ≤ n und b = 0 oder 1; Aryl oder Heteroaryl mit 2 bis 30 Kohlenstoffatomen, insbesondere Phenyl, 1-Naphthyl, 2-Naphthyl, 1-Pyrrolyl, 2-Pyrrolyl, 3-Pyrrolyl, 2-Pyridinyl, 3-Pyridinyl oder 4-Pyridinyl; Aryl oder Heteroaryl mit 6 bis 30 Kohlenstoffatomen, welche durch ein bis drei C₁-C₆-Alkyl- oder Phenyl-Reste substituiert sind, insbesondere 2-Methyl-phenyl (2-Tolyl), 3-Methyl-phenyl (3-Tolyl), 4-Methyl-phenyl, 2-Ethyl-phenyl, 3-Ethyl-phenyl, 4-Ethyl-phenyl, 2,3-Dimethyl-phenyl, 2,4-Dimethyl-phenyl, 2,5-Dimethyl-phenyl, 2,6-Dimethyl-phenyl, 3,4-Dimethyl-phenyl, 3,5-Dimethyl-phenyl oder 4-Phenyl-phenyl; oder C₆F₍₅₋ₐ)Hₐ mit 0≤ a ≤ 5; stehen.

In einer weiteren bevorzugten Ausgestaltungsform werden nach dem erfindungsgemäßen Verfahren quaternäre Carbonate der Formel I hergestellt, wobei:
R^{1'} und R²' gemeinsam eine -CHR^{a}-CHR^{b}-Kette, -CHR^{a}-CH₂-CHR^{b}-Kette oder einen -C₆H₄-1,2-diyl-Rest bilden, wobei R^{a} und R^{b} unabhängig voneinander für Wasserstoff oder C₁-C₆-Alkyl stehen;
   vorzugsweise für -CH₂-CH₂-, -CH₂-CH₂-CH₂-, -CH(CH₃)-CH₂-,-CH₂-CH(CH₃)-, -CH(CH₃)-CH(CH₃)- oder
   -C₆H₄-1,2-diyl-;

In einer weiteren bevorzugten Ausgestaltungsform werden nach dem erfindungsgemäßen Verfahren quaternäre Carbonate der Formel I hergestellt, wobei:

[A⁺] ein heteroaromatisches Kation ist ausgewählt aus der Gruppe 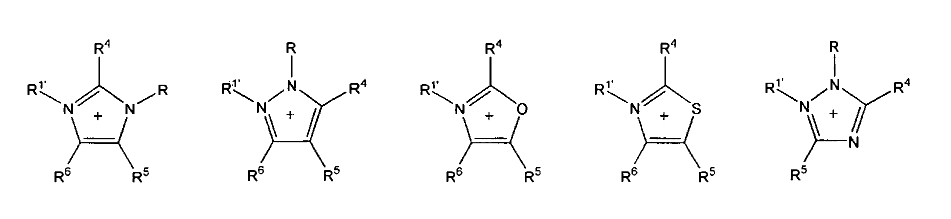 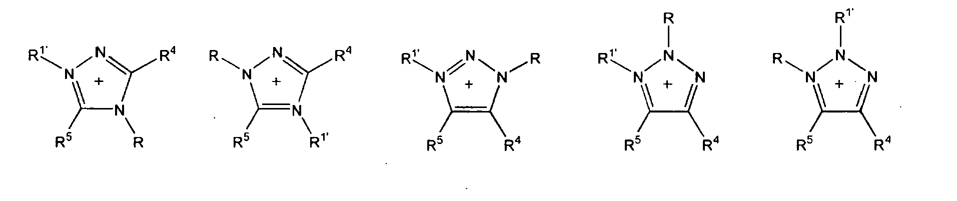 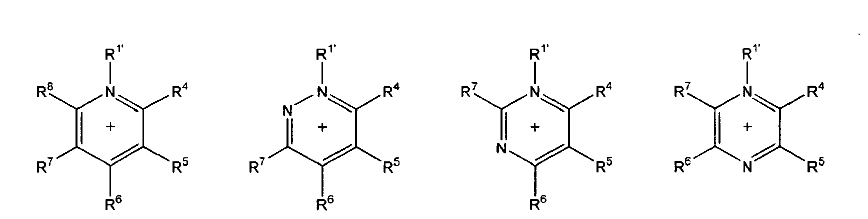 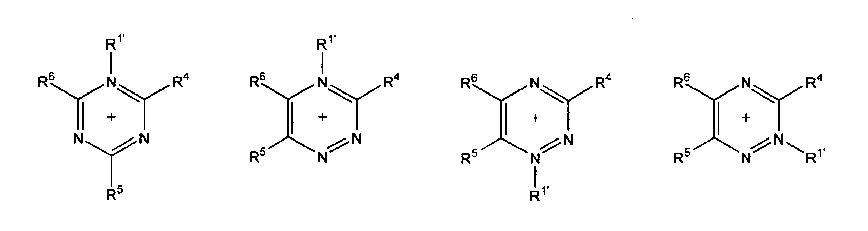 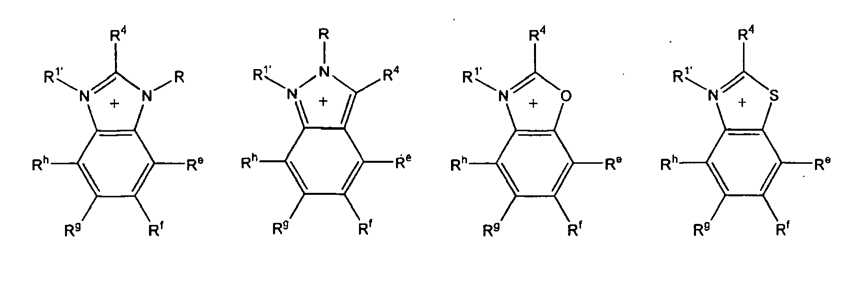 wobei
R⁴, R⁵, R⁶, R⁷, R⁸ unabhängig voneinander für Wasserstoff, Halogen, OR^{c}, COR^{c}, C₁-C₃₀-Alkyl, C₃-C₁₂-Cycloalkyl, C₂-C₃₀-Alkenyl, C₃-C₁₂-Cycloalkenyl, Aryl oder Heteroaryl, wobei die 6 letztgenannten Reste ein oder mehrere Halogenreste tragen können und/oder 1 bis 3 Reste ausgewählt aus der Gruppe C₁-C₆-Alkyl, Aryl, Heteroaryl, C₃-C₇-Cycloalkyl, Halogen, OR^{c}, SR^{c}, NR^{c}R^{d}, COR^{c}, COOR^{c}, CO-NR^{c}R^{d}, und wobei R^{c} und R^{d} für Wasserstoff, C₁-C₆-Alkyl, C₁-C₆-Halogenalkyl, Cyclopentyl, Cyclohexyl, Phenyl, Tolyl oder Benzyl stehen;
   vorzugsweise für
   Wasserstoff;
   Halogen,
   C₁-C₃₀-Alkyl, insbesondere Methyl, Ethyl, 1-Propyl, 2-Propyl, 1-Butyl, 2-Butyl, 2-Methyl-1-propyl (Isobutyl), 2-Methyl-2-propyl (tert.-Butyl), 1-Pentyl, 2-Pentyl, 3-Pentyl, 2-Methyl-1-butyl, 3-Methyl-1-butyl, 2-Methyl-2-butyl, 3-Methyl-2-butyl, 2,2-Dimethyl-1-propyl, 1-Hexyl, 2-Hexyl, 3-Hexyl, 2-Methyl-1-pentyl, 3-Methyl-1-pentyl, 4-Methyl-1-pentyl, 2-Methyl-2-pentyl, 3-Methyl-2-pentyl, 4-Methyl-2-pentyl, 2-Methyl-3-pentyl, 3-Methyl-3-pentyl, 2,2-Dimethyl-1-butyl, 2,3-Dimethyl-1-butyl, 3,3-Dimethyl-1-butyl, 2-Ethyl-1-butyl, 2,3-Dimethyl-2-butyl, 3,3-Dimethyl-2-butyl, Heptyl, Octyl, Nonyl, Decyl, Undecyl, Dodecyl, Tridecyl, Tetradecyl, Pentadecyl, Hexadecyl, Heptadecyl, Octadecyl, Nonadecyl, Icosyl, Henicosyl, Docosyl, Tricosyl, Tetracosyl, Pentacosyl, Hexacosyl, Heptacosy, Octacosyl, Nonacosyl oder Triacontyl;
   C₁-C₆-Alkyl, welches durch Phenyl oder C₅-C₇-Cycloalkyl substituiert ist, insbesondere Phenylmethyl (Benzyl), Diphenylmethyl, Triphenylmethyl, 2-Phenylethyl, 3-Phenylpropyl, Cyclopentylmethyl, 2-Cyclopentylethyl, 3-Cyclopentylpropyl, Cyclohexylmethyl, 2-Cyclohexylethyl oder 3-Cyclohexylpropyl;
   CₙF_{2(n-a)-(1-b)}H_{2a-b} mit n ≤30, 0 ≤ a ≤ n und b = 0 oder 1, insbesondere CF₃, C₂F₅, C₆F₁₃, C₈F₁₇, C₁₀F₂₁ oder C₁₂F₂₅;
   OR^{a}, insbesondere Methoxy, Ethoxy; oder
   COR^{a}, insbesondere Formyl, oder Acetyl;
   C₃-C₁₂-Cycloalkyl, insbesondere Cyclopentyl oder Cyclohexyl;
   C₃-C₁₂-Cycloalkyl, welches durch C₁-C₆-Alkyl substituiert ist, insbesondere 2-Methyl-1-cyclopentyl, 3-Methyl-1-cyclopentyl, 2-Methyl-1-cyclohexyl, 3-Methyl-1-cyclohexyl oder 4-Methyl-1-cyclohexyl;
   CₙF_{2(n-a)-(1-b)}H_{2a-b} mit n 530, 0 ≤ a ≤ n und b = 0 oder 1;
   C₂-C₃₀-Alkenyl, insbesondere 2-Propenyl, 3-Butenyl, cis-2-Butenyl oder trans-2-Butenyl;
   CₙF_{2(n-a)-(1-b)}H_{2a-b} mit n ≤30, 0 ≤ a ≤ n und b = 0 oder 1;
   C₃-C₁₂-Cycloalkenyl, insbesondere 3-Cyclopentenyl, 2-Cyclohexenyl, 3-Cyclohexenyl oder 2,5-Cyclohexadienyl;
   CₙF_{2(n-a)-(1-b)}H_{2a-b} mit n ≤30, 0 ≤ a ≤ n und b = 0 oder 1;
   Aryl oder Heteroaryl mit 2 bis 30 Kohlenstoffatomen, insbesondere Phenyl, 1-Naphthyl, 2-Naphthyl, 1-Pyrrolyl, 2-Pyrrolyl, 3-Pyrrolyl, 2-Pyridinyl, 3-Pyridinyl oder 4-Pyridinyl;
   Aryl oder Heteroaryl mit 6 bis 30 Kohlenstoffatomen, welche durch ein bis drei C₁-C₆-Alkyl- oder Phenyl-Reste substituiert sind, insbesondere 2-Methyl-phenyl (2-Tolyl), 3-Methyl-phenyl (3-Tolyl), 4-Methyl-phenyl, 2-Ethyl-phenyl, 3-Ethyl-phenyl, 4-Ethyl-phenyl, 2,3-Dimethyl-phenyl, 2,4-Dimethyl-phenyl, 2,5-Dimethyl-phenyl, 2,6-Dimethyl-phenyl, 3,4-Dimethyl-phenyl, 3,5-Dimethyl-phenyl oder 4-Phenyl-phenyl; oder
   C₆F₍₅₋ₐ₎Hₐ mit 0≤ a ≤ 5; stehen;
R^{e}, R^{f}, R^{g}, R^{h} unabhängig voneinander für Wasserstoff,C₁-C₆-Alkyl, Aryl-, Heteroaryl-, C₃-C₇-Cycloalkyl, Halogen, OR^{c} SR^{c}, NR^{c}R^{d}, COOR^{c}, CO-NR^{c}R^{d} oder COR^{c} substituiert ist, wobei R^{c}, R^{d} unabhängig voneinander für Wasserstoff, C₁-C₆-Alkyl, C₁-C₆-Halogenalkyl, Cyclopentyl, Cyclohexyl, Phenyl, Tolyl oder Benzyl stehen;
   vorzugsweise für
   Wasserstoff, Halogen oder C₁-C₆-Alkyl, insbesondere Wasserstoff oder C₁-C₆-Alkyl;
R für Wasserstoff, C₁-C₃₀-Alkyl, C₃-C₁₂-Cycloalkyl, C₂-C₃₀-Alkenyl, C₃-C₁₂-Cycloalkenyl, Aryl oder Heteroaryl, wobei die 6 letztgenannten Reste ein oder mehrere Halogenreste tragen können und/oder 1 bis 3 Reste ausgewählt aus der Gruppe C₁-C₆-Alkyl, Aryl, Heteroaryl, C₃-C₇-Cycloalkyl, Halogen, OR^{c}, SR^{c}, NR^{c}R^{d}, COR^{c}, COOR^{c}, CO-NR^{c}R^{d}, wobei R^{c} und R^{d} für Wasserstoff, C₁-C₆-Alkyl, C₁-C₆-Halogenalkyl, Cyclopentyl, Cyclohexyl, Phenyl, Tolyl oder Benzyl steht.
   vorzugsweise für
   C₁-C₃₀-Alkyl, insbesondere Methyl, Ethyl, 1-Propyl, 2-Propyl, 1-Butyl, 2-Butyl, 2-Methyl-1-propyl (Isobutyl), 2-Methyl-2-propyl (tert.-Butyl), 1-Pentyl, 2-Pentyl, 3-Pentyl, 2-Methyl-1-butyl, 3-Methyl-1-butyl, 2-Methyl-2-butyl, 3-Methyl-2-butyl, 2,2-Dimethyl-1-propyl, 1-Hexyl, 2-Hexyl, 3-Hexyl, 2-Methyl-1-pentyl, 3-Methyl-1-pentyl, 4-Methyl-1-pentyl, 2-Methyl-2-pentyl, 3-Methyl-2-pentyl, 4-Methyl-2-pentyl, 2-Methyl-3-pentyl, 3-Methyl-3-pentyl, 2,2-Dimethyl-1-butyl, 2,3-Dimethyl-1-butyl, 3,3-Dimethyl-1-butyl, 2-Ethyl-1-butyl, 2,3-Dimethyl-2-butyl, 3,3-Dimethyl-2-butyl, Heptyl, Octyl, Nonyl, Decyl, Undecyl, Dodecyl, Tridecyl, Tetradecyl, Pentadecyl, Hexadecyl, Heptadecyl, Octadecyl, Nonadecyl, Icosyl, Henicosyl, Docosyl, Tricosyl, Tetracosyl, Pentacosyl, Hexacosyl, Heptacosy, Octacosyl, Nonacosyl oder Triacontyl; C₁-C₆-Alkyl, welches durch Phenyl oder C₅-C₇-Cycloalkyl substituiert ist, insbesondere Phenylmethyl (Benzyl), Diphenylmethyl, Triphenylmethyl, 2-Phenylethyl, 3-Phenylpropyl, Cyclopentylmethyl, 2-Cyclopentylethyl, 3-Cyclopentylpropyl, Cyclohexylmethyl, 2-Cyclohexylethyl oder 3-Cyclohexylpropyl; CₙF_{2(n-a)-(1-b})H_{2a-b} mit n ≤30, 0 ≤ a ≤ n und b = 0 oder 1, insbesondere CF₃, C₂F₅, C₆F₁₃, C₈F₁₇, C₁₀F₂₁ oder C₁₂F₂₅; C₃-C₁₂-Cycloalkyl, insbesondere Cyclopentyl oder Cyclohexyl; C₃-C₁₂-Cycloalkyl, welches durch C₁-C₆-Alkyl substituiert ist, insbesondere 2-Methyl-1-cyclopentyl, 3-Methyl-1-cyclopentyl, 2-Methyl-1-cyclohexyl, 3-Methyl-1-cyclohexyl oder 4-Methyl-1-cyclohexyl; CₙF_{2(n-a)-(1-b)}H_{2a-b}mit n ≤30, 0 ≤ a ≤ n und b = 0 oder 1; C₂-C₃₀-Alkenyl, insbesondere 2-Propenyl, 3-Butenyl, cis-2-Butenyl oder trans-2-Butenyl; CₙF_{2(n-a)-(1-b)}H_{2a-b} mit n ≤30, 0 ≤ a ≤ n und b = 0 oder 1; C₃-C₁₂-Cycloalkenyl, insbesondere 3-Cyclopentenyl, 2-Cyclohexenyl, 3-Cyclohexenyl oder 2,5-Cyclohexadienyl; CₙF_{2(n-a)-(1-b)}H_{2a-b} mit n ≤30, 0 ≤ a ≤ n und b = 0 oder 1; Aryl oder Heteroaryl mit 2 bis 30 Kohlenstoffatomen, insbesondere Phenyl, 1-Naphthyl, 2-Naphthyl, 1-Pyrrolyl, 2-Pyrrolyl, 3-Pyrrolyl, 2-Pyridinyl, 3-Pyridinyl oder 4-Pyridinyl; Aryl oder Heteroaryl mit 6 bis 30 Kohlenstoffatomen, welche durch ein bis drei C₁-C₆-Alkyl- oder Phenyl-Reste substituiert sind, insbesondere 2-Methyl-phenyl (2-Tolyl), 3-Methyl-phenyl, (3-Tolyl), 4-Methyl-phenyl, 2-Ethyl-phenyl, 3-Ethyl-phenyl, 4-Ethyl-phenyl, 2,3-Dimethyl-phenyl, 2,4-Dimethyl-phenyl, 2,5-Dimethyl-phenyl, 2,6-Dimethyl-phenyl, 3,4-Dimethyl-phenyl, 3,5-Dimethyl-phenyl oder 4-Phenyl-phenyl; oder C₆F₍₅₋ₐ₎Hₐ mit 0≤ a ≤ 5; stehen.

In einer hierbei besonders bevorzugten Ausgestaltungsform werden nach dem erfindungsgemäßen Verfahren quaternäre Carbonate der Formel I hergestellt, wobei:
R⁴, R⁵, R⁶, R⁷ , R⁸, R^{e}, R^{f}, R^{g}, R^{h} unabhängig voneinander für Wasserstoff, Halogen oder C₁-C₆-Alkyl, vorzugsweise für Wasserstoff, Halogen oder C₁-C₆-Alkyl stehen;
R für C₁-C₆-Alkyl steht; und
R¹, R²' unabhängig voneinander C₁-C₆-Alkyl.

In einer weiteren besonderen Ausgestaltungsform werden nach dem erfindungsgemäßen Verfahren quaternäre Carbonate der Formel I hergestellt, wobei:

[A⁺] ein heteroaromatisches Kation ist ausgewählt aus der Gruppe 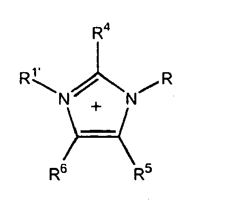 ist.

In einer weiteren bevorzugten Ausgestaltungsform werden nach dem erfindungsgemäßen Verfahren quaternäre Carbonate der Formel I hergestellt, wobei:
[A⁺] ein quaternäres Ammonium-Kation [R^{1'}R¹R²R³N]⁺ oder ein Phosphonium-Kation [R^{1'}R¹R²R³P]⁺ ist.

In einer weiteren Ausgestaltungsform werden in das erfindungsgemäßen Verfahren Alkly- und/oder Arylcarbonate der Formel III eingesetzt, wobei R^{1'} und R^{2'} identisch sind. Vorzugsweise stehen hierbei R¹, und R²' für C₁-C₆-Alkyl.

Als Lewissäure wird üblicherweise eine Lewissäure, welche mindestens ein Element der 1. bis 13. Gruppe des Periodensystems oder der Gruppe der Lanthaniden (Atomnummer 57 bis 71) enthält, eingesetzt. Vorzugsweise enthält die Lewissäure mindestens ein Element ausgewählt aus der Gruppe Lithium, Titan, Zirkon, Vanadium, Niob, Tantal, Chrom, Mangan, Eisen, Cobalt, Nickel, Kupfer, Silber, Gold, Aluminium, Cer, Lanthan, Neodym, Samarium, Gadolinium, Erbium und Lutetium; insbesondere ausgegewählt aus der Gruppe Lithium, Kupfer, Silber, Samarium, Cer, Titan und Aluminium.

Üblicherweise werden als Lewis-Säuren die Oxide, Carbonate, Halogenide oder Silikate der voranstehenden Element eingesetzt, sowie Zeolithe. Besonders bevorzugt sind Oxide, Carbonate, Halogenide oder Silikate von Lithium, Kupfer, Silber, Samarium, Cer, Titan oder Aluminium oder Gemische hiervon, oder Zeolithe.

In der Regel werden 0,001 mol% bis 90 mol%, bevorzugt 0,1 mol% bis 10 mol%, besonders bevorzugt 0,5 mol% bis 5 mol%, bezogen auf das Amin R¹R²R³N der Formel IIa, Phosphin R¹R²R³P der Formel IIb, Sulfid R¹R²S der Formel IIc oder den Heteroaromaten der Formel IId, an Lewissäure eingesetzt.

Die Lewissäuren können im Reaktionsgemisch beispielsweise homogen gelöst, suspendiert oder heterogen als Feststoff vorliegen. Gegebenenfalls können die einzusetzenden Lewissäuren auch auf einem Träger aufgebracht sein. Als geeignete Träger seien beispielsweise metallische Oxide oder Polymere genannt.

Üblicherweise wird das Alkyl- und/oder Arylcarbonat der Formel III in 0,5 bis 10 Molequivalenten, bevorzugt 1,0 bis 5,0 Molequivalenten, besonders bevorzugt 1,1 bis 2,0 Molequivalenten, bezogen auf das Amin R¹R²R³N der Formel IIa, Phosphin R¹R²R³P der Formel IIb, Sulfid R¹R²S der Formel IIc oder den Heteroaromaten der Formel IId, eingesetzt.

Die Reaktion wird in der Regel bei einer Temperatur von 30°C bis 350°C, bevorzugt 50°C bis 150°C, besonders bevorzugt 80°C bis 120°C durchgeführt.

Üblicherweise liegt die Reaktionszeit im Bereich von 0,1 Stunde bis 14 Tagen, bevorzugt 0,5 Stunden bis 5 Tagen, besonders bevorzugt 1 Stunden bis 24 Stunden.

Die Reaktion wird in der Regel bei einem Druck von 0,5bar bis 300bar, bevorzugt 1,0bar bis 50bar, besonders bevorzugt 2,0bar bis 10bar durchgeführt.

Von Fall zu Fall kann es von Vorteil sein, die Umsetzung in Gegenwart eines Reduktionsmittels oder Reduktionsmittelsystems durchzuführen.

In einer Ausgestaltungsform wird das erfindungsgemäße Verfahren in Abwesenheit eines Reduktionsmittels oder Reduktionsmittelsystems durchgeführt.

In einer weiteren Ausgestaltungsform wird das erfindungsgemäße Verfahren in Gegenwart eines Reduktionsmittels oder Reduktionsmittelsystems durchgeführt. Als Reduktionsmittel kommen beispielsweise Aluminiumhydride oder Borhydride, wie NaAlH₄, LiAlH₄, NaBH₄ oder LiBH₄ in Betracht, als Reduktionsmittelsysteme Wasserstoff in Gegenwart eines Katalysators, vorzugsweise ausgewählt aus der Gruppe der Platinmetalle, insbesondere Pt, Pd, Rh, Ru oder Ir.

Die Reaktion kann unter Luft- oder Wasserdampfatmosphäre oder unter SchutzgasAtmosphäre, insbesondere unter Stickstoff oder Argon durchgeführt werden.

Die Umsetzung kann batchweise, semikontinuierlich oder kontinuierlich durchgeführt werden.

Von Fall zu Fall kann es von Vorteil sein, die Umsetzung in Gegenwart eines Lösungsmittels durchzuführen.

In einer Ausgestaltungsform wird das erfindungsgemäße Verfahren in Substanz durchgeführt.

In einer weiteren Ausgestaltungsform wird das erfindungsgemäße Verfahren in Gegenwart eines Lösungsmittels durchgeführt. Hierbei kommen z.B. Alkohole, wie Methanol, Ethanol, Isopropanol, n-Propanol, n-Butanol, sec-Butanol, tert-Butanol, Pentanol, Hexanol, Heptanol, Octanol oder Phenol, Ether, wie diethylether, Methyltert.-butylether, Dioxan oder Tetrahydrofuran, Dialkylformamide, wie z.B. Dimethylformamid oder Diethylformamid, Ketone, wie Dimethylketon oder Dimethylketon, Sulfoxide , wie Dimethylsulfoxid, Nitrile, wie Acetontril, oder Gemische hiervon in Betracht. In der Regel beträgt der Anteil an Lösungsmittel an der Gesamtmasse der Reaktionsmischung 1% bis 90%, bevorzugt 10% bis 50%, besonders bevorzugt mit 20% bis 30%.

In einer besonderen Ausgestaltungsform wird als Lösungsmittel jener Alkohol eingesetzt, welcher zumindest zu einem der Reste des Alkyl- und/oder Arylcarbonats der Formel III korrespondiert.

Die Durchführung der Umsetzung erfolgt nach dem für den Fachmann üblichen Bedingungen. In der Regel wird die Reaktionsmischung gerührt, geschüttelt oder anderweitig durchmischt; durch Heizen oder Kühlen kann die gewünschte Reaktionstemperatur eingestellt werden.

Die Aufarbeitung des Reaktionsgemisches erfolgt ebenfalls nach gängigen Methoden, wie Destillation, Vakuumdestillation, Dünnschichtverdampfung, Kurzwegverdampfung, Rotationsverdampfung, Sprühtrocknung, Osmose, Pervaporation, Strippen mit Gas oder Wasserdampf, Ausfrieren, Gefriertrocknung, chemische oder physikalische Adsorption oder andere geeignete Verfahren.

Weiterhin können durch Extraktion nicht umgesetzte Edukte, die Lewissäure etc. abgetrennt werden. Als Lösungsmittel kommen hier z.B. Kohlenwasserstoffe wie Pentan, Hexan, Heptan, Octan, Nonan, Decan, Petrolether, Benzin, Diesel, Benzol, Toluol, o-Xylol, m-Xylol oder p-Xylol, Ether wie Diethylether, Tetrahydrofuran, Ester wie Ethylacetat, Methylacetat, chlorierte Kohlenwasserstoffe wie Chloroform oder Dichlormethan, oder Gemische hiervon in Betracht, wobei ggf. zusätzlich ein Hilfslösungsmittel wie ein Alkohol, insbesondere wie Methanol, Ethanol, Isopropanol, n-Propanol, n-Butanol, sec-Butanol, tert-Butanol, Pentanol, Hexanol, Heptanol oder Octanol, ein Ether, wie Diethylether oder Tetrahydrofuran, ein Dialkylformamid, wie Dimetylformamid, Diethylformamid, ein Keton, wie Aceton, Methylethylketon, ein Sulfoxid, wie Dimethylsulfoxid, oder ein Nitril, wie Acetonitril, oder ein Gemisches hiervon, zugegeben werden kann.

In einer besonderen Ausgestaltungsform, der Erfindung wird das das Alkyl- und/oder Arylcarbonat der Formel III durch Umsetzung von CO₂, CO oder CO/o₂ oder Gemischen hiervon mit einem Alkohol R¹OH oder R²'OH bzw. einem Gemisch hiervon, in Gegenwart einer Lewissäure, bei einem Druck von 1 bis 300 bar, bevorzugt 5 bis 100 bar, besonders bevorzugt 10 bis 50 bar, erhalten - als Lewissäuren kommen die voranstehend genannten in Betracht - oder durch oxidative, katalytische Reaktion von Alkoholen mit Alkenen oder durch katalytische Reaktion von Oxiranen mit Kohlendioxid. Dies sei nachfolgend an einigen Beispielen dargestellt:

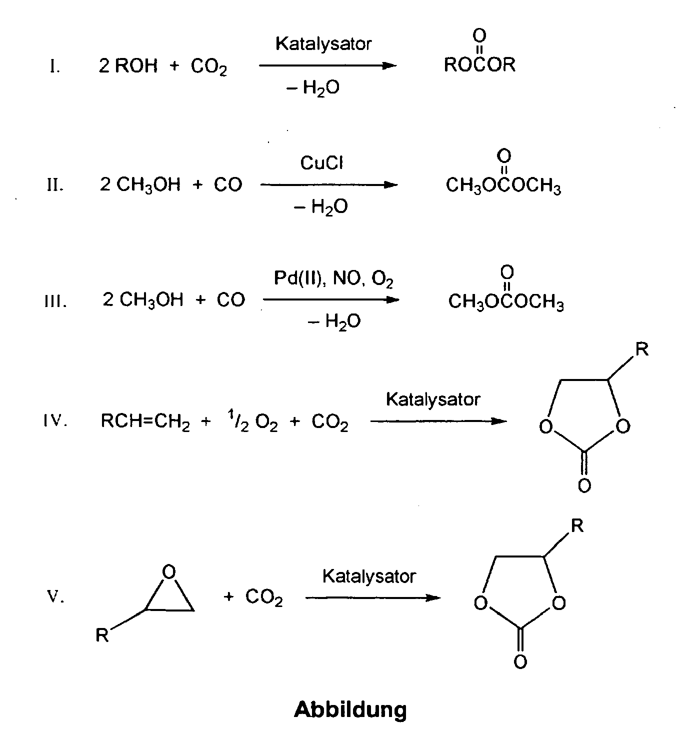

In einer hierbei bevorzugten Ausgestaltungsform wird das Alkyl-und/oder Arylcarbonat der Formel III *in situ* erzeugt. Üblicherweise wird hierbei ein Amin R¹R²R³N der Formel IIa, ein Phosphin R¹R²R³P der Formel IIb, ein Sulfid R¹R²S der Formel IIc oder ein Heteroaromaten der Formel IId, mit einem Alkohol R¹OH oder R²'OH bzw. einem Gemisch hiervon und CO₂, CO/O₂ oder CO und eines Oxidationsmittels, sowie Gemische hiervon, in Gegenwart einer Lewissäure bei 1 bis 300 bar, bevorzugt 5 bis 100 bar, besonders bevorzugt 10 bis 50 bar, umsetzt.

In einer weiteren Ausgestaltungsform wird ein quaternäres Cabonat der Formel I dargestellt, indem ein Amin R¹R²R³N der Formel IIa, ein Phosphin R¹R²R³P der Formel IIb, ein Sulfid R¹R²S der Formel IIc oder ein Heteroaromaten der Formel IId, mit einem Alkohol R¹OH oder R²OH bzw. einem Gemisch hiervon und CO₂, CO/O₂ oder CO und eines Oxidationsmittels, sowie Gemische hiervon, in Gegenwart einer Lewissäure bei 1 bis 300 bar, bevorzugt 5 bis 100 bar, besonders bevorzugt 10 bis 50 bar, umsetzt.

Die Lewissäure wird jeweils in 0,001 mol% bis 90 mol%, bevorzugt 0,1 mol% bis 10 mol%, besonders bevorzugt 0,5 mol% bis 5 mol%, bezogen auf das Amin R¹R²R³N der Formel IIa, das Phosphin R¹R²R³P der Formel IIb, das Sulfid R¹R²S der Formel IIc oder dem Heteroaromaten der Formel IId, eingesetzt.

Bei diesen Umsetzungen wird jeweils üblicherweise der Alkohol R¹'OH oder R^{2'}OH bzw. ein Gemisch hiervon in 0,5 bis 20 Molequivalenten, bevorzugt 1,0 bis 5 Molequivalenten, besonders bevorzugt 1,5 bis 2,5 Molequivalenten, bezogen auf das Amin R¹R²R³N der Formel IIa Phosphin R¹R²R³P der Formel IIb, Sulfid R¹R²S der Formel IIc oder den Heteroaromaten der Formel IId , eingesetzt.

In einer Ausgestaltungsform der vorliegenden Erfindung wird das benötigte CO₂ bzw. das für die Erzeugung des Alkyl- und/oder Arylcarbonats der Formel III benötigte Kohlendioxid aus der Reaktion eines quaternären Carbonats der Formel I und/oder einer quartären Verbindung der Formel IVa und/oder IV b 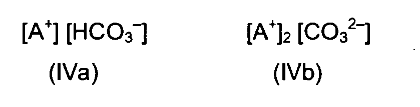 mit einer Brønsted-Säure HZ generiert. Das dabei entstehende Kohlendioxid wird also in einen Kreislauf rückgeführt. Dies sei in nachfolgender Abbildung am Beispiel der Synthese eines 1,3-Dialkylimidazolium Salzes veranschaulicht:

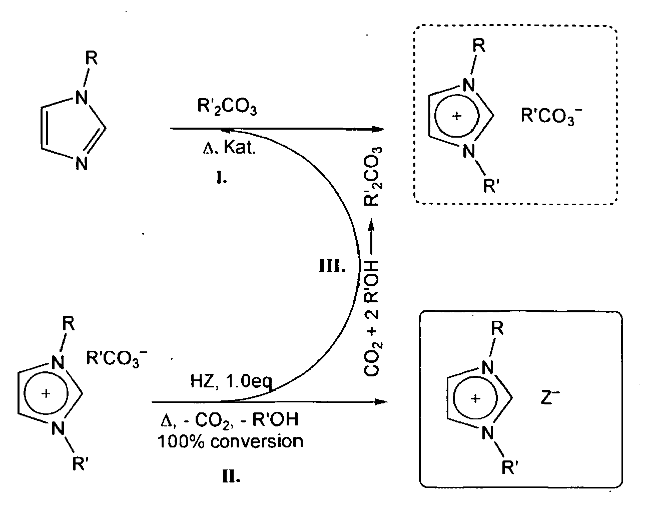

In einer weiteren Ausgestaltungsform der vorliegenden Erfindung wird der zur Erzeugung des Alkyl- und/oder Arylcarbonats der Formel I benötigte Alkohol R^{2'}OH durch die Umsetzung eine quaternären Carbonats der Formel I mit einer Bronsted-Säure HZ generiert. Der dabei entstehende Alkohol R²OH wird also in einem Kreislauf rückgeführt.

Die Umsetzungen der vorliegenden Erfindungen können je nach eingestellter Temperatur und eingestellten Druck in flüssiger Phase oder in Gasphase durchgeführt werden.

Die Aufarbeitung der Reaktionsgemische erfolgt nach für den Fachmann gängigen Methoden, beispielsweise durch Destillation, Vakuumdestillation, Dünnschichtverdampfung, Kurzwegverdampfung, Rotationsverdampfung, Sprühtrocknung, Osmose, Pervaporation, Strippen mit Gas oder Wasserdampf, Ausfrieren, Gefriertrocknung, chemische oder physikalische Adsorption oder andere geeignete Verfahren.

Weiterhin können durch Extraktion nicht umgesetzte Edukte, die Lewissäure etc. abgetrennt werden. Als Lösungsmittel kommen hier z.B. Kohlenwasserstoffe wie Pentan, Hexan, Heptan, Octan, Nonan, Decan, Petrolether, Benzin, Diesel, Benzol, Toluol, o-Xylol, m-Xylol oder p-Xylol, Ether wie Diethylether, Tetrahydrofuran, Ester wie Ethylacetat, Methylacetat, chlorierte Kohlenwasserstoffe wie Chloroform oder Dichlormethan, oder Gemische hiervon in Betracht, wobei ggf. zusätzlich ein Hilfslösungsmittel wie ein Alkohol, insbesondere wie Methanol, Ethanol, Isopropanol, n-Propanol, n-Butanol, sec-Butanol, tert-Butanol, Pentanol, Hexanol, Heptanol oder Octanol, ein Ether, wie Diethylether oder Tetrahydrofuran, ein Dialkylformamid, wie Dimetylformamid, Diethylformamid, ein Keton, wie Aceton, Methylethylketon, ein Sulfoxid, wie Dimethylsulfoxid, oder ein Nitril, wie Acetonitril, oder ein Gemisches hiervon, zugegeben werden kann.

Die Erfindung betrifft ferner ein Verfahren zur Herstellung einer Verbindungen der Formel V,

[A⁺]n[Zⁿ⁻] (V)

wobei
[A⁺] die in den Ansprüchen 1 bis 33 genannte Bedeutung hat, und [Zⁿ⁻] für ein ein-, zwei- drei- oder vierwertiges Anion;
steht;
dadurch gekennzeichnet, dass man quaternäres Carbonat der Formel I nach einem der voranstehend beschiebenen Ausgestaltungsformen herstellt und das so erhaltene Carbonat der Formel I mit einer Broensted-Säure [H⁺]ₙ[Zⁿ⁻], dessen Anhydrid oder einem Salz [Kation^{m+}]ₙ[Zⁿ-]ₘ umsetzt.

Die Verbindungen der Formel V *kann beispielsweise wie folgt durchgeführt werden:*

### Anionenaustausch durch Reaktion mit einer Brønsted-Säure:

Das gewünschte Anion [Zⁿ⁻] wird durch Reaktion des quaternäres Carbonat der Formel I mit der zum Anion [Zⁿ⁻] konjugierten Brønsted-Säure HₙZ eingeführt, wobei Kohlendioxid freigesetzt wird.

### Anionenaustausch via Metathese:

Alternativ zu der voranstehend genannten Reaktion mit einer Brønsted-Säure HₙZ, kann auch eine Metathese durchgeführt werden, wobei das quaternäres Carbonat der Formel I mit einem Metallsalz [Kation^{m+}]ₙ[Zⁿ-]ₘ = [M^{m+}]ₙ[Zⁿ⁻]ₘ, umgesetzt wird, wobei das Kation des Metallsalzes M^{m+} ein schwerlösliches M^{m}+ -Hydrogencarbonat und/oder M^{m+}-Carbonat bildet, welches ausfällt und durch Filtration oder Zentrifugation entfernt wird, sodaß die gewünschte Verbindung der Formel V zurückbleibt und isoliert werden kann. Typische Metalle M^{m+} sind z.B. Calcium, Magnesium, Zink, Mangan etc. Diese Reaktion verläuft üblicherweise nahezu quantitativ in wäßriger Lösung.

### Anionenaustausch mittels Flüssigionenaustausch:

Eine weitere Möglichkeit besteht in der Reaktion eines langkettigen qautären Carbonats der Formel I, mit einer C₄-C₃₀-, bevorzugt C₆-C₁₆-, besonders bevorzugt C₈-C₁₂-Alkylkette, welche ggf. verzweigt, funktionalisiert oder substituiert sein kann, mit einem Salz [Kation^{m+}]ₙ[Zⁿ⁻]ₘ; hierbei werden wasserunlösliche Verbindungen der Formel V gebildet, welche sich abscheiden und abgetrennt werden können.

Die Erfindung betrifft ferner quaternäre Carbonate der Formel I, welche nach dem erfindungsgemäßen Verfahren hergestellt sind.

### Beispiele:

Im Folgenden wird die Erfindung durch einige typische Beispiele näher beschrieben. ¹H-Kernmagnetresonanzspektren (¹H-NMR) wurden auf einem Bruker Avance DPX 400 in d⁶-DMSO und D₂O gemessen, Gehaltsbestimmungen mit NMR werden mit G(NMR) abgekürzt. Elektrospray-Ionisations-Massenspektren (ESI-MS) wurden auf einem Bruker Esquire 3000 in wäßrig-methanolischer Lösung im positiv und negativ Mode gemessen, acidimetrische Gehaltsbestimmungen, abgekürzt mit G(T), wurden mit einem Metrohm Basic Titrino 794 mit gestellter 1 N Salzsäure durchgeführt. Die relative molare Masse wird mit "Mᵣ" abgekürzt und in g/mol angegeben, das Molequivalent wird mit "equiv." abgekürzt. Sämtliche Ausgangschemikalien wurden bei Sigma-Aldrich bezogen.

### a. Darstellung von 1-Ethyl-3-methylimidazolium-methylcarbonat:

Am Beispiel der Synthese von 1-Ethyl-3-methylimidazolium-carbonat durch Reaktion von N-Ethylimidazol und Dimethylcarbonat in Methanol soll die Auswirkung verschiedener typischer Katalysatoren im Vergleich zur unkatalysierten Reaktion demonstriert werden: Je 10g N-Ethylimidazol (CAS 7098-07-9; 0,104mol), 10g Dimethylcarbonat (CAS 616-38-6; 1,07 equiv.) und 10g Methanol (33 massen% der Reaktionsmischung) wurden ohne Katalysator bzw. mit je 0,5g Katalysator Lithiumchlorid (11 mol%), Samarium-(III)-oxid (1,4 mol%) und Aluminiumnatriumsilikat P 820 A (CAS 1344-00-9; ca. 7 mol%) versetzt und bei einer Temperatur von 100°C bei einem Systemeigendruck von ca. 10bar in einem Druckreaktor heftig gerührt.

Die Abnahme der Konzentration des N-Ethylimidazols bzw. die Zunahme des Carbonatgehaltes wurde alle zwei Stunden durch acidimetrische Titration (Trennung durch Fällung als Calciumcarbonat) bestimmt:

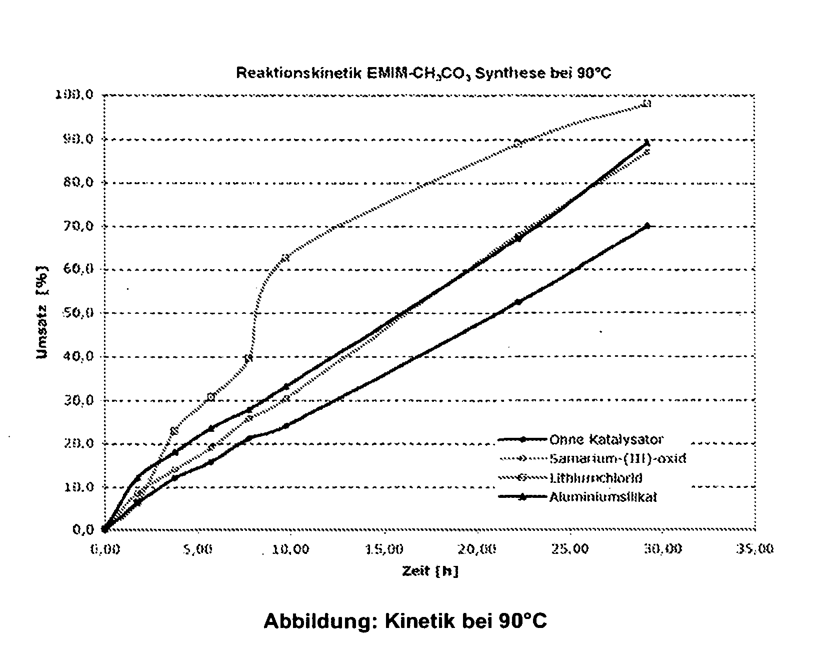

Die voranstehende Abbildung zeigt deutlich den katalytischen Effekt der homogenen Lewissäure Lithiumchlorid und der heterogenen Lewissäuren Samariumoxid und Aluminiumsilikat im Vergleich zur nicht katalysierten Reaktion: Man sieht z.B. daß nach einer Reaktionszeit von 12 Stunden bei 90°C unter Lithiumchlorid Katalyse bereits 70% Umsatz erzielt wird, während die nicht katalysierte Reaktion unter den gleichen Reaktionsbedingungen lediglich 30% Umsatz erzielt, bzw. für 70% Umsatz 29 Stunden braucht. 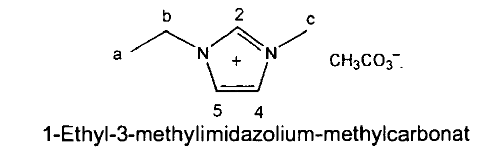 Summenformel: C₈H₁₄N₂O₃; Mᵣ = 186,1g/mol; ¹H-NMR (δ-Werte, 400MHz, d⁶-DMSO): 1,416 (3H, t, J_{a,b}=7,30HZ: Hₐ); 3,170 (3H, s: CH₃CO₃ ); 3,856 (3H, s: H_{c}); 4,199 (2H, q, J_{b,a}=7,31Hz: H_{b}); 7,716 (1H, t, J_{5,4} = J_{5,2} = 1,2Hz, B-Teil eines ABX-Systems: H₅*); 7.801 (1H, t, J_{4,5} = J_{4,2} = 1,5Hz, A-Teil eines ABX-Systems: H₄*); 9.252 (1H, s, Triplett nicht aufgelöst, X-Teil eines ABX-Systems: H₂);* Zuordnung vertauschbar; ESI-MS (+ mode, m/z): 111, 1 (K⁺) ; (- mode, m/z) : 75,0 (A⁻)

### b. Darstellung von Tetraethylammonium-Ethylcarbonat

30g Triethylamin (CAS 121-44-8; 0,30mol), 35g Diethylcarbonat (CAS 105-58-8; 1,2 equiv.) und 28g Ethanol wurden mit 0,16g Lithium-ethoxid (CAS 2388-07-0, 1mol%) versetzt und bei 140°C in einem Druckreaktor unter einem Systemeigendruck von ca. 10bar für 5 Tage heftig gerührt. Nach Abkühlen und Entfernung des Lösungsmittels und der nicht abreagierten Edukte *in vacuo* wurden 16,4g Tetraethylammonium-ethylcarbonat (25% der Theorie) als beige Kristalle erhalten, welche zur Charakterisierung aus Acetonitril/Ethylacetat unkristallisiert wurden.

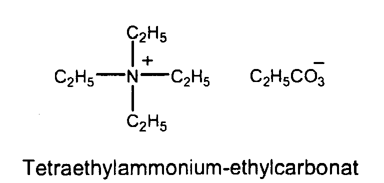

Summenformel: C₁₁H₂₅NO₃; M = 219,2g/mol; ¹H-NMR (δ-Werte, 400MHz, D₂O): 1.104 (3H, t, J=7,30Hz: CH₃CH₂CO₃⁻); 1,177 (12H, t, J=7,28Hz: (CH₃CH₂)₄N⁺); 2.891 (2H, q, J =7,30Hz: CH₃CH₂CO₃⁻); 3,173 (8H, q, J =7,28Hz, (CH₃CH₂)₄N⁺); ESI-MS (+ mode, m/z): 130.2 (K⁺); (-mode, m/z): 89.0 (A⁻) ; G(T)=99%

### c. Darstellung von Triethylmethylammonium-Methylcarbonat unter in situ Erzeugung des Dimethylcarbonats

30g Triethylamin (CAS 121-44-8; 0,30mol), 30g Methanol (CAS 67-56-1; 3 equiv.) wurden in einem Druckreaktor mit 1 g eines Aluminiumoxid-Zirkondioxid-Siliziumdioxid Mischkatalysators versetzt, mit 68 bar Kohlendioxid beaufschlagt und bei 150°C für 5 Tage heftig gerührt. Nach Abkühlen und Entfernung des Lösungsmittels und der nicht abreagierten Edukte *in vacuo* wurden 5,2g Triethylmethylammonium-Methylcarbonat (9,1% der Theorie) als beige Kristalle erhalten, welche zur Charakterisierung aus Acetonitril/Ethylacetat unkristallisiert wurden. 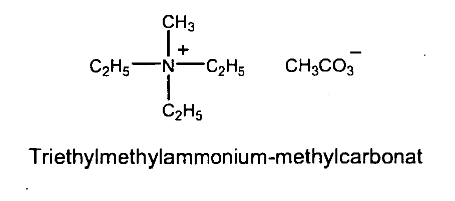 Summenformel: C₉H₂₁NO₃; Mᵣ = 191,2g/mol; ¹H-NMR (δ-Werte, 400MHz, d⁶-DMSO): 1,197 (9H, t, J=7,26Hz: (CH₃CH₂)₃N⁺CH₃); 2,888 (3H, s: CH₃CO₃); 3,163 (3H, s: (CH₃CH₂)₃N⁺CH₃); 3,265 (6H, q, J =7,26Hz: (CH₃CH₂)₃N⁺CH₃); ESI-MS (+ mode, m/z): 116,1 (K⁺); (- mode, m/z): 75,0 (A⁻) ; G(T)=99%

### d. Darstellung von Triethylmethylammonium-Methylcarbonat (in situ, Gasphase)

30g Triethylamin (CAS 121-44-8; 0,30mol), 30g Methanol (CAS 67-56-1; 3 equiv.) wurden in einem 5L Autoklaven, welcher 1g eines Aluminiumoxid-Zirkondioxid-Siliziumdioxid Mischkatalysators enthielt, mit 20 bar Kohlendioxid beaufschlagt und bei 160°C verdampft. Nach einer Reaktionszeit von 7 Tagen, Abkühlen und Entspannen des CO₂-Überducks wurden 2,2g Triethylmethylammonium-Methylcarbonat (3,8% der Theorie) als beige Kristalle erhalten, welche teilweise am Katalysator anhafteten und zur Charakterisierung aus Acetonitril/Ethylacetat unkristallisiert wurden.

## Patentansprüche

1. Verfahren zur Herstellung von quaternären Carbonaten der Formel I,
[A]⁺ [R²'OCO₂]⁻ (I)
in der
[A]⁺ für ein quaternäres Ammonium-Kation [R^{1'}R¹R²R³N]⁺, ein Phosphonium-Kation [R^{1'}R¹R²R³P]⁺, ein Sulfonium-Kation [R^{1'}R¹R²S]⁺ oder ein heteroaromatisches
Kation steht,
R¹, R², R³ unabhängig voneinander für Wasserstoff, ggf. sub. Alkyl, Alkenyl, Alkinyl,
Cycloalkyl, Cycloalkenyl, Aryl oder Heteroaryl stehen; oder
zwei der Reste R¹, R², R³ bilden gemeinsam mit dem Heteroatom, an welchem sie
gebunden sind einen Ring aus, wobei dieser gesättigt oder ungesättigt, unsubstituiert oder substituiert ist und wobei diese Kette durch ein oder mehrere Heteroatome ausgewählt aus der Gruppe O, S, NH oder N-C₁-C₄-Alkyl unterbrochen sein kann;
R^{1'}, R^{2'} unabhängig voneinander für ggf. sub. Alkyl, Alkenyl, Alkinyl, Cycloalkyl, Cycloalkenyl, Aryl oder Heteroaryl stehen; oder
R^{1'} und R^{2'} bilden gemeinsam eine -CHR^{a}-CHR^{b}-Kette, -CHR^{a}-CH₂-CHR^{b}-Kette oder
ggf. substuierten -C₆H₄-1,2-diyl-Rest stehen, wobei R^{a} und R^{b} unabhängig voneinander für Wasserstoff oder C₁-C₆-Alkyl; wobei in diesem Fall eine zwitterionische Verbindung der Formel I erhalten wird, bei der R^{1'} und [R²'OCO₂]⁻ den Rest -OCO-CHR^{a}-CHR^{b}-, -OCO-CHR^{a}-CH₂-CHR^{b}- oder ggf. sub. -OCO-1-C₆H₄-2-yl- ausbilden (Verbindung I');
**dadurch gekennzeichnet.**
**daß** man ein Amin R¹R²R³N der Formel IIa, Phosphin R¹R²R³P der Formel IIb, Sulfid R¹R²S der Formel IIc oder einen Heteroaromat der Formel IId, wobei die Reste R¹, R² und R³ die oben genannte Bedeutung haben,
mit einem Alkyl-und/oder Arylcarbonat der Formel III
R^{1'}O(CO)OR²' (III)
wobei
R^{1'}, R²' unabhängig voneinander für ein ggf. sub. Alkyl, Alkenyl, Alkinyl, Cycloalkyl,
Cycloalkenyl, Aryl oder Heteroaryl stehen; oder
R^{1'} und R²' gemeinsam eine -CHR^{a}-CHR^{b}-Kette, -CHR^{a}-CH₂-CHR^{b}-Kette oder ggf.
substuierten -C₆H₄-1,2-diyl-Rest bilden, wobei R^{a} und R^{b} unabhängig voneinander für Wasserstoff oder C₁-C₆-Alkyl stehen; wobei in diesem Fall eine
zwitterionische Verbindung der Verbindung der Formel l'erhalten wird;
in Gegenwart katalytischer Mengen an einer Lewis-Säure umsetzt.

2. Verfahren nach Anspruch 1, **dadurch gekennzeichnet, daß** ein quaternäres Carbonat der Formel I hergestellt wird, wobei
[A]⁺ für ein quaternäres Ammonium-Kation [R^{1'}R¹R²R³N]⁺, ein Phosphonium-Kation [R^{1'}R¹R²R³P]⁺, ein Sulfonium-Kation [R^{1'}R¹R²S]⁺ oder ein heteroaromatisches Kation steht, das ausgewählt ist aus der Gruppe 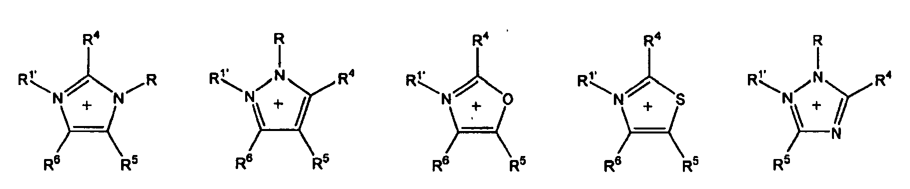 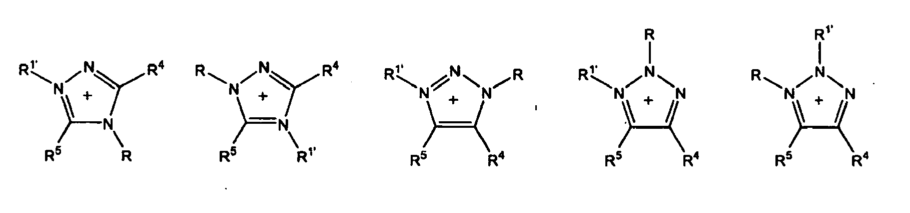 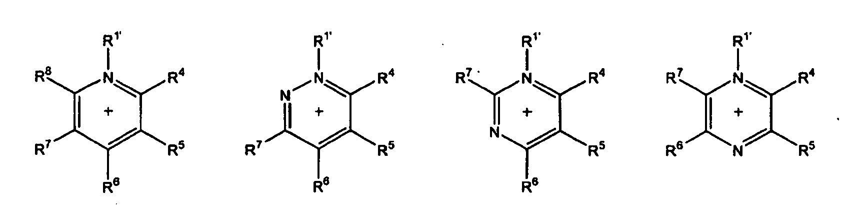 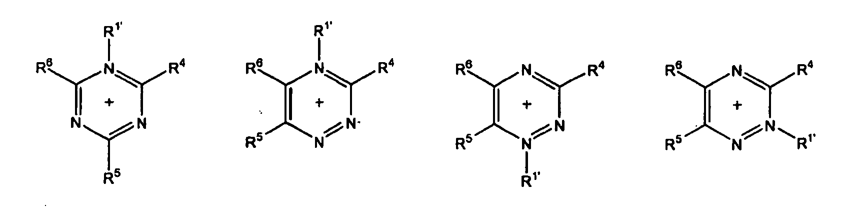 wobei die Reste folgende Bedeutung haben:
R^{1'} ggf. sub. Alkyl, Alkenyl, Alkinyl, Cycloalkyl, Cycloalkenyl, Aryl oder Heteroaryl;
R Wasserstoff, ggf. sub. Alkyl, Alkenyl, Alkinyl, Cycloalkyl, Cycloalkenyl, Aryl oder
Heteroaryl;
R⁴, R⁵, R⁶, R⁷, R⁸ Wasserstoff, Halogen, Nitro, Cyano, OR^{c}, SR^{c}, NR^{c}R^{d}, COR^{c}, COOR^{c},
CO-NR^{c}R^{d}, C₁-C₃₀-Alkyl, C₃-C₁₂-Cycloalkyl, C₂-C₃₀-Alkenyl, C₃-C₁₂-Cycloalkenyl, Aryl oder Heteroaryl, wobei die 6 letztgenannten Reste ein oder mehrere Halogenreste tragen können und/oder 1 bis 3 Reste ausgewählt aus der Gruppe C₁-C₆-Alkyl, Aryl, Heteroaryl, C₃-C₇-Cycloalkyl, Halogen, OR^{c} SR^{c}, NR^{c}R^{d}, COR^{c}, COOR^{c}, CO-NR^{c}R^{d}, wobei R^{c} und R^{d} unabhängig voneinander für Wasserstoff, C₁-C₆-Alkyl, C₁-C₆-Halogenalkyl, Cyclopentyl, Cyclohexyl, Phenyl, Tolyl oder Benzyl stehen; oder
zwei der Reste R, R⁴, R⁵, R⁶, R⁷, R⁸, welche benachbart sind, bilden gemeinsam mit
dem Atom, an welchem sie gebunden sind einen Ring aus, wobei dieser ungesättigt oder aromatisch, unsubstituiert oder substituiert ist und wobei die durch die betreffenden Reste gebildetete Kette durch ein oder mehrere Heteroatome ausgewählt aus der Gruppe O, S, N, NH oder N-C₁-C₄-Alkyl unterbrochen sein kann; oder
im Fall der Verbindungen I' stehen R¹' und R^{2,} gemeinsam für eine -CHR^{a}-CHR^{b}-Kette, -CHR^{a}-CH₂-CHR^{b}-Kette oder ggf. substuierten -C₆H₄-1,2-diyl-Rest, wobei R^{a} und R^{b} unabhängig voneinander für Wasserstoff oder C₁-C₆-Alkyl stehen.

3. Verfahren nach den Anspruch 1 oder 2, **dadurch gekennzeichnet, daß** ein quaternäres Carbonat der Formel I dargestellt wird, wobei
R¹, R², R³ unabhängig voneinander für Wasserstoff, C₁-C₃₀-Alkyl, C₃-C₁₂-Cycloalkyl, C₂-C₃₀-Alkenyl, C₃-C₁₂-Cycloalkehyl, C₂-C₃₀-Alkinyl, Aryl oder Heteroaryl, wobei die 7 letztgenannten Reste ein oder mehrere Halogenreste tragen können und/oder 1 bis 3 Reste ausgewählt aus der Gruppe C₁-C₆-Alkyl, Aryl, Heteroaryl, C₃-C₇-Cycloalkyl, Halogen, OR^{c} SR^{c}, NR^{c}R^{d}, COR^{c}, COOR^{c}, CO-NR^{c}R^{d}, und wobei R^{c} und R^{d} für Wasserstoff, C₁-C₆-Alkyl, C₁-C₆-Halogenalkyl, Cyclopentyl, Cyclohexyl, Phenyl, Tolyl oder Benzyl stehen; oder
zwei der Reste R¹, R², R³ gemeinsam mit dem Heteroatom, an welchem sie gebunden
sind einen Ring ausbilden, wobei dieser gesättigt oder ungesättigt, unsubstituiert oder substituiert ist und wobei diese Kette durch ein oder mehrere Heteroatome ausgewählt aus der Gruppe O, S, NH oder N-C₁-C₄-Alkyl unterbrochen sein kann;
R⁴, R⁵, R⁶, R⁷, R⁸ unabhängig voneinander für Wasserstoff, Halogen, Nitro, Cyano, OR^{c},
SR^{c}, NR^{c}R^{d}, COR^{c}, COOR^{c}, CO-NR^{c}R^{d}, C₁-C₃₀-Alkyl, C₃-C₁₂-Cycloalkyl, C₂-C₃₀-Alkenyl, C₃-C₁₂-Cycloalkenyl, Aryl oder Heteroaryl, wobei die 6 letztgenannten Reste ein oder mehrere Halogenreste tragen können und/oder 1 bis 3 Reste ausgewählt aus der Gruppe C₁-C₆-Alkyl, Aryl, Heteroaryl, C₃-C₇-Cycloalkyl, Halogen, OR^{c}, SR^{c}, NR^{c}R^{d}, COR^{c}, COOR^{c}, CO-NR^{c}R^{d}, und wobei R^{c} und R^{d} für Wasserstoff, C₁-C₆-Alkyl, C₁-C₆-Halogenalkyl, Cyclopentyl, Cyclohexyl, Phenyl, Tolyl oder Benzyl stehen; oder
zwei der Reste R, R⁴, R⁵, R⁶, R⁷, R⁸, welche benachbart sind, bilden gemeinsam mit
dem Atom, an welchem sie gebunden sind einen Ring aus, wobei dieser ungesättigt oder aromatisch, unsubstituiert oder substituiert ist und wobei die durch die betreffenden Reste gebildetete Kette durch ein oder mehrere Heteroatome ausgewählt aus der Gruppe O, S, N, NH oder N-C₁-C₄-Alkyl unterbrochen sein kann; oder
R für Wasserstoff, C₁-C₃₀-Alkyl, C₃-C₁₂-Cycloalkyl, C₂-C₃₀-Alkenyl,
C₃-C₁₂-Cycloalkenyl, C₂-C₃₀-Alkinyl, Aryl oder Heteroaryl, wobei die 7 letztgenannten Reste ein oder mehrere Halogenreste tragen können und/oder 1 bis 3 Reste ausgewählt aus der Gruppe C₁-C₆-Alkyl, Aryl, Heteroaryl, C₃-C₇-Cycloalkyl, Halogen, OR^{c}, SR^{c}, NR^{c}R^{d}, COR^{c}, COOR^{c}, CO-NR^{c}R^{d}, wobei R^{c} und R^{d} für Wasserstoff, C₁-C₆-Alkyl, C₁-C₆-Halogenalkyl, Cyclopentyl, Cyclohexyl, Phenyl, Tolyl oder Benzyl steht;
R¹, R²' unabhängig voneinander für ein C₁-C₃₀-Alkyl, C₃-C₁₂-Cycloalkyl, C₂-C₃₀-Alkenyl,
C₃-C₁₂-Cycloalkenyl, C₂-C₃₀-Alkinyl, Aryl oder Heteroaryl, wobei die 7 letztgenannten Reste ein oder mehrere Halogenreste tragen können und/oder 1 bis 3 Reste ausgewählt aus der Gruppe C₁-C₆-Alkyl, Aryl, Heteroaryl, C₃-C₇-Cycloalkyl, Halogen, OR^{c}, SR^{c}, NR^{c}R^{d}, COR^{c}, COOR^{c}, CO-NR^{c}R^{d}, wobei R^{c} und R^{d} für Wasserstoff, C₁-C₆-Alkyl, C₁-C₆-Halogenalkyl, Cyclopentyl, Cyclohexyl, Phenyl, Tolyl oder Benzyl stehen; oder
R^{1'} und R²' gemeinsam eine -CHR^{a}-CHR^{b}-Kette, -CHR^{a}-CH₂-CHR^{b}-Kette oder ggf.
substuierten -C₆H₄-1,2-diyl-Rest bilden, wobei R^{a} und R^{b} unabhängig voneinander für Wasserstoff oder C₁-C₆-Alkyl stehen;

4. Verfahren nach einem der Ansprüche 1 bis 3, **dadurch gekennzeichnet, daß** ein quaternäres Carbonat der Formel I dargestellt wird, wobei
R¹, R², R³ unabhängig voneinander für Wasserstoff, C₁-C₃₀-Alkyl, C₃-C₁₂-Cycloalkyl,
C₂-C₃₀-Alkenyl, C₃-C₁₂-Cycloalkenyl, Aryl oder Heteroaryl, wobei die 6 letztgenannten Reste ein oder mehrere Halogenreste tragen können und/oder 1 bis 3 Reste ausgewählt aus der Gruppe C₁-C₆-Alkyl, Aryl, Heteroaryl, C₃-C₇-Cycloalkyl, Halogen, OR^{c}, SR^{c}, NR^{c}R^{d}, COR^{c}, COOR^{c}, CO-NR^{c}R^{d}, und wobei R^{c} und R^{d} für Wasserstoff, C₁-C₆-Alkyl, C₁-C₆-Halogenalkyl, Cyclopentyl, Cyclohexyl, Phenyl, Tolyl oder Benzyl stehen;
vorzugsweise für
Wasserstoff;
C₁-C₃₀-Alkyl, insbesondere Methyl, Ethyl, 1-Propyl, 2-Propyl, 1-Butyl, 2-Butyl, 2-Methyl-1-propyl (Isobutyl), 2-Methyl-2-propyl (tert.-Butyl), 1-Pentyl, 2-Pentyl, 3-Pentyl, 2-Methyl-1-butyl, 3-Methyl-1-butyl, 2-Methyl-2-butyl, 3-Methyl-2-butyl, 2,2-Dimethyl-1-propyl, 1-Hexyl, 2-Hexyl, 3-Hexyl, 2-Methyl-1-pentyl, 3-Methyl-1-pentyl, 4-Methyl-1-pentyl, 2-Methyl-2-pentyl, 3-Methyl-2-pentyl, 4-Methyl-2-pentyl, 2-Methyl-3-pentyl, 3-Methyl-3-pentyl, 2,2-Dimethyl-1-butyl, 2,3-Dimethyl-1-butyl, 3,3-Dimethyl-1-butyl, 2-Ethyl-1-butyl, 2,3-Dimethyl-2-butyl, 3,3-Dimethyl-2-butyl, Heptyl, Octyl, Nonyl, Decyl, Undecyl, Dodecyl, Tridecyl, Tetradecyl, Pentadecyl, Hexadecyl, Heptadecyl, Octadecyl, Nonadecyl, Icosyl, Henicosyl, Docosyl, Tricosyl, Tetracosyl, Pentacosyl, Hexacosyl, Heptacosy, Octacosyl, Nonacosyl oder Triacontyl;
C₁-C₆-Alkyl, welches durch Phenyl oder C₅-C₇-Cycloalkyl substituiert ist, insbesondere Phenylmethyl (Benzyl), Diphenylmethyl, Triphenylmethyl, 2-Phenylethyl, 3-Phenylpropyl, Cyclopentylmethyl, 2-Cyclopentylethyl, 3-Cyclopentylpropyl, Cyclohexylmethyl, 2-Cyclohexylethyl oder 3-Cyclohexylpropyl;
CₙF_{2(n-a)-(1-b)}H_{2a-b} mit n ≤30, 0 ≤ a ≤ n und b = 0 oder 1, insbesondere CF₃, C₂F₅, C₆F₁₃, C₈F₁₇, C₁₀F₂₁ oder C₁₂F₂₅;
C₃-C₁₂-Cycloalkyl, insbesondere Cyclopentyl oder Cyclohexyl,; C₃-C₁₂-Cycloalkyl, welches durch C₁-C₆Alkyl substituiert ist, insbesondere 2-Methyl-1-cyclopentyl, 3-Methyl-1-cyclopentyl, 2-Methyl-1-cyclohexyl, 3-Methyl-1-cyclohexyl oder 4-Methyl-1-cyclohexyl;
CₙF_{2(n-a)-(1-b)}H_{2a-b} mit n ≤30, 0 ≤ a ≤ n und b = 0 oder 1;
C₂-C₃₀-Alkenyl, insbesondere 2-Propenyl, 3-Butenyl, cis-2-Butenyl oder trans-2-Butenyl;
CₙF_{2(n-a)-(1-b)}H_{2a-b} mit n ≤30, 0 ≤ a ≤ n und b = 0 oder 1;
C₃-C₁₂-Cycloalkenyl, insbesondere 3-Cyclopentenyl, 2-Cyclohexenyl, 3-Cyclohexenyl oder 2,5-Cyclohexadienyl;
CₙF_{2(n-a)-(1-b)}H_{2a-b} mit n ≤30, 0 ≤ a ≤ n und b = 0 oder 1;
Aryl oder Heteroaryl mit 2 bis 30 Kohlenstoffatomen, insbesondere Phenyl, 1-Naphthyl, 2-Naphthyl, 1-Pyrrolyl, 2-Pyrrolyl, 3-Pyrrolyl, 2-Pyridinyl, 3-Pyridinyl oder 4-Pyridinyl;
Aryl oder Heteroaryl mit 6 bis 30 Kohlenstoffatomen, welche durch ein bis drei C₁-C₆-Alkyl- oder Phenyl-Reste substituiert sind, insbesondere 2-Methyl-phenyl (2-Tolyl), 3-Methyl-phenyl (3-Tolyl), 4-Methyl-phenyl, 2-Ethyl-phenyl, 3-Ethyl-phenyl, 4-Ethyl-phenyl, 2,3-Dimethyl-phenyl, 2,4-Dimethyl-phenyl, 2,5-Dimethyl-phenyl, 2,6-Dimethyl-phenyl, 3,4-Dimethyl-phenyl, 3,5-Dimethyl-phenyl oder 4-Phenyl-phenyl; oder
C₆F₍₅₋ₐ₎Hₐ mit 0≤ a ≤ 5; stehen;
oder
zwei der Reste R¹, R², R³ bilden gemeinsam mit dem Heteroatom, an das sie gebunden
sind eine -(CH₂)ₙ-Kette, wobei n 4, 5, 6 oder 7 ist, und wobei diese Kette durch 1 bis maximal drei C₁-C₄-Alkylreste substituiert sein kann und/oder durch O, S, NH oder N-C₁-C₄-Alkyl unterbrochen sein kann;
R⁴, R⁵, R⁶, R⁷, R⁸ unabhängig voneinander für Wasserstoff, Halogen, OR^{c}, COR^{c},
C₁-C₃₀-Alkyl, C₃-C₁₂-Cycloalkyl, C₂-C₃₀-Alkenyl, C₃-C₁₂-Cycloalkenyl, Aryl oder Heteroaryl, wobei die 6 letztgenannten Reste ein oder mehrere Halogenreste tragen können und/oder 1 bis 3 Reste ausgewählt aus der Gruppe C₁-C₆-Alkyl, Aryl, Heteroaryl, C₃-C₇-Cycloalkyl, Halogen, OR^{c}, SR^{c}, NR^{c}R^{d}, COR^{c}, COOR^{c}, CO-NR^{c}R^{d}, und wobei R^{c} und R^{d} für Wasserstoff, C₁-C₆-Alkyl, C₁-C₆-Halogenalkyl, Cyclopentyl, Cyclohexyl, Phenyl, Tolyl oder Benzyl stehen;
vorzugsweise für
Wasserstoff;
Halogen,
C₁-C₃₀-Alkyl, insbesondere Methyl, Ethyl, 1-Propyl, 2-Propyl, 1-Butyl, 2-Butyl, 2-Methyl-1-propyl (Isobutyl), 2-Methyl-2-propyl (tert.-Butyl), 1-Pentyl, 2-Pentyl, 3-Pentyl, 2-Methyl-1-butyl, 3-Methyl-1-butyl, 2-Methyl-2-butyl, 3-Methyl-2-butyl, 2,2-Dimethyl-1-propyl, 1-Hexyl, 2-Hexyl, 3-Hexyl, 2-Methyl-1-pentyl, 3-Methyl-1-pentyl, 4-Methyl-1-pentyl, 2-Methyl-2-pentyl, 3-Methyl-2-pentyl, 4-Methyl-2-pentyl, 2-Methyl-3-pentyl, 3-Methyl-3-pentyl, 2,2-Dimethyl-1-butyl, 2,3-Dimethyl-1-butyl,
3,3-Dimethyl-1-butyl, 2-Ethyl-1-butyl, 2,3-Dimethyl-2-butyl, 3,3-Dimethyl-2-butyl, Heptyl, Octyl, Nonyl, Decyl, Undecyl, Dodecyl, Tridecyl, Tetradecyl, Pentadecyl, Hexadecyl, Heptadecyl, Octadecyl, Nonadecyl, Icosyl, Henicosyl, Docosyl, Tricosyl, Tetracosyl, Pentacosyl, Hexacosyl, Heptacosy, Octacosyl, Nonacosyl oder Triacontyl;
C₁-C₆-Alkyl, welches durch Phenyl oder C₅-C₇-Cycloalkyl substituiert ist, insbesondere Phenylmethyl (Benzyl), Diphenylmethyl, Triphenylmethyl, 2-Phenylethyl, 3-Phenylpropyl, Cyclopentylmethyl, 2-Cyclopentylethyl, 3-Cyclopentylpropyl, Cyclohexylmethyl, 2-Cyclohexylethyl oder 3-Cyclohexylpropyl;
CₙF₂₍ₙ-_{a)-(1-b)}H_{2a-b} mit n ≤30, 0 ≤ a ≤ n und b = 0 oder 1, insbesondere CF₃, C₂F₅, C₆F₁₃, C₈F₁₇, C₁₀F₂₁ oder C₁₂F₂₅;
OR^{a}, insbesondere Methoxy, Ethoxy; oder
COR^{a}, insbesondere Formyl, oder Acetyl;
C₃-C₁₂-Cycloalkyl, insbesondere Cyclopentyl oder Cyclohexyl,;
C₃-C₁₂-Cycloalkyl, welches durch C₁-C₆Alkyl substituiert ist, insbesondere 2-Methyl-1-cyclopentyl, 3-Methyl-1-cyclopentyl, 2-Methyl-1-cyclohexyl, 3-Methyl-1-cyclohexyl oder 4-Methyl-1-cyclohexyl;
CₙF_{2(n-a)-(1-b)}H_{2a-b} mit n ≤30, 0 ≤ a ≤ n und b = 0 oder 1;
C₂-C₃₀-Alkenyl, insbesondere 2-Propenyl, 3-Butenyl, cis-2-Butenyl oder trans-2-Butenyl;
CₙF_{2(n-a)-(1-b)}H_{2a-b} mit n ≤30, 0 ≤ a ≤ n und b = 0 oder 1;
C₃-C₁₂-Cycloalkenyl, insbesondere 3-Cyclopentenyl, 2-Cyclohexenyl, 3-Cyclohexenyl oder 2,5-Cyclohexadienyl;
CₙF_{2(n-a)-(1-b)}H_{2a-b}mit n ≤30, 0 ≤ a ≤ n und b = 0 oder 1;
Aryl oder Heteroaryl mit 2 bis 30 Kohlenstoffatomen, insbesondere Phenyl, 1-Naphthyl, 2-Naphthyl, 1-Pyrrolyl, 2-Pyrrolyl, 3-Pyrrolyl, 2-Pyridinyl, 3-Pyridinyl oder 4-Pyridinyl;
Aryl oder Heteroaryl mit 6 bis 30 Kohlenstoffatomen, welche durch ein bis drei C₁-C₆-Alkyl- oder Phenyl-Reste substituiert sind, insbesondere 2-Methyl-phenyl (2-Tolyl), 3-Methyl-phenyl (3-Tolyl), 4-Methyl-phenyl, 2-Ethyl-phenyl, 3-Ethyl-phenyl, 4-Ethyl-phenyl, 2,3-Dimethyl-phenyl, 2,4-Dimethyl-phenyl, 2,5-Dimethyl-phenyl, 2,6-Dimethyl-phenyl, 3,4-Dimethyl-phenyl, 3,5-Dimethyl-phenyl oder 4-Phenyl-phenyl; oder
C₆F₍₅₋ₐ₎Hₐ mit 0≤ a ≤ 5; stehen; oder
zwei der Reste R, R⁴, R⁵, R⁸, R⁷, R⁸, welche benachbart sind, bilden gemeinsam mit
dem Atom, an welchem sie gebunden sind ein 5- oder 6 gliedriges Ringsystem oder ein 5- oder 6-gliedriges aromatisches Ringsystem, welches unsubstituiert ist, oder durch 1 bis 4 Reste aus der Gruppe C₁-C₈-Alkyl, Aryl-, Heteroaryl-, C₃-C₇-Cycloalkyl, Halogen, OR^{a}, SR^{a}, NR^{a}R^{b}, COOR^{a}, CO-NR^{a}R^{b} oder COR^{a} substituiert ist ,wobei R^{a}, R^{b} unabhängig voneinander für Wasserstoff, C₁-C₆-Alkyl, C₁-C₆-Halogenalkyl, Cyclopentyl,- Cyclohexyl, Phenyl, Tolyl oder Benzyl stehen;
vorzugsweise
zwei der Reste R, R⁴, R⁵, R⁶, R⁷, R⁸, welche benachbart sind, bilden gemeinsam mit
dem Atom, an welchem sie gebunden sind ein 5- oder 6-gliedriges aromatisches Ringsystem, welches unsubstituiert ist, oder durch 1 bis 4 Reste aus der Gruppe C₁-C₆-Alkyl, Aryl-, Heteroaryl-, C₃-C₇-Cycloalkyl, Halogen, OR^{a}, SR^{a}, NR^{a}R^{b}, COOR^{a}, CO-NR^{a}R^{b} oder COR^{a} substituiert ist ,wobei R^{a}, R^{b} unabhängig voneinander für Wasserstoff, C₁-C₆-Alkyl, C₁-C₆-Halogenalkyl, Cyclopentyl, Cyclohexyl, Phenyl, Tolyl oder Benzyl stehen;
R für Wasserstoff, C₁-C₃₀-Alkyl, C₃-C₁₂-Cycloalkyl, C₂-C₃₀-Alkenyl,
C₃-C₁₂-Cycloalkenyl, Aryl oder Heteroaryl, wobei die 6 letztgenannten Reste ein oder mehrere Halogenreste tragen können und/oder 1 bis 3 Reste ausgewählt aus der Gruppe C₁-C₆-Alkyl, Aryl, Heteroaryl, C₃-C₇-Cycloalkyl, Halogen, OR^{c}, SR^{c}, NR^{c}R^{d}, COR^{c}, COOR^{c}, CO-NR^{c}R^{d}, wobei R^{c} und R^{d} für Wasserstoff, C₁-C₆-Alkyl, C₁-C₆-Halogenalkyl, Cyclopentyl, Cyclohexyl, Phenyl, Tolyl oder Benzyl steht;
vorzugsweise für
Wasserstoff;
C₁-C₃₀-Alkyl, insbesondere Methyl, Ethyl, 1-Propyl, 2-Propyl, 1-Butyl, 2-Butyl, 2-Methyl-1-propyl (Isobutyl), 2-Methyl-2-propyl (tert.-Butyl), 1-Pentyl, 2-Pentyl, 3-Pentyl, 2-Methyl-1-butyl, 3-Methyl-1-butyl, 2-Methyl-2-butyl, 3-Methyl-2-butyl, 2,2-Dimethyl-1-propyl, 1-Hexyl, 2-Hexyl, 3-Hexyl, 2-Methyl-1-pentyl, 3-Methyl-1-pentyl, 4-Methyl-1-pentyl, 2-Methyl-2-pentyl, 3-Methyl-2-pentyl, 4-Methyl-2-pentyl, 2-Methyl-3-pentyl, 3-Methyl-3-pentyl, 2,2-Dimethyl-1-butyl, 2,3-Dimethyl-1-butyl, 3,3-Dimethyl-1-butyl, 2-Ethyl-1-butyl, 2,3-Dimethyl-2-butyl, 3,3-Dimethyl-2-butyl, Heptyl, Octyl, Nonyl, Decyl, Undecyl, Dodecyl, Tridecyl, Tetradecyl, Pentadecyl, Hexadecyl, Heptadecyl, Octadecyl, Nonadecyl, Icosyl, Henicosyl, Docosyl, Tricosyl, Tetracosyl, Pentacosyl, Hexacosyl, Heptacosy, Octacosyl, Nonacosyl oder Triacontyl;
C₁-C₆-Alkyl, welches durch Phenyl oder C₅-C₇-Cycloalkyl substituiert ist, insbesondere Phenylmethyl (Benzyl), Diphenylmethyl, Triphenylmethyl, 2-Phenylethyl, 3-Phenylpropyl, Cyclopentylmethyl, 2-Cyclopentylethyl, 3-Cyclopentylpropyl, Cyclohexylmethyl, 2-Cyclohexylethyl oder 3-Cyclohexylpropyl;
CₙF_{2(n-a)-(1-b)}H_{2a-b} mit n ≤30, 0 ≤ a ≤ n und b = 0 oder 1, insbesondere CF₃, C₂F₅, C₆F₁₃, C₈F₁₇, C₁₀F₂₁ oder C₁₂F₂₅;
C₃-C₁₂-Cycloalkyl, insbesondere Cyclopentyl oder Cyclohexyl;
C₃-C₁₂-Cycloalkyl, welches durch C₁-C₆-Alkyl substituiert ist, insbesondere 2-Methyl-1-cyclopentyl, 3-Methyl-1-cyclopentyl, 2-Methyl-1-cyclohexyl, 3-Methyl-1-cyclohexyl oder 4-Methyl-1-cyclohexyl;
CₙF_{2(n-a)-(1-b)}H_{2a-b} mit n ≤30, 0 ≤ a ≤ n und b = 0 oder 1;
C₂-C₃₀-Alkenyl, insbesondere 2-Propenyl, 3-Butenyl, cis-2-Butenyl oder trans-2-Butenyl;
CₙF_{2(n-a)(1-b)}H_{2a-b} mit n ≤30, 0 ≤ a ≤ n und b = 0 oder 1;
C₃-C₁₂-Cycloalkenyl, insbesondere 3-Cyclopentenyl, 2-Cyclohexenyl, 3-Cyclohexenyl oder 2,5-Cyclohexadienyl;
CₙF_{2(n-a)-(1-b)}H_{2a-b} mit n ≤30, 0≤ a ≤ n und b = 0 oder 1;
Aryl oder Heteroaryl mit 2 bis 30 Kohlenstoffatomen, insbesondere Phenyl, 1-Naphthyl, 2-Naphthyl, 1-Pyrrolyl, 2-Pyrrolyl, 3-Pyrrolyl, 2-Pyridinyl, 3-Pyridinyl oder 4-Pyridinyl;
Aryl oder Heteroaryl mit 6 bis 30 Kohlenstoffatomen, welche durch ein bis drei C₁-C₆-Alkyl- oder Phenyl-Reste substituiert sind, insbesondere 2-Methyl-phenyl (2-Tolyl), 3-Methyl-phenyl (3-Tolyl), 4-Methyl-phenyl, 2-Ethyl-phenyl, 3-Ethyl-phenyl, 4-Ethyl-phenyl, 2,3-Dimethyl-phenyl, 2,4-Dimethyl-phenyl, 2,5-Dimethyl-phenyl, 2,6-Dimethyl-phenyl, 3,4-Dimethyl-phenyl, 3,5-Dimethyl-phenyl oder 4-Phenyl-phenyl; oder
C₆F₍₅₋ₐ₎Hₐ mit 0≤ a ≤ 5; stehen;
R^{1'}, R², unabhängig voneinander für ein C₁-C₃₀-Alkyl, C₃-C₁₂-Cycloalkyl, C₂-C₃₀-Alkenyl,
C₃-C₁₂-Cycloalkenyl, C₂-C₃₀-Alkinyl, Aryl oder Heteroaryl, wobei die 7 letztgenannten Reste ein oder mehrere Halogenreste tragen können und/oder 1 bis 3 Reste ausgewählt aus der Gruppe C₁-C₆-Alkyl, Aryl, Heteroaryl, C₃-C₇-Cycloalkyl, Halogen, OR^{c}, SR^{c}, NR^{c}R^{d}, COR^{c}, COOR^{c}, CO-NR^{c}R^{d}, wobei R^{c} und R^{d} für Wasserstoff, C₁-C₆-Alkyl, C₁-C₆-Halogenalkyl, Cyclopentyl, Cyclohexyl, Phenyl, Tolyl oder Benzyl stehen;
vorzugsweise für
C₁-C₃₀-Alkyl, insbesondere Methyl, Ethyl, 1-Propyl, 2-Propyl, 1-Butyl, 2-Butyl, 2-Methyl-1-propyl (Isobutyl), 2-Methyl-2-propyl (tert.-Butyl), 1-Pentyl, 2-Pentyl, 3-Pentyl, 2-Methyl-1-butyl, 3-Methyl-1-butyl, 2-Methyl-2-butyl, 3-Methyl-2-butyl, 2,2-Dimethyl-1-propyl, 1-Hexyl, 2-Hexyl, 3-Hexyl, 2-Methyl-1-pentyl, 3-Methyl-1-pentyl, 4-Methyl-1-pentyl, 2-Methyl-2-pentyl, 3-Methyl-2-pentyl, 4-Methyl-2-pentyl, 2-Methyl-3-pentyl, 3-Methyl-3-pentyl, 2,2-Dimethyl-1-butyl, 2,3-Dimethyl-1-butyl, 3,3-Dimethyl-1-butyl, 2-Ethyl-1-butyl, 2,3-Dimethyl-2-butyl, 3,3-Dimethyl-2-butyl, Heptyl, Octyl, Nonyl, Decyl, Undecyl, Dodecyl, Tridecyl, Tetradecyl, Pentadecyl, Hexadecyl, Heptadecyl, Octadecyl, Nonadecyl, Icosyl, Henicosyl, Docosyl, Tricosyl, Tetracosyl, Pentacosyl, Hexacosyl, Heptacosy, Octacosyl, Nonacosyl oder Triacontyl;
C₁-C₆-Alkyl, welches durch Phenyl oder C₅-C₇-Cycloalkyl substituiert ist, insbesondere Phenylmethyl (Benzyl), Diphenylmethyl, Triphenylmethyl, 2-Phenylethyl, 3-Phenylpropyl, Cyclopentylmethyl, 2-Cyclopentylethyl, 3-Cyclopentylpropyl, Cyclohexylmethyl, 2-Cyclohexylethyl oder 3-Cyclohexylpropyl;
CₙF₂(_{n-a)-(1-b)}H_{2a-b} mit n ≤30, 0 ≤ a ≤ n und b = 0 oder 1, insbesondere CF₃, C₂F₅, C₆F₁₃, C₈F₁₇, C₁₀F₂₁ oder C₁₂F₂₅;
C₃-C₁₂-Cycloalkyl, insbesondere Cyclopentyl oder Cyclohexyl;
C₃-C₁₂-Cycloalkyl, welches durch C₁-C₆-Alkyl substituiert ist, insbesondere 2-Methyl-1-cyclopentyl, 3-Methyl-1-cyclopentyl, 2-Methyl-1-cyclohexyl, 3-Methyl-1-cyclohexyl oder 4-Methyl-1-cyclohexyl;
CₙF_{2(n-a)-(1-b)}H_{2a-b} mit n ≤30, 0 ≤ a ≤ n und b = 0 oder 1;
C₂-C₃₀-Alkenyl, insbesondere 2-Propenyl, 3-Butenyl, cis-2-Butenyl oder trans-2-Butenyl;
CₙF_{2(n-a)-(1-b)}H_{2a-b} mit n ≤ 30, 0 ≤ a ≤ n und b = 0 oder 1;
C₃-C₁₂-Cycloalkenyl, insbesondere 3-Cyclopentenyl, 2-Cyclohexenyl, 3-Cyclohexenyl oder 2,5-Cyclohexadienyl;
CₙF_{2(n-a)-(1-b)}H_{2a-b} mit n ≤ 30, 0 ≤ a ≤ n und b = 0 oder 1;
Aryl oder Heteroaryl mit 2 bis 30 Kohlenstoffatomen, insbesondere Phenyl, 1-Naphthyl, 2-Naphthyl, 1-Pyrrolyl, 2-Pyrrolyl, 3-Pyrrolyl, 2-Pyridinyl, 3-Pyridinyl oder 4-Pyridinyl;
Aryl oder Heteroaryl mit 6 bis 30 Kohlenstoffatomen, welche durch ein bis drei C₁-C₆-Alkyl- oder Phenyl-Reste substituiert sind, insbesondere 2-Methyl-phenyl (2-Tolyl), 3-Methyl-phenyl (3-Tolyl), 4-Methyl-phenyl, 2-Ethyl-phenyl, 3-Ethyl-phenyl, 4-Ethyl-phenyl, 2,3-Dimethyl-phenyl, 2,4-Dimethyl-phenyl, 2,5-Dimethyl-phenyl, 2,6-Dimethyl-phenyl, 3,4-Dimethyl-phenyl, 3,5-Dimethyl-phenyl oder 4-Phenyl-phenyl; oder
C₆F₍₅₋ₐ₎Hₐ mit 0 ≤ a ≤ 5; stehen; oder
R^{1'} und R^{2'} gemeinsam eine -CHR^{a}-CHR^{b}-Kette, -CHR^{a}-CH₂-CHR^{b}-Kette oder einen -C₆H₄-1,2-diyl-Rest bilden, wobei R^{a} und R^{b} unabhängig voneinander für Wasserstoff oder C₁-C₆-Alkyl stehen;
Vorzugsweise für -CH₂-CH₂-, -CH₂-CH₂-CH₂-, -CH(CH₃)-CH₂-,-CH₂-CH(CH₃)-, -CH(CH₃)-CH(CH₃)-oder -C₆H₄-1,2-diyl-;

5. Verfahren nach einem der Ansprüche 1 bis 4, **dadurch gekennzeichnet, daß** ein quaternäres Carbonat der Formel I dargstellt wird, wobei
R^{1'}, R²' unabhängig voneinander für ein C₁-C₃₀-Alkyl, C₃-C₁₂-Cycloalkyl, C₂-C₃₀-Alkenyl,
C₃-C₁₂-Cycloalkenyl, C₂-C₃₀-Alkinyl, Aryl oder Heteroaryl, wobei die 7 letztgenannten Reste ein oder mehrere Halogenreste tragen können und/oder 1 bis 3 Reste ausgewählt aus der Gruppe C₁-C₆-Alkyl, Aryl, Heteroaryl, C₃-C₇-Cycloalkyl, Halogen, OR^{c}, SR^{c}, NR^{c}R^{d}, COR^{c}, COOR^{c}, CO-NR^{c}R^{d}, wobei R^{c} und R^{d} für Wasserstoff, C₁-C₈-Alkyl, C₁-C₆-Halogenalkyl, Cyclopentyl, Cyclohexyl, Phenyl, Tolyl oder Benzyl stehen;
vorzugsweise für
C₁-C₃₀-Alkyl, insbesondere Methyl, Ethyl, 1-Propyl, 2-Propyl, 1-Butyl, 2-Butyl, 2-Methyl-1-propyl (Isobutyl), 2-Methyl-2-propyl (tert.-Butyl), 1-Pentyl, 2-Pentyl, 3-Pentyl, 2-Methyl-1-butyl, 3-Methyl-1-butyl, 2-Methyl-2-butyl, 3-Methyl-2-butyl, 2,2-Dimethyl-1-propyl, 1-Hexyl, 2-Hexyl, 3-Hexyl, 2-Methyl-1-pentyl, 3-Methyl-1-pentyl, 4-Methyl-1-pentyl, 2-Methyl-2-pentyl, 3-Methyl-2-pentyl, 4-Methyl-2-pentyl, 2-Methyl-3-pentyl, 3-Methyl-3-pentyl, 2,2-Dimethyl-1-butyl, 2,3-Dimethyl-1-butyl, 3,3-Dimethyl-1-butyl, 2-Ethyl-1-butyl, 2,3-Dimethyl-2-butyl, 3,3-Dimethyl-2-butyl, Heptyl, Octyl, Nonyl, Decyl, Undecyl, Dodecyl, Tridecyl, Tetradecyl, Pentadecyl, Hexadecyl, Heptadecyl, Octadecyl, Nonadecyl, Icosyl, Henicosyl, Docosyl, Tricosyl, Tetracosyl, Pentacosyl, Hexacosyl, Heptacosy, Octacosyl, Nonacosyl oder Triacontyl;
C₁-C₆-Alkyl, welches durch Phenyl oder C₅-C₇-Cycloalkyl substituiert ist, insbesondere Phenylmethyl (Benzyl), Diphenylmethyl, Triphenylmethyl, 2-Phenylethyl, 3-Phenylpropyl, Cyclopentylmethyl, 2-Cyclopentylethyl, 3-Cyclopentylpropyl, Cyclohexylmethyl, 2-Cyclohexylethyl oder 3-Cyclohexylpropyl;
CₙF_{2(n-a)-(1-b)}H_{2a-b} mit n ≤ 30, 0 ≤ a ≤ n und b = 0 oder 1, insbesondere CF₃, C₂F₅, C₆F₁₃, C₈F₁₇, C₁₀F₂₁ oder C₁₂F₂₅;
C₃-C₁₂-Cycloalkyl, insbesondere Cyclopentyl oder Cyclohexyl;
C₃-C₁₂-Cycloalkyl, welches durch C₁-C₆-Alkyl substituiert ist, insbesondere 2-Methyl-1-cyclopentyl, 3-Methyl-1-cyclopentyl, 2-Methyl-1-cyclohexyl, 3-Methyl-1-cyclohexyl oder 4-Methyl-1-cyclohexyl;
CₙF_{2(n-8)-(1-b)}H_{2a-b} mit n ≤ 30, 0 ≤ a ≤ n und b = 0 oder 1;
C₂-C₃₀-Alkenyl, insbesondere 2-Propenyl, 3-Butenyl, cis-2-Butenyl oder trans-2-Butenyl;
CₙF_{2(n-a)-(1-b)}H_{2a-b} mit n ≤30, 0 ≤ a ≤ n und b = 0 oder 1;
C₃-C₁₂-Cycloalkenyl, insbesondere 3-Cyclopentenyl, 2-Cyclohexenyl, 3-Cyclohexenyl oder 2,5-Cyclohexadienyl;
CₙF_{2(n-a)-(1-b)}H_{2a-b} mit n ≤ 30, 0 ≤ a ≤ n und b = 0 oder 1;
Aryl oder Heteroaryl mit 2 bis 30 Kohlenstoffatomen, insbesondere Phenyl, 1-Naphthyl, 2-Naphthyl, 1-Pyrrolyl, 2-Pyrrolyl, 3-Pyrrolyl, 2-Pyridinyl, 3-Pyridinyl oder 4-Pyridinyl;
Aryl oder Heteroaryl mit 6 bis 30 Kohlenstoffatomen, welche durch ein bis drei C₁-C₆-Alkyl- oder Phenyl-Reste substituiert sind, insbesondere 2-Methyl-phenyl (2-Tolyl), 3-Methyl-phenyl (3-Tolyl), 4-Methyl-phenyl, 2-Ethyl-phenyl, 3-Ethyl-phenyl, 4-Ethyl-phenyl, 2,3-Dimethyl-phenyl, 2,4-Dimethyl-phenyl, 2,5-Dimethyl-phenyl, 2,6-Dimethyl-phenyl, 3,4-Dimethyl-phenyl, 3,5-Dimethyl-phenyl oder 4-Phenyl-phenyl; oder
C₆F₍₅₋ₐ₎Hₐ mit 0 ≤ a ≤ 5; stehen.

6. Verfahren nach einem der Ansprüche 1 bis 4, **dadurch gekennzeichnet, daß** ein quaternäres Carbonat der Formel I dargestellt wird, wobei
R^{1'} und R^{2'} gemeinsam eine -CHR^{a}-CHR^{b}-Kette, -CHR^{a}-CH₂-CHR^{b}-Kette oder einen -C₆H₄-1,2-diyl-Rest bilden, wobei R^{a} und R^{b} unabhängig voneinander für Wasserstoff oder C₁-C₆-Alkyl stehen;
vorzugsweise für
-CH₂-CH₂-, -CH₂-CH₂-CH₂-, -CH(CH₃)-CH₂-,-CH₂-CH(CH₃)-, -CH(CH₃)-CH(CH₃)-oder -C₆H₄-1,2-diyl-;

7. Verfahren nach einem der Ansprüche 1 bis 6, **dadurch gekennzeichnet, daß** ein quaternäres Carbonat der Formel I dargestellt wird, wobei
[A⁺] ein heteroaromatisches Kation ist ausgewählt aus der Gruppe 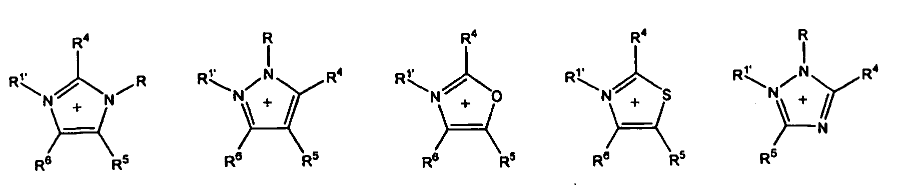 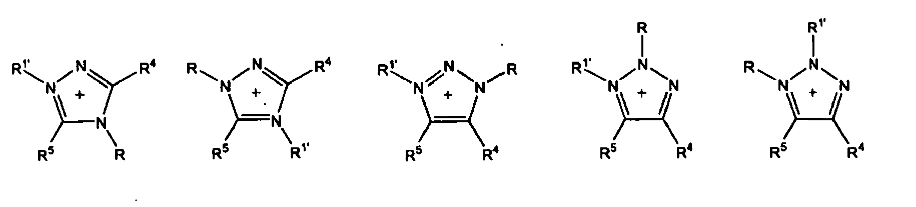 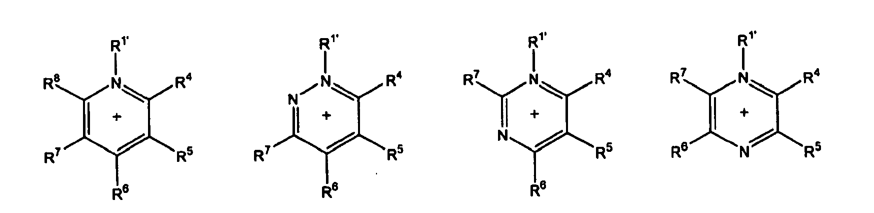 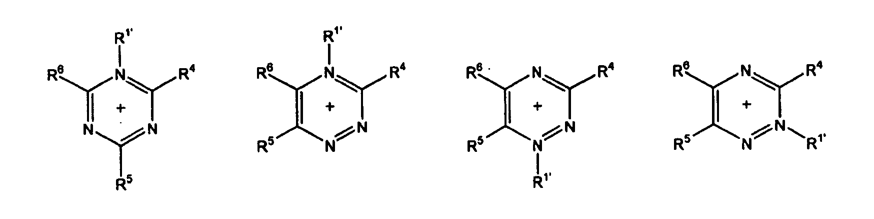 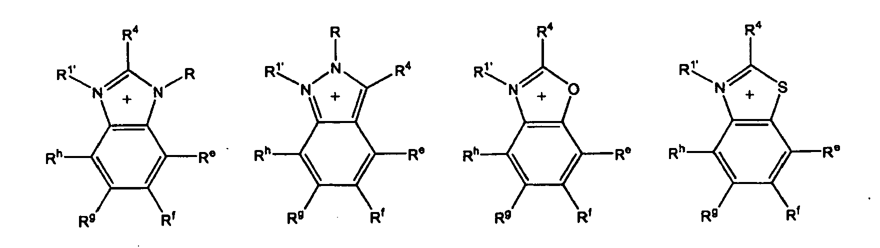 wobei
R⁴, R⁵, R⁶, R⁷, R⁸ unabhängig voneinander für Wasserstoff, Halogen, OR^{c}, COR^{c},
C₁-C₃₀-Alkyl, C₃-C₁₂-Cycloalkyl, C₂-C₃₀-Alkenyl, C₃-C₁₂-Cycloalkenyl, Aryl oder Heteroaryl, wobei die 6 letztgenannten Reste ein oder mehrere Halogenreste tragen können und/oder 1 bis 3 Reste ausgewählt aus der Gruppe C₁-C₈-Alkyl, Aryl, Heteroaryl, C₃-C₇-Cycloalkyl, Halogen, OR^{c}, SR^{c}, NR^{c}R^{d}, COR^{c}, COOR^{c}, CO-NR^{c}R^{d}, und wobei R^{c} und R^{d} für Wasserstoff, C₁-C₆-Alkyl, C₁-C₆-Halogenalkyl, Cyclopentyl, Cyclohexyl, Phenyl, Tolyl oder Benzyl stehen;
vorzugsweise für
Wasserstoff;
Halogen,
C₁-C₃₀-Alkyl, insbesondere Methyl, Ethyl, 1-Propyl, 2-Propyl, 1-Butyl, 2-Butyl, 2-Methyl-1-propyl (Isobutyl), 2-Methyl-2-propyl (tert.-Butyl), 1-Pentyl, 2-Pentyl, 3-Pentyl, 2-Methyl-1-butyl, 3-Methyl-1-butyl, 2-Methyl-2-butyl, 3-Methyl-2-butyl, 2,2-Dimethyl-1-propyl, 1-Hexyl, 2-Hexyl, 3-Hexyl, 2-Methyl-1-pentyl, 3-Methyl-1-pentyl, 4-Methyl-1-pentyl, 2-Methyl-2-pentyl, 3-Methyl-2-pentyl, 4-Methyl-2-pentyl, 2-Methyl-3-pentyl, 3-Methyl-3-pentyl, 2,2-Dimethyl-1-butyl, 2,3-Dimethyl-1-butyl, 3,3-Dimethyl-1-butyl, 2-Ethyl-1-butyl, 2,3-Dimethyl-2-butyl, 3,3-Dimethyl-2-butyl, Heptyl, Octyl, Nonyl, Decyl, Undecyl, Dodecyl, Tridecyl, Tetradecyl, Pentadecyl, Hexadecyl, Heptadecyl, Octadecyl, Nonadecyl, Icosyl, Henicosyl, Docosyl, Tricosyl, Tetracosyl, Pentacosyl, Hexacosyl, Heptacosy, Octacosyl, Nonacosyl oder Triacontyl;
C₁-C₆-Alkyl, welches durch Phenyl oder C₅-C₇-Cycloalkyl substituiert ist, insbesondere Phenylmethyl (Benzyl), Diphenylmethyl, Triphenylmethyl, 2-Phenylethyl, 3-Phenylpropyl, Cyclopentylmethyl, 2-Cyclopentylethyl, 3-Cyclopentylpropyl, Cyclohexylmethyl, 2-Cyclohexylethyl oder 3-Cyclohexylpropyl; CₙF_{2(n-a)-(1-b)}H_{2a-b} mit n ≤ 30, 0 ≤ a ≤ n und b = 0 oder 1, insbesondere CF₃, C₂F₅, C₆F₁₃, C₈F₁₇, C₁₀F₂₁ oder C₁₂F₂₅;
OR^{a}, insbesondere Methoxy, Ethoxy; oder
COR^{a}, insbesondere Formyl, oder Acetyl;
C₃-C₁₂-Cycloalkyl, insbesondere Cyclopentyl oder Cyclohexyl,; C₃-C₁₂-Cycloalkyl, welches durch C₁-C₆-Alkyl substituiert ist, insbesondere 2-Methyl-1-cyclopentyl, 3-Methyl-1-cyclopentyl, 2-Methyl-1-cyclohexyl, 3-Methyl-1-cyclohexyl oder 4-Methyl-1-cyclohexyl;
CₙF_{2(n-a)-(1-b)}H_{2a-b} mit n ≤ 30, 0 ≤ a ≤ n und b = 0 oder 1;
C₂-C₃₀-Alkenyl, insbesondere 2-Propenyl, 3-Butenyl, cis-2-Butenyl oder trans-2-Butenyl;
CₙF_{2(n-a)-(1-b)}H_{2a-b} mit n ≤ 30, 0 ≤ a ≤ n und b = 0 oder 1;
C₃-C₁₂-Cycloalkenyl, insbesondere 3-Cyclopentenyl, 2-Cyclohexenyl, 3-Cyclohexenyl oder 2,5-Cyclohexadienyl;
CₙF_{2(n-a)-(1-b)}H_{2a-b} mit n ≤ 30, 0 ≤ a ≤ n und b = 0 oder 1;
Aryl oder Heteroaryl mit 2 bis 30 Kohlenstoffatomen, insbesondere Phenyl, 1-Naphthyl, 2-Naphthyl, 1-Pyrrolyl, 2-Pyrrolyl, 3-Pyrrolyl, 2-Pyridinyl, 3-Pyridinyl oder 4-Pyridinyl;
Aryl oder Heteroaryl mit 6 bis 30 Kohlenstoffatomen, welche durch ein bis drei C₁-C₆-Alkyl- oder Phenyl-Reste substituiert sind, insbesondere 2-Methyl-phenyl (2-Tolyl), 3-Methyl-phenyl (3-Tolyl), 4-Methyl-phenyl, 2-Ethyl-phenyl, 3-Ethyl-phenyl, 4-Ethyl-phenyl, 2,3-Dimethyl-phenyl, 2,4-Dimethyl-phenyl, 2,5-Dimethyl-phenyl, 2,6-Dimethyl-phenyl, 3,4-Dimethyl-phenyl, 3,5-Dimethyl-phenyl oder 4-Phenyl-phenyl; oder
C₆F₍₅₋ₐ₎Hₐ mit 0 ≤ a ≤ 5; stehen;
R^{e}, R^{f}, R^{g}, R^{h} unabhängig voneinander für Wasserstoff, C₁-C₆-Alkyl, Aryl-, Heteroaryl-,
C₃-C₇-Cycloalkyl, Halogen, OR^{c}, SR^{c}, NR^{c}R^{d}, COOR^{c}, CO-NR^{c}R^{d} oder COR^{c} substituiert ist ,wobei R^{c}, R^{d} unabhängig voneinander für Wasserstoff, C₁-C₆-Alkyl, C₁-C₆-Halogenalkyl, Cyclopentyl, Cyclohexyl, Phenyl, Tolyl oder Benzyl stehen;
vorzugsweise für
Wasserstoff, Halogen oder C₁-C₆-Alkyl, insbesondere Wasserstoff oder C₁-C₆-Alkyl;
R für Wasserstoff, C₁-C₃₀-Alkyl, C₃-C₁₂-Cycloalkyl, C₂-C₃₀-Alkenyl,
C₃-C₁₂-Cycloalkenyl, Aryl oder Heteroaryl, wobei die 6 letztgenannten Reste ein oder mehrere Halogenreste tragen können und/oder 1 bis 3 Reste ausgewählt aus der Gruppe C₁-C₆-Alkyl, Aryl, Heteroaryl, C₃-C₇-Cycloalkyl, Halogen, OR^{c}, SR^{c}, NR^{c}R^{d}, COR^{c}, COOR^{c}, CO-NR^{c}R^{d}, wobei R^{c} und R^{d} für Wasserstoff, C₁-C₆-Alkyl, C₁-C₆-Halogenalkyl, Cyclopentyl, Cyclohexyl, Phenyl, Tolyl oder Benzyl steht.
vorzugsweise für
C₁-C₃₀-Alkyl, insbesondere Methyl, Ethyl, 1-Propyl, 2-Propyl, 1-Butyl, 2-Butyl, 2-Methyl-1-propyl (Isobutyl), 2-Methyl-2-propyl (tert.-Butyl), 1-Pentyl, 2-Pentyl, 3-Pentyl, 2-Methyl-1-butyl, 3-Methyl-1-butyl, 2-Methyl-2-butyl, 3-Methyl-2-butyl, 2,2-Dimethyl-1-propyl, 1-Hexyl, 2-Hexyl, 3-Hexyl, 2-Methyl-1-pentyl, 3-Methyl-1-pentyl, 4-Methyl-1-pentyl, 2-Methyl-2-pentyl, 3-Methyl-2-pentyl, 4-Methyl-2-pentyl, 2-Methyl-3-pentyl, 3-Methyl-3-pentyl, 2,2-Dimethyl-1-butyl, 2,3-Dimethyl-1-butyl, 3,3-Dimethyl-1-butyl, 2-Ethyl-1-butyl, 2,3-Dimethyl-2-butyl, 3,3-Dimethyl-2-butyl, Heptyl, Octyl, Nonyl, Decyl, Undecyl, Dodecyl, Tridecyl, Tetradecyl, Pentadecyl, Hexadecyl, Heptadecyl, Octadecyl, Nonadecyl, Icosyl, Henicosyl, Docosyl, Tricosyl, Tetracosyl, Pentacosyl, Hexacosyl, Heptacosy, Octacosyl, Nonacosyl oder Triacontyl;
C₁-C₆-Alkyl, welches durch Phenyl oder C₅-C₇-Cycloalkyl substituiert ist, insbesondere Phenylmethyl (Benzyl), Diphenylmethyl, Triphenylmethyl, 2-Phenylethyl, 3-Phenylpropyl, Cyclopentylmethyl, 2-Cyclopentylethyl, 3-Cyclopentylpropyl, Cyclohexylmethyl, 2-Cyclohexylethyl oder 3-Cyclohexylpropyl;
CₙF_{2(n-a)-(1-b)}H_{2a-b} mit n ≤ 30, 0 ≤ a ≤ n und b = 0 oder 1, insbesondere CF₃, C₂F₅, C₆F₁₃, C₈F₁₇, C₁₀F₂₁ oder C₁₂F₂₅;
C₃-C₁₂-Cycloalkyl, insbesondere Cyclopentyl oder Cyclohexyl;
C₃-C₁₂-Cycloalkyl, welches durch C₁-C₆Alkyl substituiert ist, insbesondere 2-Methyl-1-cyclopentyl, 3-Methyl-1-cyclopentyl, 2-Methyl-1-cyclohexyl, 3-Methyl-1-cyclohexyl oder 4-Methyl-1-cyclohexyl;
CₙF_{2(n-a)-(1-b)}H_{2a-b} mit n ≤ 30, 0 ≤ a ≤ n und b = 0 oder 1;
C₂-C₃₀-Alkenyl, insbesondere 2-Propenyl, 3-Butenyl, cis-2-Butenyl oder trans-2-Butenyl;
CₙF_{2(n-a)-(1-b)}H_{2a-b} mit n ≤ 30, 0 ≤ a ≤ n und b = 0 oder 1;
C₃-C₁₂-Cycloalkenyl, insbesondere 3-Cyclopentenyl, 2-Cyclohexenyl, 3-Cyclohexenyl oder 2,5-Cyclohexadienyl;
CₙF_{2(n-a)-(1-b)}H_{2a-b} mit n ≤ 30, 0 ≤ a ≤ n und b = 0 oder 1;
Aryl oder Heteroaryl mit 2 bis 30 Kohlenstoffatomen, insbesondere Phenyl, 1-Naphthyl, 2-Naphthyl, 1-Pyrrolyl, 2-Pyrrolyl, 3-Pyrrolyl, 2-Pyridinyl, 3-Pyridinyl oder 4-Pyridinyl;
Aryl oder Heteroaryl mit 6 bis 30 Kohlenstoffatomen, welche durch ein bis drei C₁-C₆-Alkyl- oder Phenyl-Reste substituiert sind, insbesondere 2-Methyl-phenyl (2-Tolyl), 3-Methyl-phenyl (3-Tolyl), 4-Methyl-phenyl, 2-Ethyl-phenyl, 3-Ethyl-phenyl, 4-Ethyl-phenyl, 2,3-Dimethyl-phenyl, 2,4-Dimethyl-phenyl, 2,5-Dimethyl-phenyl, 2,6-Dimethyl-phenyl, 3,4-Dimethyl-phenyl, 3,5-Dimethyl-phenyl oder 4-Phenyl-phenyl; oder
C₆F₍₅₋ₐ₎Hₐ mit 0 ≤ a ≤ 5; stehen.

8. Verfahren nach Anspruch 7, **dadurch gekennzeichnet, daß** ein quaternäres Carbonat der Formel I dargestellt wird, wobei
R⁴, R⁵, R⁶, R⁷, R⁸, R^{e}, R^{f}, R^{g}, R^{h} unabhängig voneinander für Wasserstoff, Halogen oder
C₁-C₆-Alkyl, vorzugsweise für Wasserstoff, Halogen oder C₁-C₆-Alkyl stehen;
R für C₁-C₆-Alkyl steht.

9. Verfahren nach einem der Ansprüche 1 bis 6 oder 8, **dadurch gekennzeichnet, daß** ein quaternäres Carbonat der Formel I dargestellt wird, wobei
[A⁺] ein quaternäres Ammonium-Kation [R^{1'}R¹R²R³N]⁺ oder ein Phosphonium-Kation [R^{1'}R¹R²R³P]⁺ ist.

10. Verfahren nach einem der Ansprüche 1 bis 9, **dadurch gekennzeichnet, daß** eine Lewis-Säure, welche mindestens ein Element aus der 1. bis 13. Gruppe des Periodensystems oder der Gruppe der Lanthaniden (Atomnummer 57 bis 71) enthält, eingesetzt wird, vorzugsweise eine Lewis-Säure welche mindestens ein Element ausgewählt aus der Gruppe Lithium, Titan, Zirkon, Vanadium, Niob, Tantal, Chrom, Mangan, Eisen, Cobalt, Nickel, Kupfer, Silber, Gold, Aluminium, Cer, Lanthan, Neodym, Samarium, Gadolinium, Erbium und Lutetium, insbesondere aus der Gruppe aus Lithium, Kupfer, Silber, Samarium, Cer, Titan und Aluminium enthält.

11. Verfahren nach Anspruch 10, **dadurch gekennzeichnet, daß** als Lewis-Säure mindestens ein Oxid, Carbonat, Halogenid oder Silikat von Lithium, Kupfer, Silber, Samarium, Cer, Titan oder Aluminium oder Gemische hiervon, oder ein Zeolith eingesetzt wird.

12. Verfahren nach Anspruch 10 oder 11, **dadurch gekennzeichnet, daß** die Lewis-Säure in 0,001 mol% bis 90 mol%, bevorzugt 0,1 mol% bis 10 mol%, besonders bevorzugt 0,5 mol% bis 5 mol% bezogen auf das Amin R¹R²R³N der Formel IIa, Phosphin R¹R²R³P der Formel IIb, Sulfid R¹R²S der Formel IIc oder den Heteroaromaten der Formel IId eingesetzt wird.

13. Verfahren nach einem der Ansprüche 1 bis 12, **dadurch gekennzeichnet, daß** das Alkyl- und/oder Arylcarbonat der Formel III in 0,5 bis 10 Molequivalenten, bevorzugt 1,0 bis 5,0 Molequivalenten, besonders bevorzugt 1,1 bis 2,0 Molequivalenten, bezogen auf das Amin R¹R²R³N der Formel IIa, Phosphin R¹R²R³P der Formel IIb, Sulfid R¹R²S der Formel IIc oder den Heteroaromaten der Formel IId, eingesetzt wird.

14. Verfahren nach einem der Ansprüche 1 bis 13, **dadurch gekennzeichnet, daß** die Reaktion bei einer Temperatur von 30°C bis 350°C, bevorzugt 50°C bis 150°C, besonders bevorzugt 80°C bis 120°C durchgeführt wird.

15. Verfahren nach einem der Ansprüche 1 bis 14, **dadurch gekennzeichnet, daß** die Reaktionszeit im Bereich von 0,1 Stunden bis 14 Tagen, bevorzugt 0,5 Stunden bis 5 Tagen, besonders bevorzugt 1 Stunde bis 24 Stunden, liegt.

16. Verfahren nach einem der Ansprüche 1 bis 15, **dadurch gekennzeichnet, daß** die Umsetzung in Gegenwart eines Reduktionsmittels, vorzugsweise einem Aluminiumhydrid, einem Borhydrid oder in Gegenwart von Wasserstoff und einem Katalysator ausgewählt aus der Gruppe der Platinmetalle; insbesondere NaAlH₄, LiAlH₄, NaBH₄, LiBH₄ oder H₂ in Gegenwart eines Katalysator der Platingruppe, wie Pt, Pd, Rh, Ru oder Ir, durchgeführt wird.

17. Verfahren nach einem der Ansprüche 1 bis 16, **dadurch gekennzeichnet, daß** die Umsetzung unter Luft- oder Wasserdampfatmosphäre oder unter Schutzgasatmosphäre, insbesondere unter Stickstoff oder Argon durchgeführt wird.

18. Verfahren nach einem der Ansprüche 1 bis 17, **dadurch gekennzeichnet, daß** die Umsetzung batchweise, semikontinuierlich oder kontinuierlich durchgeführt wird.

19. Verfahren nach einem der Ansprüche 1 bis 18, **dadurch gekennzeichnet, daß** die Umsetzung in Substanz oder in Gegenwart eines Lösungsmittels durchgeführt wird.

20. Verfahren nach Anspruch 19, **dadurch gekennzeichnet, daß** die Umsetzung in Substanz durchgeführt wird.

21. Verfahren nach Anspruch 19, **dadurch gekennzeichnet, daß** die Umsetzung in Gegenwart eines Lösungsmittels, vorzugsweise einem Alkohol, Ether, Dialkylformamid, Keton, Sulfoxid, Nitril oder einem Gemisch hiervon, insbesondere einem Alkohol, besonders vorzugsweise Methanol, Ethanol, Isopropanol, n-Propanol, n-Butanol, sec-Butanol, tert-Butanol, Pentanol, Hexanol, Heptanol, Octanol oder Phenol oder einem Gemisch hiervon, durchgeführt wird, wobei der Anteil an Lösungsmittel bei 1 bis 90%, bevorzugt bei 10 bis 50%, vorzugsweise bei 20 bis 30%, bezogen auf die Gesamtmasse des Reaktionsgemisches liegt.

22. Verfahren nach Anspruch 21, **dadurch gekennzeichnet, daß** als Lösungsmittel jener Alkohol eingesetzt wird, welcher zumindest zu einem der Reste des Alkyl- und/oder Arylcarbonats der Formel III korrespondiert.

23. Verfahren nach einem der Ansprüche 1 bis 22, **dadurch gekennzeichnet, daß** das Reaktionsgemisch gerührt, geschüttelt oder anderweitig durchmischt wird.

24. Verfahren nach den Ansprüchen 1 bis 23, **dadurch gekennzeichnet, dass** das Alkyl-und/oder Arylcarbonat der Formel III durch Umsetzung von CO₂, CO oder CO/O₂ oder Gemischen hiervon mit einem Alkohol R^{1'}OH oder R^{2'}OH bzw. einem Gemisch hiervon, in Gegenwart einer Lewissäure, bei einem Druck von 1 bis 300 bar, bevorzugt 5 bis 100 bar, besonders bevorzugt 10 bis 50 bar, erhalten wird.

25. Verfahren nach Anspruch 24, **dadurch gekennzeichnet, daß** das Alkyl- und/oder Arylcarbonat der Formel III in situ erzeugt wird.

26. Verfahren zur Darstellung von quaternären Carbonaten, der Formel I, wie in den Ansprüchen 1 bis 9 beschrieben, **dadurch gekennzeichnet, daß** man ein Amin R¹R²R³N der Formel IIa, das Phosphin R¹R²R³P der Formel IIb, Sulfid R¹R²S der Formel IIc oder den Heteroaromaten der Formel IId, mit einem Alkohol R^{1'}OH oder R^{2'}OH bzw. einem Gemisch hiervon und CO₂, CO/O₂ oder CO in Kombination mit einem Oxidationsmittel, oder Gemischen hiervon, in Gegenwart mindestens einer Lewissäure bei 1 bis 300 bar, bevorzugt 5 bis 100 bar, besonders bevorzugt 10 bis 50 bar, umsetzt.

27. Verfahren nach einem der Ansprüche 24 bis 26, **dadurch gekennzeichnet, daß** die Lewissäure in 0,001 mol% bis 90 mol%, bevorzugt 0,1 mol% bis 10 mol%, besonders bevorzugt 0,5 mol% bis 5 mol%, bezogen auf das Amin R¹R²R³N der Formel IIa, Phosphin R¹R²R³P der Formel IIb, Sulfid R¹R²S der Formel IIc oder den Heteroaromaten der Formel IId eingesetzt wird.

28. Verfahren nach einem der Ansprüche 24 bis 27 , **dadurch gekennzeichnet, daß** der Alkohol R^{1'}OH oder R^{2'}OH, bzw. ein Gemisch hiervon in 0,5 bis 20 Molequivalenten, bevorzugt 1,0 bis 5 Molequivalenten, besonders bevorzugt 1,5 bis 2,5 Molequivalenten, bezogen auf das Amin R¹R²R³N der Formel IIa, Phosphin R¹R²R³P der Formel IIb, Sulfid R¹R²S der Formel IIc oder dem Heteroaromaten der Formel IId, eingesetzt wird.

29. Verfahren nach einem der Ansprüche 24 bis 28, **dadurch gekennzeichnet, daß** das zur Erzeugung des Alkyl-und/oder Arylcarbonats der Formel III benötigte Kohlendioxid durch die Umsetzung eines quaternären Carbonats der Formel I und/oder einer quaternären Verbindung der Formel IVa und/oder IVb 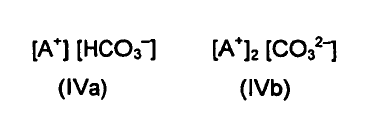 wobei [A⁺] die in den Ansprüchen 1 bis 27 genannte Bedeutung hat,
mit einer Bronsted-Säure HZ generiert wird.

30. Verfahren nach einem der Ansprüche 24 bis 28, **dadurch gekennzeichnet, daß** der zur Erzeugung des Alkyl-und/oder Arylcarbonats der Formel III benötigte Alkohol R^{2'}OH durch die Umsetzung eines quaternären Carbonats der Formel I mit einer Brønsted-Säure HZ generiert wird.

31. Verfahren nach einem der Ansprüche 24 bis 30, **dadurch gekennzeichnet, daß** die Lewissäure zur Alkylierung oder Arylierung des Amins R¹R²R³N der Formel IIa, Phosphins R¹R²R³P der Formel IIb, Sulfids R¹R²S der Formel IIc oder des Heteroaromaten der Formel IId mit dem Alkyl- und/oder Arylcarbonat der Formel III identisch mit der Lewissäure zur in situ Erzeugung des Alkyl- und/oder Arylcarbonats der Formel III ist.

32. Verfahren nach einem der Ansprüche 1 bis 31, **dadurch gekennzeichnet, daß** die Reaktion in flüssiger Phase oder in Gasphase abläuft.

33. Verfahren nach einem der Ansprüche 1 bis 32, **dadurch gekennzeichnet, daß** die Aufarbeitung der Reaktionsgemische durch Destillation, Vakuumdestillation, Dünnschichtverdampfung, Kurzwegverdampfung, Rotationsverdampfung, Sprühtrocknung, Osmose, Pervaporation, Strippen mit Gas oder Wasserdampf, Ausfrieren, Gefriertrocknung, chemische oder physikalische Adsorption oder andere geeignete Verfahren erfolgt.

34. Verfahren zur Herstellung einer Verbindungen der Formel V,
[A⁺]ₙ [Zⁿ⁻] (V)
wobei
[A⁺] die in den Ansprüchen 1 bis 33 genannte Bedeutung hat, und
[Zⁿ⁻] für ein ein-, zwei- drei- oder vierwertiges Anion;
steht;
**dadurch gekennzeichnet, daß** man quaternäres Carbonat der Formel I nach einem der Verfahren 1 bis 33 herstellt und das so erhaltene Carbonat der Formel I mit einer Broensted-Säure [H⁺]ₙ[Zⁿ⁻], dessen Anhydrid oder einem Salz [Kation^{m+}]ₙ[Zⁿ⁻]ₘ umsetzt.

## Claims

1. A method for producing quaternary carbonates of formula I,
[A]⁺ [R²OCO₂]⁻ (I)
wherein
[A]⁺ represents a quaternary ammonium cation [R¹R¹R²R³N]⁺, a phosphonium cation [R¹R¹R²R³P]⁺, a sulphonium cation [R¹R¹R²S]⁺ or a heteroaromatic cation K,
R¹, R², R³ represent, independently of one another, hydrogen, possibly sub. alkyl, alkenyl, alkinyl, cycloalkyl, cycloalkenyl, aryl or heteroaryl; or
two of the residues R¹, R², R³ form a ring with the heteroatom to which they are attached, wherein this is saturated or unsaturated, unsubstituted or substituted and wherein this chain may be interrupted by one or more heteroatoms chosen from the group O, S, NH or N-C₁-C₄-alkyl;
R¹, R² represent, independently of one another, possibly sub. alkyl, alkenyl, alkinyl, cycloalkyl, cycloalkenyl, aryl or heteroaryl; or
R^{1'} and R^{2'} together form a -CHR^{a}-CHR^{b} chain, -CHR^{a}-CH₂-CHR^{b} chain or possibly represent substituted -C₆H₄-1,2-diyl residue, wherein R^{a} and R^{b} represent, independently of one another, hydrogen or C₁-C₆-alkyl; wherein in this case a zwitterionic compound of formula I is obtained, in which R¹ and [R²OCO₂]⁻ form the residue -OCO-CHR^{a}-CHR^{b}-, -OCO-CHR^{a}-CH₂-CHR^{b}- or possibly sub. OCO-1-C₆H₄-2-yl (compound I');
**characterized in that**
an amine R¹R²R³N of formula IIa, phosphine R¹R²R³P of formula IIb, sulphide R¹R²S of formula IIc or a heteroaromate of formula IId, wherein the residues R¹, R² and R³ have the above meaning, react
with an alkyl- and/or aryl carbonate of formula III
R¹O(CO)OR^{2'} (III)
wherein
R¹, R²' represent, independently of one another, a possibly sub. alkyl, alkenyl, alkinyl, cycloalkyl, cycloalkenyl, aryl or heteroaryl; or
R^{1'} and R²' together form a -CHR^{a}-CHR^{b} chain, -CHR^{a}-CH₂-CHR^{b} chain or possibly substituted -C₆H₄-1,2-diyl residue, wherein R^{a} and R^{b} represent, independently of one another, hydrogen or C₁-C₆-alkyl, wherein in this case a zwitterionic compound of formula I' is obtained,
in the presence of catalytic amounts of a Lewis acid.

2. The method according to claim 1, **characterised in that** a quarternary carbonate of formula I is produced, wherein
[A]⁺ stands for a quarternary ammonium cation [R¹R¹R²R³N]⁺, a phosphonium cation [R¹R¹R²R³P]⁺, a sulphonium cation [R¹R¹R²S]⁺ or a heteroaromatic cation chosen from the group 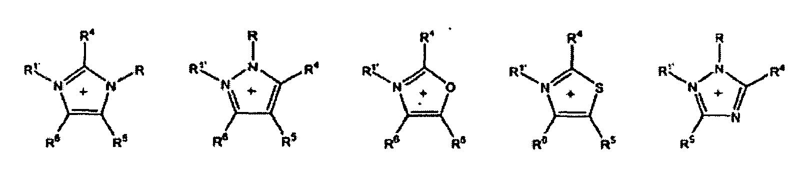 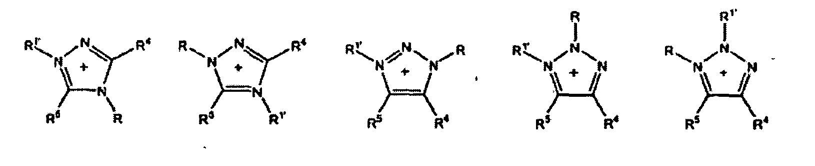 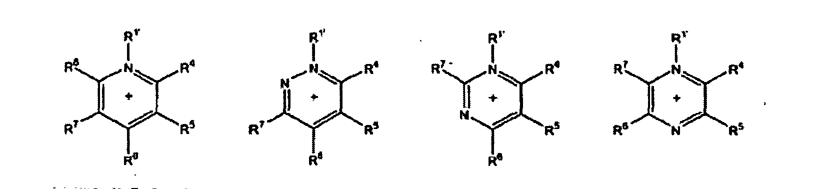 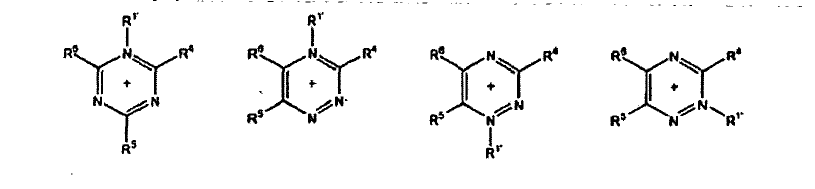 wherein die residues have the following meaning:
R¹' possibly sub. alkyl, alkenyl, alkinyl, cyclo-alkyl, cycloalkenyl aryl or
heteroaryl;
R hydrogen, possibly sub. alkyl, alkenyl, alkinyl, cycloalkyl, cycloalkenyl aryl or
heteroaryl;
R⁴, R⁵, R⁶,
R⁷, R⁸ hydrogen, halogen, nitro, cyano, OR^{C}, SR^{C}, NR^{c}R^{d}, COR^{C}, COOR^{C}, CO-NR^{c}R^{d}, Ci-C₃₀-alkyl, C₃-C₁₂-cydoalkyl, C₂-C₃₀-alkenyl, C₃-C₁₂-cycloalkenyl, aryl or heteroaryl, wherein the 6 latter residues can carry one or more halogen residues and/or 1 to 3 residues are chosen from the group C₁-C₆-alkyl, aryl, heteroaryl, C₃-C₇-cycloalkyl, halogen, OR^{C} SR^{C}, NR^{c}R^{d}, COR^{C}, COOR^{C}, CO-NR^{c}R^{d}, wherein R^{c} and R^{d} represent, independently of one another, hydrogen, C₁-C₆-alkyl, C₁C₆-alkyl halide, cyclopentyl, cyclohexyl, phenyl, tolyl or benzyl; or
two of the residues R, R⁴, R⁵, R⁶, R⁷, R⁸, which are adjacent to one another, form a ring with the atom to which they are attached, wherein this is unsaturated or aromatic, unsubstituted or substituted and wherein the chain formed by the residues concerned may be interrupted by one or more heteroatoms chosen from the group O, S, N, NH or N-C₁C₄-alkyl; or
in the case of I' compounds, R¹' and R²' jointly represent a -CHR^{a}-CHR^{b} chain, -CHR^{a}-CH₂-CHR^{b}-chain or possibly substituted -C₆H₄-1,2-diyl residue, wherein R^{a} and R^{b} represent, independently of one another, hydrogen or C₁C₆-alkyl.

3. The method according to claim 1 or 2, **characterised in that** a quaternary carbonate of formula I is produced, wherein
R¹, R², R³ represent, independently of one another, hydrogen, C₁C₃₀-alkyl, C₃-C₁₂-cycloalkyl, C₂-C₃₀-alkenyl, C₃-C₁₂-cycloalkenyl, C₂-C₃₀-alkinyl, aryl or heteroaryl, wherein the 7 latter residues can carry one or more halogen residues and/or 1 to 3 residues are chosen from the group C₁-C₆alkyl, aryl, heteroaryl, C₃-C₇-cycloalkyl, halogen, OR^{C} SR^{C}, NR^{c}R^{d}, COR^{C}, COOR^{C}, CO-NR^{c}R^{d}, wherein R^{c} and R^{d} represent, independently of one another, hydrogen, C₁C₆-alkyl, C₁C₆-alkyl halide, cyclopentyl, cyclohexyl, phenyl, tolyl or benzyl; or
two of the residues R¹, R², R³ form a ring with the heteroatom to which they are attached, wherein this is saturated or unsaturated, unsubstituted or substituted and wherein this chain may be interrupted by one or more heteroatoms chosen from the group O, S, NH or N-C₁-C₄-alkyl;
R⁴, R⁵, R⁶, R⁷, R⁸ represent, independently of one another, hydrogen, halogen, nitro, cyano, OR^{c}, SR^{C}, NRR^{c}R^{d}, COR^{C}, COOR^{C}, CO-NR^{c}R^{d}, C₁C₃₀-alkyl, C₃-C₁₂-cycloalkyl, C₂-C₃₀-alkenyl, C₃-C₁₂-cycloalkenyl, aryl or heteroaryl, wherein the 6 latter residues can carry one or more halogen residues and/or 1 to 3 residues chosen from the group C₁-C₆-alkyl, aryl, heteroaryl, C₃-C₇-cycloalkyl, halogen, OR^{c} SR^{C}, NR^{c}R^{d}, COR^{C}, COOR^{C}, CO-NR^{c}R^{d} and wherein R^{c} and R^{d} represent hydrogen, C₁C₆-alkyl, C₁C₆-alkyl halide, cyclopentyl, cyclohexyl, phenyl, tolyl or benzyl; or
two of the residues R, R⁴, R⁵, R⁶, R⁷, R⁸, which are adjacent, for a ring with the atom to which they are attached, wherein this is unsaturated or aromatic, unsubstituted or substituted and wherein the chain formed by the residues concerned may be interrupted by one or more heteroatoms chosen from the group O, S, N, NH or N-C₁C₄-alkyl; or
R stands for hydrogen, C₁-C₃₀-alkyl, C₃-C₁₂-cydoalkyl, C₂-C₃₀-alkenyl, C₃-C₁₂-cycloalkenyl, C₂-C₃₀-alkinyl, aryl or heteroaryl, wherein die 7 latter residues can carry one or more halogen residues and/or 1 to 3 residues chosen from the group C₁C₆-alkyl, aryl, heteroaryl, C₃-C₇-cycloalkyl, halogen, OR^{C} SR^{C}, NR^{c}R^{d}, COR^{C}, COOR^{C}, CO-NR^{c}R^{d}, wherein R^{c} and R^{d} represent hydrogen, C₁C₆-alkyl, C₁C₆-alkyl halide, cyclopentyl, cyclohexyl, phenyl, tolyl or benzyl;
R¹, R²' represent, independently of one another, C₁C₃₀-alkyl, C₃-C₁₂-cycloalkyl, C₂-C₃₀-alkenyl, C₃-C₁₂-cycloalkenyl, C₂-C₃₀-alkinyl, aryl or heteroaryl, wherein the 7 latter residues can carry one or more halogen residues and/or 1 to 3 residues chosen from the group C₁-C₆-alkyl, aryl, heteroaryl, C₃-C₇-cycloalkyl, halogen, OR^{C} SR^{C}, NR^{c}R^{d}, COR^{C}, COOR^{C}, CO-NR^{c}R^{d}, wherein R^{c} and R^{d} represent hydrogen, C₁-C₆-alkyl, C₁-C₆-alkyl halide, cyclopentyl, cyclohexyl, phenyl, tolyl or benzyl; or
R^{1'} and R²' together form a -CHR^{a}-CHR^{b} chain, -CHR^{a}-CH₂-CHR^{b} chain or possibly substituted -C₆H₄-1,2-diyl residue, wherein R^{a} and R^{b} represent, independently of one another, hydrogen or C₁-C₆-alkyl;

4. The method according to one of the claims 1 to 3, **characterised in that** a quarternary carbonate of formula I is produced, wherein
R¹, R², R³ represent, independently of one another, hydrogen, C₁C₃₀-alkyl, C₃-C₁₂-cycloalkyl, C₂-C₃₀-alkenyl, C₃-C₁₂-cycloalkenyl, aryl or heteroaryl, wherein die 6 latter residues can carry one or more halogen residues and/or 1 to 3 residues chosen from the group C₁-C₆-alkyl, aryl, heteroaryl, C₃-C₇-cycloalkyl, halogen, OR^{C} SR^{C}, NR^{c}R^{d}, COR^{C}, COOR^{C}, CO-NR^{c}R^{d} and wherein R^{c} and R^{d} represent hydrogen, C₁-C₆-alkyl, C₁-C₆-alkyl halide, cyclopentyl, cyclohexyl, phenyl, tolyl or benzyl;
preferably
hydrogen;
C₁-C₃₀-alkyl, particularly methyl, ethyl, 1-propyl, 2-propyl, 1-butyl, 2-butyl, 2-methyl-1-propyl (isobutyl), 2-methyl-2-propyl (tert-butyl), 1-pentyl, 2-pentyl, 3-pentyl, 2-methyl-1-butyl, 3-methyl-1-butyl, 2-methyl-2-butyl, 3-methyl-2-butyl, 2,2-dimethyl-1-propyl, 1-hexyl, 2-hexyl, 3-hexyl, 2-methyl-1-pentyl, 3-methyl-1-pentyl, 4-methyl-1-pentyl, 2-methyl-2-pentyl, 3-methyl-2-pentyl, 4-methyl-2-pentyl, 2-methyl-3-pentyl, 3-methyl-3-pentyl, 2,2-dimethyl-1-butyl, 2,3-dimethyl-1-butyl, 3,3-dimethyl-1-butyl, 2-ethyl-1-butyl, 2,3-dimethyl-2-butyl, 3,3-dimethyl-2-butyl, heptyl, octyl, nonyl, decyl, undecyl, dodecyl, tridecyl, tetradecyl, pentadecyl, hexadecyl, heptadecyl, octadecyl, nonadecyl, icosyl, henicosyl, docosyl, tricosyl, tetracosyl, pentacosyl, hexacosyl, heptacosy, octacosyl, nonacosyl or triacontyl;
C₁C₆-alkyl, which is substituted by phenyl or C₅-C₇-cycloalkyl, particularly phenylmethyl (benzyl), diphenylmethyl, triphenylmethyl, 2-phenylethyl, 3-phenylpropyl, cyclopentylmethyl, 2-cyclopentylethyl, 3-cyclopentylpropyl, cyclohexylmethyl, 2-cyclohexylethyl or 3-cyclohexylpropyl;
CₙF_{2(n-a)-(1-b)}H_{2a-b} where n ≤ 30, 0 ≤ a ≤ n and b = 0 or 1, particularly CF₃, C₂F₅, C₆F₁₃, C₈F₁₇, C₁₀F₂₁ or C₁₂F₂₅;
C₃-C₁₂-cycloalkyl, particularly cyclopentyl or cyclohexyl;
C₃-C₁₂-cycloalkyl, which is substituted by C₁-C₆-alkyl, particularly 2-methyl-1-cyclopentyl, 3-methyl-1-cyclopentyl, 2-methyl-1-cyclohexyl, 3-methyl-1-cyclohexyl or 4-methyl-1-cyclohexyl;
CₙF_{2(n-a)-(1-b)}H_{2a-b} where n ≤30, 0 ≤ a ≤ n and b = 0 or 1;
C₂-C₃₀-alkenyl, particularly 2-propenyl, 3-butenyl, cis-2-butenyl or trans-2-butenyl; CₙF_{2(n-a)-(1-b)}H_{2a-b} where n ≤ 30, 0 ≤ a ≤ n and b = 0 or 1;
C₃-C₁₂-cycloalkenyl, particularly 3-cyclopentenyl, 2-cyclohexenyl, 3-cyclohexenyl or 2,5-cyclohexadienyl;
CₙF_{2(n-a)-(1-b)}H_{2a-b} where n ≤ 30, 0 ≤ a ≤ n and b = 0 or 1;
aryl or heteroaryl with 2 to 30 carbon atoms, particularly phenyl, 1-naphthyl, 2-naphthyl, 1-pyrrolyl, 2-pyrrolyl, 3-pyrrolyl, 2-pyridinyl, 3-pyridinyl or 4-pyridinyl;
aryl or heteroaryl with 6 to 30 carbon atoms, which are substituted by one to three C₁-C₆-alkyl or phenyl residues, particularly 2-methyl-phenyl (2-tolyl), 3-methyl-phenyl (3-tolyl), 4-methyl-phenyl, 2-ethyl-phenyl, 3-ethyl-phenyl, 4-ethyl-phenyl, 2,3-dimethyl-phenyl, 2,4-dimethyl-phenyl, 2,5-dimethyl-phenyl, 2,6-dimethyl-phenyl, 3,4-dimethyl-phenyl, 3,5-dimethyl-phenyl or 4-phenyl-phenyl; or
C₆F₍₅₋ₐ₎Hₐ where 0 ≤ a ≤ 5;
or
two of the residues R¹, R², R³ form along with the heteroatom to which they are attached a -(CH₂)ₙ chain, wherein n is 4, 5, 6 or 7 and wherein this chain can be substituted by 1 to maximum three C₁-C₄-alkyl residues and/or interrupted by O, S, NH or N-C₁C₄-alkyl;
R⁴, R⁵, R⁶, R⁷, R⁸ represent, independently of one another, hydrogen, halogen, OR^{C}, COR^{C}, C₁-C₃₀-alkyl, C₃-C₁₂-cycloalkyl, C₂-C₃₀-alkenyl, C₃-C₁₂-cycloalkenyl, aryl or heteroaryl, wherein the 6 latter residues can carry one or more halogen residues and/or 1 to 3 residues chosen from the group C₁-C₈-alkyl, aryl, heteroaryl, C₃-C₇-cycloalkyl, halogen, OR^{c} SR^{c}, NR^{c}R^{d}, COR^{C}, COOR^{C}, CO-NR^{c}R^{d} and wherein R^{c} and R^{d} represent hydrogen, C₁C₆-alkyl, C₁C₆-alkyl halide, cyclopentyl, cyclohexyl, phenyl, tolyl or benzyl;
preferably hydrogen;
halogen,
C₁C₃₀-alkyl, particularly methyl, ethyl, 1-propyl, 21-propyl, 1-butyl, 2-butyl, 2-methyl-1-propyl (isobutyl), 2-methyl-2-propyl (tert-butyl), 1-pentyl, 2-pentyl, 3-pentyl, 2-methyl-1-butyl, 3-methyl-1-butyl, 2-methyl-2-butyl, 3-methyl-2-butyl, 2,2-dimethyl-1-propyl, 1-hexyl, 2-hexyl, 3-hexyl, 2-methyl-1-pentyl, 3-methyl-1-pentyl, 4-methyl-1-pentyl, 2-methyl-2-pentyl, 3-methyl-2-pentyl, 4-methyl-2-pentyl, 2-methyl-3-pentyl, 3-methyl-3-pentyl, 2,2-dimethyl-1-butyl, 2,3-dimethyl-1-butyl, 3,3-dimethyl-1-butyl, 2-ethyl-1-butyl, 2,3-dimethyl-2-butyl, 3,3-dimethyl-2-butyl, heptyl, octyl, nonyl, decyl, undecyl, dodecyl, tridecyl, tetradecyl, pentadecyl, hexadecyl, heptadecyl, octadecyl, nonadecyl, icosyl, henicosyl, docosyl, tricosyl, tetracosyl, pentacosyl, hexacosyl, heptacosy, octacosyl, nonacosyl or triacontyl;
C₁C₆-alkyl, which is substituted by phenyl or C₅-C₇-cycloalkyl, particularly phenylmethyl (benzyl), diphenylmethyl, triphenylmethyl, 2-phenylethyl,
3-phenylpropyl, cyclopentylmethyl, 2-cyclopentylethyl, 3-cyclopentylpropyl, cyclohexylmethyl, 2-cyclohexylethyl or 3-cyclohexylpropyl;
CₙF_{2(n-a)-(1-b)}H_{2a-b} where n ≤ 30, 0 ≤ a ≤ n and b = 0 or 1, particularly CF₃, C₂F₅, C₆F₁₃, C₈F₁₇, C₁₀F₂₁ or C₁₂F₂₅;
OR^{a}, particularly methoxy, ethoxy; or
COR^{a}, particularly formyl or acetyl;
C₃-C₁₂-cycloalkyl, particularly cyclopentyl or cyclohexyl;
C₃-C₁₂-cycloalkyl, which is substituted by C₁-C₆-alkyl, particularly 2-methyl-1-cyclopentyl, 3-methyl-1-cyclopentyl, 2-methyl-1-cyclohexyl, 3-methyl-1-cyclohexyl or 4-methyl-1-cyclohexyl;
CₙF_{2(n-a)-(1-b)}H_{2a-b} where n ≤ 30, 0 ≤ a ≤ n and b = 0 or 1;
C₂-C₃₀-alkenyl, particularly 2-propenyl, 3-butenyl, cis-2-butenyl or trans-2-butenyl; CₙF_{2(n-a)-(1-b)}H_{2a-b} where n ≤ 30, 0 ≤ a ≤ n and b = 0 or 1;
C₃-C₁₂-cycloalkenyl, particularly 3-cyclopentenyl, 2-cyclohexenyl, 3-cyclohexenyl or 2,5-cyclohexadienyl;
CₙF_{2(n-a)-(1-b)}H_{2a-b} where n ≤ 30, 0 ≤ a ≤ n and b = 0 or 1;
aryl or heteroaryl with 2 to 30 carbon atoms, particularly phenyl, 1-naphthyl, 2-naphthyl, 1-pyrrolyl, 2-pyrrolyl, 3-pyrrolyl, 2-pyridinyl, 3-pyridinyl or 4-pyridinyl;
aryl or heteroaryl with 6 to 30 carbon atoms, which are substituted by one to three C₁-C₆-alkyl or phenyl residues, particularly 2-methyl-phenyl (2-tolyl), 3-methyl-phenyl (3-tolyl), 4-methyl-phenyl, 2-ethyl-phenyl, 3-ethyl- phenyl, 4-ethyl-phenyl, 2,3-dimethyl-phenyl, 2,4-dimethyl-phenyl, 2,5-dimethyl-phenyl, 2,6-dimethyl-phenyl, 3,4-dimethyl-phenyl, 3,5-dimethyl-phenyl or 4-phenyl-phenyl; or
C₆F₍₅₋ₐ₎Hₐ where 0 ≤ a ≤ 5; or
two of the residues R, R⁴, R⁵, R⁶, R⁷, R⁸, which are adjacent to one another, form a 5 or 6 link ring system or a 5 or 6 link aromatic ring system with the atom to which they are attached, said ring system being unsubstituted or substituted by 1 to 4 residues from the group C₁C₆-alkyl, aryl-, heteroaryl-, C₃-C₇-cycloalkyl, halogen, OR^{a} SR^{a}, NR^{a}R^{b}, COOR^{a}, CO-NR^{a}R^{b} or COR³, wherein R^{a}, R^{b}, independently of one another, represent hydrogen, C₁C₆-alkyl, C₁C₆-alkyl halide, cyclopentyl, cyclohexyl, phenyl, tolyl or benzyl;
preferably
two of the residues R, R⁴, R⁵, R⁶, R⁷, R⁸, which are adjacent to one another, form a 5 or 6 link ring system or a 5 or 6 link aromatic ring system with the atom to which they are attached, said ring system being unsubstituted or substituted by 1 to 4 residues from the group C₁C₆-alkyl, aryl-, heteroaryl-, C₃-C₇-cycloalkyl, halogen, OR^{a} SR^{a}, NR^{a}R^{b}, COOR^{a}, CO-NR^{a}R^{b} or COR³, wherein R^{a}, R^{b}, independently of one another, represent hydrogen, C₁C₆-alkyl, C₁C₆-alkyl halide, cyclopentyl, cyclohexyl, phenyl, tolyl or benzyl;
R represents hydrogen, C₁C₃₀-alkyl, C₃-C₁₂-cycloalkyl, C₂-C₃₀-alkenyl, C₃-C₁₂-cycloalkenyl, aryl or heteroaryl, wherein the 6 latter residues can carry one or more halogen residues and/or 1 to 3 residues are chosen from the group C₁C₆-alkyl, aryl, heteroaryl, C₃-C₇-cycloalkyl, halogen, OR^{C} SR^{C}, NR^{c}R^{d}, COR^{C}, COOR^{C}, CO-NR^{c}R^{d}, wherein R^{c} and R^{d} represent, independently of one another, hydrogen, C₁C₆-alkyl, C₁C₆-alkyl halide, cyclopentyl, cyclohexyl, phenyl, tolyl or benzyl;
preferably
hydrogen;
C₁-C₃₀-alkyl, particularly methyl, ethyl, 1-propyl, 2-propyl, 1-butyl, 2-butyl, 2-methyl-1-propyl (isobutyl), 2-methyl-2-propyl (tert-butyl), 1-pentyl, 2-pentyl, 3-pentyl, 2-methyl-1-butyl, 3-methyl-1-butyl, 2-methyl-2-butyl, 3-methyl-2-butyl, 2,2-dimethyl-1-propyl, 1-hexyl, 2-hexyl, 3-hexyl, 2-methyl-1-pentyl, 3-methyl-1- pentyl, 4-methyl-1-pentyl, 2-methyl-2-pentyl, 3-methyl-2-pentyl, 4-methyl-2-pentyl, 2-methyl-3-pentyl, 3-methyl-3-pentyl, 2,2-dimethyl-1-butyl, 2,3-dimethyl-1-butyl, 3,3-dimethyl-1-butyl, 2-ethyl-1-butyl, 2,3-dimethyl-2-butyl, 3,3-dimethyl-2-butyl, heptyl, octyl, nonyl, decyl, undecyl, dodecyl, tridecyl, tetradecyl, pentadecyl, hexadecyl, heptadecyl, octadecyl, nonadecyl, icosyl, henicosyl, docosyl, tricosyl, tetracosyl, pentacosyl, hexacosyl, heptacosy, octacosyl, nonacosyl or triacontyl;
C₁C₆-alkyl, which is substituted by phenyl or C₅-C₇-cycloalkyl, particularly phenylmethyl (benzyl), diphenylmethyl, triphenylmethyl, 2-phenylethyl, 3-phenylpropyl, cyclopentylmethyl, 2-cyclopentylethyl, 3-cyclopentylpropyl, cyclohexylmethyl, 2-cyclohexylethyl or 3-cyclohexylpropyl;
CₙF_{2(n-a)-(1-b)}H_{2a-b} where n ≤30, 0 ≤ a ≤ n and b = 0 or 1, particularly CF₃, C₂F₅, C₆F₁₃, C₈F₁₇, C₁₀F₂₁ or C₁₂F₂₅;
C₃-C₁₂-cycloalkyl, particularly cyclopentyl or cyclohexyl;
C₃-C₁₂-cycloalkyl, which is substituted by C₁C₆-alkyl, particularly 2-methyl-1-cyclopentyl, 3-methyl-1-cyclopentyl, 2-methyl-1-cyclohexyl, 3-methyl-1-cyclohexyl or 4-methyl-1-cyclohexyl;
CₙF_{2(n-a)-(1-b)}H_{2a-b} where n ≤ 30, 0 ≤ a ≤ n and b = 0 or 1;
C₂-C₃₀-alkenyl, particularly 2-propenyl, 3-butenyl, cis-2-butenyl or trans-2-butenyl; CₙF_{2(n-a)-(1-b)}H_{2a-b} where n ≤ 30, 0 ≤ a ≤ n and b = 0 or 1;
C₃-C₁₂-cycloalkenyl, particularly 3-cyclopentenyl, 2-cyclohexenyl, 3-cyclohexenyl or 2,5-cyclohexadienyl;
CₙF_{2(n-a)-(1-b)}H_{2a-b} where n ≤30, 0 ≤ a ≤ n and b = 0 or 1;
aryl or heteroaryl with 2 to 30 carbon atoms, particularly phenyl, 1-naphthyl, 2-naphthyl, 1-pyrrolyl, 2-pyrrolyl, 3-pyrrolyl, 2-pyridinyl, 3-pyridinyl or 4-pyridinyl;
aryl or heteroaryl with 6 to 30 carbon atoms, which are substituted by one to three C₁C₆-alkyl or phenyl residues, particularly 2-methyl-phenyl (2-tolyl), 3-methyl-phenyl (3-tolyl), 4-methyl-phenyl, 2-ethyl-phenyl, 3-ethyl-phenyl, 4-ethyl-phenyl, 2,3-dimethyl-phenyl, 2,4-dimethyl-phenyl, 2,5-dimethyl-phenyl, 2,6-dimethyl-phenyl, 3,4-dimethyl-phenyl, 3,5-dimethyl-phenyl or 4-phenyl-phenyl; or
C₆F₍₅₋ₐ₎Hₐ where 0≤ a ≤ 5;
R¹, R²' represent, independently of one another, a C₁-C₃₀-alkyl, C₃-C₁₂-cycloalkyl, C₂-C₃₀-alkenyl, C₃-C₁₂-cycloalkenyl, C₂-C₃₀-alkinyl, aryl or heteroaryl, wherein the 7 latter residues can carry one or more halogen residues and/or 1 to 3 residues chosen from the group C₁C₆-alkyl, aryl, heteroaryl, C₃-C₇-cycloalkyl, halogen, OR^{C} SR^{C}, NR^{c}R^{d}, COR^{C}, COOR^{C}, CO-NR^{c}R^{d}, wherein R^{c} and R^{d} represent hydrogen, C₁C₆-alkyl, C₁C₆-alkyl halide, cyclopentyl, cyclohexyl, phenyl, tolyl or benzyl;
preferably
C₁-C₃₀-alkyl, particularly methyl, ethyl, 1-propyl, 2-propyl, 1-butyl, 2-butyl, 2-methyl-1-propyl (isobutyl), 2-methyl-2-propyl (tert-butyl), 1-pentyl, 2-pentyl, 3-pentyl, 2-methyl-1-butyl, 3-methyl-1-butyl, 2-methyl-2-butyl, 3-methyl-2-butyl, 2,2-dimethyl-1-propyl, 1-hexyl, 2-hexyl, 3-hexyl, 2-methyl-1-pentyl, 3-methyl-1- pentyl, 4-methyl-1-pentyl, 2-methyl-2-pentyl, 3-methyl-2-pentyl, 4-methyl-2-pentyl, 2-methyl-3-pentyl, 3-methyl-3-pentyl, 2,2-dimethyl-1-butyl, 2,3-dimethyl-1-butyl, 3,3-dimethyl-1-butyl, 2-ethyl-1-butyl, 2,3-dimethyl-2-butyl, 3,3-dimethyl-2-butyl, heptyl, octyl, nonyl, decyl, undecyl, dodecyl, tridecyl, tetradecyl, pentadecyl, hexadecyl, heptadecyl, octadecyl, nonadecyl, icosyl, henicosyl, docosyl, tricosyl, tetracosyl, pentacosyl, hexacosyl, heptacosyl, octacosyl, nonacosyl or triacontyl;
C₁-C₆-alkyl, which is substituted by phenyl or C₅-C₇-cycloalkyl, particularly phenylmethyl (benzyl), diphenylmethyl, triphenylmethyl, 2-phenylethyl, 3-phenylpropyl, cyclopentylmethyl, 2-cyclopentylethyl, 3-cyclopentylpropyl, cyclohexylmethyl, 2-cyclohexylethyl or 3-cyclohexylpropyl;
CₙF_{2(n-a)-(1-b)}H_{2a-b} where n ≤30, 0 ≤ a ≤ n and b = 0 or 1, particularly CF₃, C₂F₅, C₆F₁₃, C₈F₁₇, C₁₀F₂₁ or C₁₂F₂₅;
C₃-C₁₂-cycloalkyl, particularly cyclopentyl or cyclohexyl;
C₃-C₁₂-cycloalkyl, which is substituted by C₁-C₆-alkyl, particularly 2-methyl-1-cyclopentyl, 3-methyl-1-cyclopentyl, 2-methyl-1-cyclohexyl, 3-methyl-1-cyclohexyl or 4-methyl-1-cyclohexyl;
CₙF_{2(n-a)-(1-b)}H_{2a-d} where n ≤30, 0 ≤ a ≤ n and b = 0 or 1;
C₂-C₃₀-alkenyl, particularly 2-propenyl, 3-butenyl, cis-2-butenyl or trans-2-butenyl; CₙF_{2(n-a)-(1-b)}H_{2a-b} where n ≤30, 0 ≤ a ≤ n and b = 0 or 1;
C₃-C₁₂-cycloalkenyl, particularly 3-cyclopentenyl, 2-cyclohexenyl, 3-cyclohexenyl or 2,5-cyclohexadienyl;
CₙF_{2(n-a)-(1-b)}H_{2a-b} where n ≤30, 0 ≤ a ≤ n and b = 0 or 1;
aryl or heteroaryl with 2 to 30 carbon atoms, particularly phenyl, 1-naphthyl, 2-naphthyl, 1-pyrrolyl, 2-pyrrolyl, 3-pyrrolyl, 2-pyridinyl, 3-pyridinyl or 4-pyridinyl;
aryl or heteroaryl with 6 to 30 carbon atoms, which are substituted by one to three C₁-C₆-alkyl or phenyl residues, particularly 2-methyl-phenyl (2-tolyl), 3-methyl-phenyl (3-tolyl), 4-methyl-phenyl, 2-ethyl-phenyl, 3-ethyl-phenyl, 4-ethyl-phenyl, 2,3-dimethyl-phenyl, 2,4-dimethyl-phenyl, 2,5-dimethyl-phenyl, 2,6-dimethyl-phenyl, 3,4-dimethyl-phenyl, 3,5-dimethyl-phenyl or 4-phenyl-phenyl; or
C₆F₍₅₋ₐ₎Hₐ where 0≤ a ≤ 5; or
R^{1'} and R^{2'} together form a -CHR^{a}-CHR^{b} chain, -CHR^{a}-CH₂-CHR^{b} chain or a -C₆H₄-1,2-diyl residue, wherein R^{a} and R^{b} represent, independently of one another, hydrogen or C₁-C₆-alkyl;
preferably -CH₂-CH₂-, -CH₂-CH₂-CH₂-, -CH(CH₃)-CH₂-, -CH₂-CH(CH₃)-, -CH(CH₃)-CH(CH₃)- or - C₆H₄-1, 2-diyl-;

5. The method according to one of the claims 1 to 4, **characterised in that** a quaternary carbonate of formula I is produced, wherein
R¹, R²' represent, independently of one another, C₁C₃₀-alkyl, C₃-C₁₂-cycloalkyl, C₂-C₃₀-alkenyl, C₃-C₁₂-cycloalkenyl, C₂-C₃₀-alkinyl, aryl or heteroaryl, wherein the 7 latter residues can carry one or more halogen residues and/or 1 to 3 residues chosen from the group C₁C₆-alkyl, aryl, heteroaryl, C₃-C₇-cycloalkyl, halogen, OR^{c} SR^{c}, NR^{c}R^{d}, COR^{c}, COOR^{c}, CO-NR^{c}R^{d}, wherein R^{c} and R^{d} represent hydrogen, C₁C₆-alkyl, C₁C₆-alkyl halide, cyclopentyl, cyclohexyl, phenyl, tolyl or benzyl;
preferably
C₁C₃₀-alkyl, particularly methyl, ethyl, 1-propyl, 2-propyl, 1-butyl, 2-butyl, 2-methyl-1-propyl (isobutyl), 2-methyl-2-propyl (tert-butyl), 1-pentyl, 2-pentyl, 3-pentyl, 2-methyl-1-butyl, 3-methyl-1-butyl, 2-methyl-2-butyl, 3-methyl-2-butyl, 2,2-dimethyl-1-propyl, 1-hexyl, 2-hexyl, 3-hexyl, 2-methyl-1-pentyl, 3-methyl-1-pentyl, 4-methyl-1-pentyl, 2-methyl-2-pentyl, 3-methyl-2-pentyl, 4-methyl-2-pentyl, 2-methyl-3-pentyl, 3-methyl-3-pentyl, 2,2-dimethyl-1-butyl, 2,3-dimethyl-1-butyl, 3,3-dimethyl-1-butyl, 2-ethyl-1-butyl, 2,3-dimethyl-2-butyl, 3,3-dimethyl-2-butyl, heptyl, octyl, nonyl, decyl, undecyl, dodecyl, tridecyl, tetradecyl, pentadecyl, hexadecyl, heptadecyl, octadecyl, nonadecyl, icosyl, henicosyl, docosyl, tricosyl, tetracosyl, pentacosyl, hexacosyl, heptacosy, octacosyl, nonacosyl or triacontyl;
C₁C₆-alkyl, which is substituted by phenyl or C₅-C₇-cycloalkyl, particularly phenylmethyl (benzyl), diphenylmethyl, triphenylmethyl, 2-phenylethyl, 3-phenylpropyl, cyclopentylmethyl, 2-cyclopentylethyl, 3-cyclopentylpropyl, cyclohexylmethyl, 2-cyclohexylethyl or 3-cyclohexylpropyl;
CₙF_{2(n-a)-(1-b)}H_{2a-b} where n ≤ 30, 0 ≤ a ≤ n and b = 0 or 1, particularly CF₃, C₂F₅, C₆F₁₃, C₈F₁₇, C₁₀F₂₁ or C₁₂F₂₅;
C₃-C₁₂-cycloalkyl, particularly cyclopentyl or cyclohexyl;
C₃-C₁₂-cycloalkyl, which is substituted by C₁-C₆-alkyl, particularly 2-methyl-1-cyclopentyl, 3-methyl-1-cyclopentyl, 2-methyl-1-cyclohexyl, 3-methyl-1-cyclohexyl or 4-methyl-1-cyclohexyl;
CₙF_{2(n-a)-(1-b)}H_{2a-b} where n ≤30, 0 ≤ a ≤ n and b = 0 or 1;
C₂-C₃₀-alkenyl, particularly 2-propenyl, 3-butenyl, cis-2-butenyl or trans-2-butenyl; CₙF_{2(n-a)-(1-b)}H_{2a-b} where n ≤ 30, 0 ≤ a ≤ n and b = 0 or 1;
C₃-C₁₂-cycloalkenyl, particularly 3-cyclopentenyl, 2-cyclohexenyl, 3-cyclohexenyl or 2,5-cyclohexadienyl;
CₙF_{2(n-a)-(1-b)}H_{2a-b} where n ≤ 30, 0 ≤ a ≤ n and b = 0 or 1;
aryl or heteroaryl mit 2 to 30 carbon atoms, particularly phenyl, 1-naphthyl, 2-naphthyl, 1-pyrrolyl, 2-pyrrolyl, 3-pyrrolyl, 2-pyridinyl, 3-pyridinyl or 4-pyridinyl;
aryl or heteroaryl with 6 to 30 carbon atoms, which are substituted by one to three C₁-C₆-alkyl or phenyl residues, particularly 2-methyl-phenyl (2-tolyl), 3-methyl-phenyl (3-tolyl), 4-methyl-phenyl, 2-ethyl-phenyl, 3-ethyl- phenyl, 4-ethyl-phenyl, 2,3-dimethyl-phenyl, 2,4-dimethyl-phenyl, 2,5-dimethyl-phenyl, 2,6-dimethyl-phenyl, 3,4-dimethyl-phenyl, 3,5-dimethyl-phenyl or 4-phenyl-phenyl; or
C₆F₍₅₋ₐ₎Hₐ where 0 ≤ a ≤ 5.

6. The method according to one of the claims 1 to 4, **characterised in that** a quaternary carbonate of formula I is produced, wherein
R^{1'} and R²' together form a -CHR^{a}-CHR^{b} chain, a -CHR^{a}-CH₂-CHR^{b} chain or a -C₆H₄-1,2-diyl residue, wherein R^{a} and R^{b} represent, independently of one another, hydrogen or C₁-C₆-alkyl;
preferably
- CH₂-CH₂-, -CH₂-CH₂-CH₂-, -CH(CH₃)-CH₂-,-CH₂-CH(CH₃)-, -CH(CH₃)-CH(CH₃)- or - C₆H₄-1,2-diyl-;

7. The method according to one of the claims 1 to 6, **characterised in that** a quaternary carbonate of formula I is produced, wherein
[A⁺] is a heteroaromatic cation chosen from the group 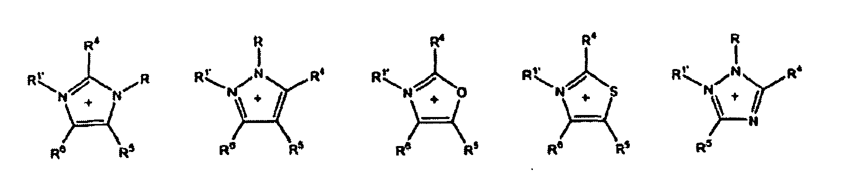 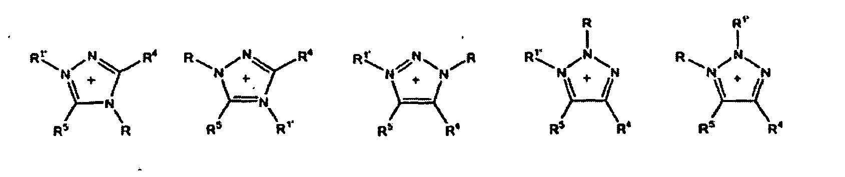 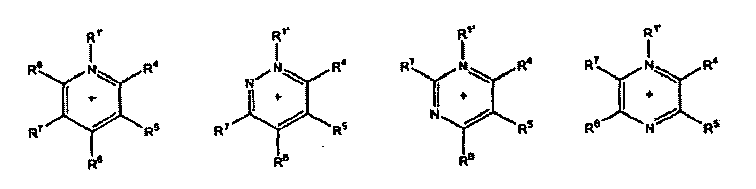 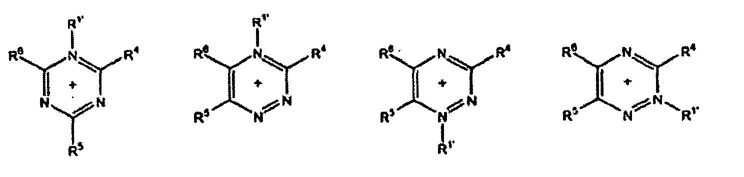 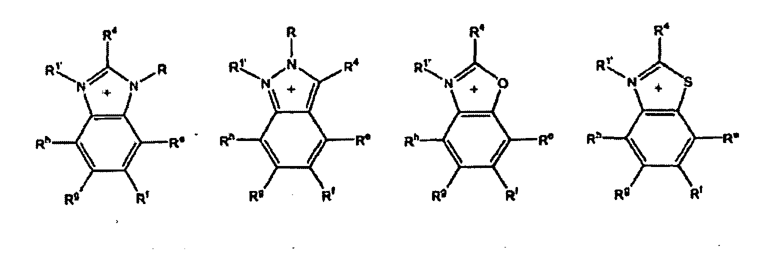 wherein
R⁴, R⁵, R⁶, R⁷, R⁸ represent, independently of one another, hydrogen, halogen, OR^{c}, COR^{c}, C₁-C₃₀-alkyl, C₃-C₁₂-cycloalkyl, C₂-C₃₀-alkenyl, C₃-C₁₂-cycloalkenyl, aryl or heteroaryl, wherein the 6 latter residues can carry one or more halogen residues and/or 1 to 3 residues chosen from the group C₁-C₆-alkyl, aryl, heteroaryl, C₃-C₇-cycloalkyl, halogen, OR^{C} SR^{C}, NR^{c}R^{d}, COR^{c}, COOR^{C}, CO-NR^{c}R^{d}, and wherein R^{c} and R^{d} represent hydrogen, C₁C₆-alkyl, C₁C₆-alkyl halide, cyclopentyl, cyclohexyl, phenyl, tolyl or benzyl;
preferably
hydrogen;
halogen
C₁-C₃₀-alkyl, particularly methyl, ethyl, 1-propyl, 2-propyl, 1-butyl, 2-butyl, 2-methyl-1-propyl (isobutyl), 2-methyl-2-propyl (tert-butyl), 1-pentyl, 2-pentyl, 3-pentyl, 2-methyl-1-butyl, 3-methyl-1-butyl, 2-methyl-2-butyl, 3-methyl-2-butyl, 2,2-dimethyl-1-propyl, 1-hexyl, 2-hexyl, 3-hexyl, 2-methyl-1-pentyl, 3-methyl-1- pentyl, 4-methyl-1-pentyl, 2-methyl-2-pentyl, 3-methyl-2-pentyl, 4-methyl-2-pentyl, 2-methyl-3-pentyl, 3-methyl-3-pentyl, 2,2-dimethyl-1-butyl, 2,3-dimethyl-1-butyl, 3,3-dimethyl-1-butyl, 2-ethyl-1-butyl, 2,3-dimethyl-2-butyl, 3,3-dimethyl-2-butyl, heptyl, octyl, nonyl, decyl, undecyl, dodecyl, tridecyl, tetradecyl, pentadecyl, hexadecyl, heptadecyl, octadecyl, nonadecyl, icosyl, henicosyl, docosyl, tricosyl, tetracosyl, pentacosyl, hexacosyl, heptacosy, octacosyl, nonacosyl or triacontyl;
C₁C₆-alkyl, which is substituted by phenyl or C₅-C₇-cycloalkyl, particularly phenylmethyl (benzyl), diphenylmethyl, triphenylmethyl, 2-phenylethyl, 3-phenylpropyl, cyclopentylmethyl, 2-cyclopentylethyl, 3-cyclopentylpropyl, cyclohexylmethyl, 2-cyclohexylethyl or 3-cyclohexylpropyl;
CₙF_{2(n-a)-(1-b)}H_{2a-b} where n ≤30, 0 ≤ a ≤ n and b = 0 or 1, particularly CF₃, C₂F₅, C₆F₁₃, C₈F₁₇, C₁₀F₂₁ or C₁₂F₂₅;
OR^{a}, particularly methoxy, ethoxy; or
COR^{a}, particularly formyl or acetyl;
C₃-C₁₂-cycloalkyl, particularly cyclopentyl or cyclohexyl;
C₃-C₁₂-cycloalkyl, which is substituted by C₁C₆-alkyl, particularly 2-methyl-1-cyclopentyl, 3-methyl-1-cyclopentyl, 2-methyl-1-cyclohexyl, 3-methyl-1-cyclohexyl or 4-methyl-1-cyclohexyl;
CₙF_{2(n-a)-(1-b)}H_{2a-b} where n ≤30, 0 ≤ a ≤ n and b = 0 or 1;
C₂-C₃₀-alkenyl, particularly 2-propenyl, 3-butenyl, cis-2-butenyl or trans-2-butenyl; CₙF_{2(n-a)-(1-b)}H_{2a-b} where n ≤30, 0 ≤ a ≤ n and b = 0 or 1;
C₃-C₁₂-cycloalkenyl, particularly 3-cyclopentenyl, 2-cyclohexenyl, 3-cyclohexenyl or 2,5-cyclohexadienyl;
CₙF_{2(n-a)-(1-b)}H_{2a-b} where n ≤30, 0 ≤ a ≤ n and b = 0 or 1;
aryl or heteroaryl with 2 to 30 carbon atoms, particularly phenyl, 1-naphthyl, 2-naphthyl, 1-pyrrolyl, 2-pyrrolyl, 3-pyrrolyl, 2-pyridinyl, 3-pyridinyl or 4-pyridinyl;
aryl or heteroaryl with 6 to 30 carbon atoms, which are substituted by one to three C₁-C₆-alkyl or phenyl residues, particularly 2-methyl-phenyl (2-tolyl), 3-methyl-phenyl (3-tolyl), 4-methyl-phenyl, 2-ethyl-phenyl, 3-ethyl-phenyl, 4-ethyl-phenyl, 2,3-dimethyl-phenyl, 2,4-dimethyl-phenyl, 2,5-dimethyl-phenyl, 2,6-dimethyl-phenyl, 3,4-dimethyl-phenyl, 3,5-dimethyl-phenyl or 4-phenyl-phenyl; or
C₆F₍₅₋ₐ₎Hₐ where 0 ≤ a ≤ 5;
R^{e}, R^{f}, R⁹, R^{h} represent, independently of one another, hydrogen, C₁-C₆-alkyl, aryl-, heteroaryl-, C₃-C₇-cycloalkyl, halogen, OR^{C} SR^{C}, NR^{c}R^{d}, COOR^{c}, CO-NR^{c}R^{d} or COR^{c} is substituted, wherein R^{c}, R^{d} represent, independently of one another, hydrogen, C₁C₆-alkyl, C₁C₆-alkyl halide, cyclopentyl, cyclohexyl, phenyl, tolyl or benzyl;
preferably
hydrogen, halogen or C₁C₆-alkyl, particularly hydrogen or C₁-C₆-alkyl;
R represents hydrogen, C₁C₃₀-alkyl, C₃-C₁₂-cycloalkyl, C₂-C₃₀-alkenyl, C₃-C₁₂-cycloalkenyl, aryl or heteroaryl, wherein the 6 latter residues can carry one or more halogen residues and/or 1 to 3 residues are chosen from the group C₁C₆-alkyl, aryl,
heteroaryl, C₃-C₇-cycloalkyl, halogen, OR^{C} SR^{C}, NR^{c}R^{d}, COR^{C}, COOR^{C}, CO-NR^{c}R^{d}, wherein R^{c} and R^{d} represent, independently of one another, hydrogen, C₁C₆-alkyl, C₁C₆-alkyl halide, cyclopentyl, cyclohexyl, phenyl, tolyl or benzyl;
preferably
C₁-C₃₀-alkyl, particularly methyl, ethyl, 1-propyl, 2-propyl, 1-butyl, 2-butyl, 2-methyl-1-propyl (isobutyl), 2-methyl-2-propyl (tert-butyl), 1-pentyl, 2-pentyl, 3-pentyl, 2-methyl-1-butyl, 3-methyl-1-butyl, 2-methyl-2-butyl, 3-methyl-2-butyl, 2,2-dimethyl-1-propyl, 1-hexyl, 2-hexyl, 3-hexyl, 2-methyl-1-pentyl, 3-methyl-1- pentyl, 4-methyl-1-pentyl, 2-methyl-2-pentyl, 3-methyl-2-pentyl, 4-methyl-2-pentyl, 2-methyl-3-pentyl, 3-methyl-3-pentyl, 2,2-dimethyl-1-butyl, 2,3-dimethyl-1-butyl, 3,3-dimethyl-1-butyl, 2-ethyl-1-butyl, 2,3-dimethyl-2-butyl, 3,3-dimethyl-2-butyl, heptyl, octyl, nonyl, decyl, undecyl, dodecyl, tridecyl, tetradecyl, pentadecyl, hexadecyl, heptadecyl, octadecyl, nonadecyl, icosyl, henicosyl, docosyl, tricosyl, tetracosyl, pentacosyl, hexacosyl, heptacosy, octacosyl, nonacosyl or triacontyl;
C₁C₆-alkyl, which is substituted by phenyl or C₅-C₇-cycloalkyl, particularly phenylmethyl (benzyl), diphenylmethyl, triphenylmethyl, 2-phenylethyl, 3-phenylpropyl, cyclopentylmethyl, 2-cyclopentylethyl, 3-cyclopentylpropyl, cyclohexylmethyl, 2-cyclohexylethyl or 3-cyclohexylpropyl;
CₙF_{2(n-a)-(1-b)}H_{2a-b} where n ≤30, 0 ≤ a ≤ n and b = 0 or 1, particularly CF₃, C₂F₅, C₆F₁₃, C₈F₁₇, C₁₀F₂₁ or C₁₂F₂₅;
C₃-C₁₂-cycloalkyl, particularly cyclopentyl or cyclohexyl;
C₃-C₁₂-cycloalkyl, which is substituted by C₁C₆-alkyl, particularly 2-methyl-1-cyclopentyl, 3-methyl-1-cyclopentyl, 2-methyl-1-cyclohexyl, 3-methyl-1-cyclohexyl or 4-methyl-1-cyclohexyl;
CₙF_{2(n-a)-(1-b)}H_{2a-b} where n ≤30, 0 ≤ a ≤ n and b = 0 or 1;
C₂-C₃₀-alkenyl, particularly 2-propenyl, 3-butenyl, cis-2-butenyl or trans-2-butenyl; CₙF_{2(n-a)-(1-b)}H_{2a-b} where n ≤30, 0 ≤ a ≤ n and b = 0 or 1;
C₃-C₁₂-cycloalkenyl, particularly 3-cyclopentenyl, 2-cyclohexenyl, 3-cyclohexenyl or 2,5-cyclohexadienyl;
CₙF_{2(n-a)-(1-b)}H_{2a-b} where n ≤30, 0 ≤ a ≤ n and b = 0 or 1;
aryl or heteroaryl with 2 to 30 carbon atoms, particularly phenyl, 1-naphthyl, 2-naphthyl, 1-pyrrolyl, 2-pyrrolyl, 3-pyrrolyl, 2-pyridinyl, 3-pyridinyl or 4-pyridinyl;
aryl or heteroaryl with 6 to 30 carbon atoms, which are substituted by one to three C₁C₆-alkyl or phenyl residues, particularly 2-methyl-phenyl (2-tolyl), 3-methyl-phenyl (3-tolyl), 4-methyl-phenyl, 2-ethyl-phenyl, 3-ethyl-phenyl, 4-ethyl-phenyl, 2,3-dimethyl-phenyl, 2,4-dimethyl-phenyl, 2,5-dimethyl-phenyl, 2,6-dimethyl-phenyl, 3,4-dimethyl-phenyl, 3,5-dimethyl-phenyl or 4-phenyl-phenyl; or
C₆F₍₅₋ₐ₎Hₐ where 0≤ a ≤ 5.

8. The method according to claim 7, **characterised in that** a quaternary carbonate of formula I is produced, wherein
R⁴, R⁵, R⁶, R⁷, R⁸ R^{e}, R^{f}, R⁹, R^{h} represent, independently of one another, hydrogen, halogen or C₁C₆-alkyl, preferably hydrogen, halogen or C₁C₆-alkyl;
R represents C₁C₆-alkyl.

9. The method according to one of the claims 1 to 6 or 8, **characterised in that** a quaternary carbonate of formula I is produced, wherein
[A⁺] is a quaternary ammonium cation [R^{1'}R¹R²R³N]⁺ or a phosphonium cation [R^{1'}R¹R²R³P]⁺.

10. The method according to one of the claims 1 to 9, **characterised in that** a Lewis acid containing at least one element from groups 1 to 13 in the periodic table or the group containing lanthanides (atomic numbers 57 to 71) is used, preferably a Lewis acid containing at least one element chosen from the group comprising lithium, titanium, zirconium, vanadium, niobium, tantalum, chromium, manganese, iron, cobalt, nickel, copper, silver, gold, aluminium, cerium, lanthanum, neodymium, samarium, gadolinium, erbium and lutetium, particularly from the group comprising lithium, copper, silver, samarium, cerium, titanium and aluminium.

11. The method according to claim 10, **characterised in that** at least an oxide, carbonate, halide or silicate of lithium, copper, silver, samarium, cerium, titanium or aluminium or mixtures thereof, or a zeolite is used as the Lewis acid.

12. The method according to claim 10 or 11, **characterised in that** the Lewis acid is used in 0.001 mol% to 90 mol%, preferably 0.1 mol% to 10 mol%, particularly preferably 0.5 mol% to 5 mol% relative to the amine R¹R²R³N of formula IIa, phosphine R¹R²R³P of formula IIb, sulphide R¹R²S of formula IIc or the heteroaromatic of formula IId.

13. The method according to one of the claims 1 to 12, **characterised in that** the alkyl-and/or arylcarbonate of formula III is used in 0.5 to 10 mole equivalents, preferably 1.0 to 5.0 mole equivalents, particularly preferably 1.1 to 2.0 mole equivalents, relative to the amine R¹R²R³N of formula IIa, phosphine R¹R²R³P of formula IIb, sulphide R¹R²S of formula IIc or the heteroaromatic of formula IId.

14. The method according to one of the claims 1 to 13, **characterised in that** the reaction takes place at a temperature of 30°C to 350°C, preferably 50°C to 150°C, particularly preferably 80°C to 120°C.

15. The method according to one of the claims 1 to 14, **characterised in that** the reaction time is within the range of 0.1 hours to 14 days, preferably 0.5 hours to 5 days, particularly preferably 1 hour to 24 hours.

16. The method according to one of the claims 1 to 15, **characterised in that** the reaction takes place in the presence of a reducing agent, preferably an aluminium hydride, a boron hydride or in the presence of hydrogen and a catalyst chosen from the group of platinum metals; particularly NaAlH₄, LiAlH₄, NaBH₄, LiBH₄ or H₂ in the presence of a platinum group catalyst, such as Pt, Pd, Rh, Ru or Ir.

17. The method according to one of the claims 1 to 16, **characterised in that** the reaction takes place in an air or steam atmosphere or in a protective atmosphere, particularly with nitrogen or argon.

18. The method according to one of the claims 1 to 17, **characterised in that** the reaction takes place in batches, semi-continuously or continuously.

19. The method according to one of the claims 1 to 18, **characterised in that** the reaction takes place in substance or in the presence of a solvent.

20. The method according to claim 19, **characterised in that** the reaction takes place in substance.

21. The method according to claim 19, **characterised in that** the reaction takes place in the presence of a solvent, preferably an alcohol, ether, dialkyl-ormamide, ketone, sulfoxide, nitril or a mixture thereof, particularly an alcohol, particularly preferably methanol, ethanol, isopropanol, n-propanol, n-butanol, sec-butanol, tert-butanol, pentanol, hexanol, heptanol, octanol or phenol or a mixture thereof, wherein the proportion of solvent is around 1 to 90%, preferably around 10 to 50%, preferably around 20 to 30% relative to the total mass of the reaction mixture.

22. The method according to claim 21, **characterised in that** alcohol is used as the solvent, which at least corresponds to one of the residues of the alkyl- and/or arylcarbonate of formula III.

23. The method according to one of the claims 1 to 22, **characterised in that** the reaction mixture is stirred, shaken or combined in some other way.

24. The method according to claims 1 to 23, **characterised in that** the alkyl- and/or arylcarbonate of formula III is obtained from the reaction of CO₂, CO or CO/O₂ or mixtures thereof with an alcohol R^{1'}OH or R^{2'}OH or a mixture thereof, in the presence of a Lewis acid at a pressure of 1 to 300 bar, preferably 5 to 100 bar, particularly preferably 10 to 50 bar.

25. The method according to claim 24, **characterised in that** the alkyl- and/or arylcarbonate of formula III is produced in situ.

26. The method of producing quaternary carbonates of formula I, as described in claims 1 to 9, **characterised in that** an amine R¹R²R³N of formula IIa, the phosphine R¹R²R³P of formula IIb, sulphide R¹R²S of formula IIc or the heteroaromatics of formula IId react with an alcohol R^{1'}OH or R^{2'}OH or a mixture thereof and CO₂, CO/O₂ or CO combined with an oxidising agent, or mixtures thereof, in the presence of at least one Lewis acid at 1 to 300 bar, preferably 5 to 100 bar, particularly preferably 10 to 50 bar.

27. The method according to one of the claims 24 to 26, **characterised in that** the Lewis acid is used in 0.001 mol% to 90 mol%, preferably 0.1 mol% to 10 mol%, particularly preferably 0.5 mol% to 5 mol%, relative to the amine R¹R²R³N of formula IIa, phosphine R¹R²R³P of formula IIb, sulphide R¹R²S of formula IIc or the heteroaromatics of formula IId.

28. The method according to one of claims 24 to 27, **characterised in that** the alcohol R^{1'}OH or R^{2'}OH or a mixture thereof is used in 0.5 to 20 mole equivalents, preferably 1.0 to 5 mole equivalents, particularly preferably 1.5 to 2.5 mole equivalents, relative to the amine R¹R²R³N of formula IIa, phosphine R¹R²R³P of formula IIb, sulphide R¹R²S of formula IIc or the heteroaromatic of formula IId.

29. The method according to one of the claims 24 to 28, **characterised in that** the carbon dioxide needed to produce the alkyl- and/or arylcarbonate of formula III is generated by the reaction of a quaternary carbonate of formula I and/or a quaternary compound of formula IVa and/or Ivb 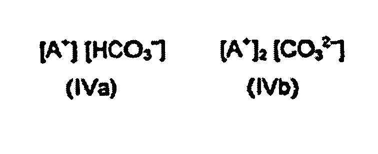 wherein
[A⁺] has the meaning indicated in claims 1 to 27,
with a Brønsted acid HZ.

30. The method according to one of the claims 24 to 28, **characterised in that** the alcohol R^{2'}OH needed to produce the alkyl- and/or arylcarbonate of formula III is generated by the reaction of a quaternary carbonate of formula I with a Bronsted acid HZ.

31. The method according to one of the claims 24 to 30, **characterised in that** the Lewis acid used for the alkylation or arylation of the amine R¹R²R³N of formula IIa, of the phosphine R¹R²R³P of formula IIb, of the sulphide R¹R²S of formula IIc or of the heteroaromatic of formula IId with the alkyl- and/or arylcarbonate of formula III is identical to the Lewis acid used for the in situ generation of the alkyl- and/or arylcarbonate of formula III.

32. The method according to one of the claims 1 to 31, **characterised in that** the reaction takes place in the liquid or gas phase.

33. The method according to one of the claims 1 to 32, **characterised in that** the processing of the reaction mixtures involves distillation, vacuum distillation, thin-film evaporation, molecular evaporation, rotation evaporation, spray drying, osmosis, pervaporation, gas or steam stripping, freezing out, freeze-drying, chemical or physical adsorption or other suitable processes.

34. A method for producing a compound of formula V,
[A⁺]ₙ [Zⁿ⁻] (V)
wherein
[A⁺] has the meaning indicated in claims 1 to 33 and
[Zⁿ⁻] represents a univalent, bivalent, tervalent or quadrivalent anion;
**characterised in that** a quaternary carbonate of formula I is produced according to one of the methods 1 to 33 and the carbonate of formula I thereby obtained reacts with a Brønsted acid [H⁺]ₙ [Zⁿ⁻], its anhydride or a salt [cation^{m+}]ₙ[Zⁿ⁻]ₘ.

## Revendications

1. Procédé de production de carbonates quaternaires de la formule I,
[A]⁺ [R^{2'}OCO₂]⁻ (I)
dans lequel
(A]⁺ représente un cation d'ammonium quaternaire [R^{1'}R¹R²R³N]⁺, un cation de phosphonium [R^{1'}R¹R²R³P]⁺, un cation de sulfonium [R^{1'}R¹R²S]⁺ ou un cation hétéroaromatique,
R¹, R², R³ représentent, indépendamment l'un de l'autre de l'hydrogène, un groupe
alkyle, alkényle, alkinyle, un groupe cycloalkyle, cycloalkényle, aryle ou hétéoroaryle substitué le cas échéant; ou
deux des radicaux R¹, R², R³ forment ensemble avec l'hétéroatome auquel ils sont liés un
anneau, celui-ci étant saturé ou insaturé, insubstitué ou substitué et cette chaîne pouvant être interrompue par un ou plusieurs hétéroatome(s), choisis dans un groupe O, S, NH ou groupe N-alkyle en C₁ à C₄;
R^{1'}, R^{2'} représentent, indépendamment l'un de l'autre un groupe alkyle, alkényle,
alkinyle, un groupe cycloalkyle, cycloalkényle, aryle ou hétéoroaryle substitué le cas échéant; ou
R^{1'} et R^{2'} forment en commun une chaîne -CHR^{a}-CHR^{b}, une chaîne -CHR^{a}-CH₂-CHR^{b}
ou un radical 1,2-di-yle C₆H₄-substitué le cas échéant, R^{a} et représentent indépendamment l'un de l'autre de l'hydrogène ou un groupe alkyle en C₁ à C₆; alors que dans ce cas, il est obtenu un composé zwiterrionique de la formule I dans lequel R^{1'} et [R^{2'}OCO₂]⁻ forment le radical -OCO- CHR^{a}- CHR^{b}-, -OCO à CHR^{a}- CH₂-CHR^{b}- ou -OCO-1 à C₆H₄-2-yle-, substitué le cas échéant (composé l');
**caractérisé en ce**
**qu'**on transforme en présence de quantité catalytiques d'un acide de Lewis une amine R¹R²R³N de la formule IIa, une phosphine R¹R²R³P de la formule IIb, un sulfure R¹R²S de la formule IIc ou un hétéroarome de la formule IId, les radicaux R', R² et R³ ayant la signification précitée,
avec un carbonate d'alkyle et/ou d'aryle de la formule III
R^{1'}O(CO)OR^{2'} (III)
R^{1'}, R^{2'} correspondant indépendamment l'un de l'autre à un groupe alkyle,
alkényle, alkinyle, un groupe cycloalkyle, cycloalkényle, aryle ou
hétéoroaryle substitué les cas échéant; ou
R^{1'} et R^{2'} formant en commun une chaîne -CHR^{a}- CHR^{b-}, à CHR^{a} une chaîne -CH₂-CHR^{b}- ou un radical C₆H₄-1,2-di-yle, R^{a} et R^{b} représentant indépendamment l'un de l'autre de l'hydrogène ou un groupe alkyle en C₁ à C₆; alors que dans ce cas, il est obtenu un composé zwiterrionique de la formule I'.

2. Procédé selon I revendication 1, **caractérisé en ce qu'**on produit un carbonate quaternaire de la formule I,
[A]+ représentant un cation d'ammonium quaternaire [R^{1'}R¹R²R³N]⁺, un cation de phosphonium [R^{1'}R¹ R²R³P]⁺, un cation de sulfonium [R^{1'}R¹R²S]⁺ ou un cation hétéroaromatique, lequel est choisi dans un groupe 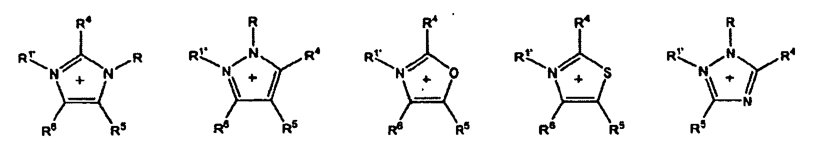 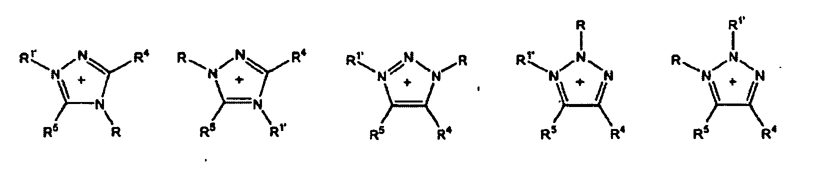 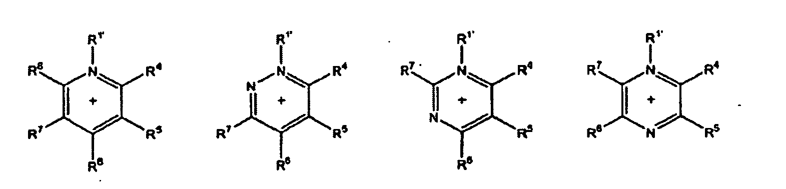 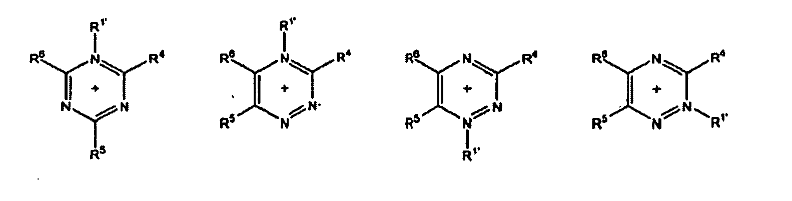 les radicaux ayant la signification suivante:
R^{1'} un groupe alkyle, alkényle, alkinyle, cycloalkyle, cycloalkényle, aryle ou
hétéroaryle, substitué le cas échéant;
R de l'hydrogène, un groupe alkyle, alkényle, alkinyle, cycloalkyle,
cycloalkényle, aryle ou hétéoroaryle, substitué le cas échéant;
R⁴, R⁵, R^{e}, R⁷, R⁸ de l'hydrogène, un halogène, nitro, cyano, OR^{c}, SR^{c}, NR^{c}R^{d}, COR^{c},
COOR^{c}, CO-NR^{c}R^{d}, un groupe alkyle en C₁ à C₃₀, un groupe cycloalkyle en C₃ à C₁₂ un groupe alkényle en C₂ à C₃₀, un groupe cycloalkényle en C₃ à C₁₂, un groupe aryle ou hétéoroaryle, les 6 derniers radicaux cités pouvant porter un ou plusieurs radicaux halogènes et/ou de 1 à 3 radicaux choisis dans le groupe comprenant un groupe alkyle en C₁ à C₆, un groupe aryle, hétéoroaryle, cycloalkyle en C₃ à C₇, halogène, OR^{c}, SR^{c}, NR^{c}R^{d}, COR^{c}, COOR^{c},CO-NR^{c}R^{d}, R^{c} et R^{d} représentant indépendamment l'un de l'autre l'hydrogène, un groupe alkyle en C₁ à C₆ un groupe alkyle halogéné en C¹ à C₆, un groupe cyclopentyle, cyclohexyle, phényle, tolyle ou benzyle; ou deux des radicaux R, ^{R4}, R⁵, R⁶, R⁷, R⁸, lesquels sont voisins formant en commun
avec l'atome auquel ils sont liés un anneau, celui-ci étant insaturé, saturé ou aromatique, insubstitué ou substitué et la chaîne formée par les radicaux concernés pouvant être interrompue par un ou plusieurs hétéroatome(s) choisis dans un groupe composé de O, S, N, NH ou d'un groupe N-alkyle en C¹ à C₄-; ou
dans le cas des composés I' R^{1'} et R^{2'} représentent en commun une chaîne -
CHR^{a}-CHR^{b}-, une chaîne -CHR^{a}-CH₂ -CHR^{b} ou un radical 1,2-di-yle C₆H₄-, R^{a} et R^{b} représentant indépendamment l'un de l'autre de l'hydrogène ou un groupe alkyle en C₁ à C₆.

3. Procédé selon la revendication 1 ou 2, **caractérisé en ce qu'**on produit un carbonate quaternaire de la formule I,
R¹, R², R³ représentant, indépendamment l'un de l'autre l'hydrogène, un groupe
alkyle en C¹ à C₃₀, un groupe cycloalkyle en C₃ à C₁₂, un groupe alkényle en C₂ à C₃₀, un groupe cycloalkényle en C₃ à C₁₂, un groupe alkinyle en C₂ à C₃₀, un groupe aryle ou hétéoroaryle, les 7 derniers radicaux cités pouvant porter un ou plusieurs radicaux halogènes et/ou de 1 à 3 radicaux choisis dans un groupe alkyle en C₁ à C₆, un groupe aryle, hétéoroaryle, un groupe cycloalkyle en C₃ à C₇, halogène, OR^{c}, SR^{c} NR^{c}R^{d}, COR^{c}, COOR^{C}, CO-NR^{c}R^{d}, et R^{c} et R^{d} représentant l'hydrogène, un groupe alkyle en C₁ à C₆, un groupe alkyle halogéné en C₁ à C₆, un groupe cyclopentyle, cyclohexyle, phényle, tolyle ou benzyle; ou
deux des radicaux R¹, R², R³ formant en commun avec l'atome auquel ils sont liés
un anneau, celui-ci étant insaturé ou saturé, insubstitué ou substitué et cette chaîne pouvant être interrompue par un ou plusieurs hétéroatomes choisis dans un groupe O, S, NH ou dans un groupe N-alkyle en C₁ à C_{4;}
R⁴, R⁵, R^{e}, R⁷, R⁸ représentant, indépendamment l'un de l'autre l'hydrogène,
l'halogène, le nitro, lecyano, OR^{c}, SR^{c}, NR^{c}R^{d}, COR^{c}, COOR^{c}, CO-NR^{c}R^{d}, un groupe alkyle en C₁ à C₃₀, un groupe cycloalkyle en C₃ à C₁₂, un groupe alkényle en C₂ à C₃₀, un groupe cycloalkényle en C₃ à C₁₂, un groupe aryle ou hétéoroaryle, les 6 derniers radicaux cités pouvant porter un ou plusieurs radicaux halogènes et/ou de 1 à 3 radicaux choisis dans un groupe comprent un groupe alkyle en C₁ à C₆, un groupe aryle, hétéoroaryle, un groupe cyclocalkyle en C₃ à C₇, halogène, OR^{c}, SR^{c}, NR^{c}R^{d}, COR^{c}, COOR^{c}, CO-NR^{c}R^{d}, et R^{c} et R^{d} représentant l'hydrogène, un groupe alkyle en C₁ à C₆, un groupe alkyle halogéné en C₁ à C₆, un groupe cyclopentyle, cyclohexyle, phényle, tolyle ou benzyle; ou
deux des radicaux R, R⁴, R⁵, R⁶, R⁷, R⁸, lesquels sont voisins, formant en commun
avec l'atome auquel ils sont liés un anneau, celui-ci étant insaturé ou aromatique, insubstitué ou substitué et la chaîne formée par les radicaux concernés pouvant être interrompue par un ou plusieurs hétéroatomes choisis dans un groupe O, S, N, NH ou dans un groupe N-alkyle en C₁ à C₄; ou
R représentant l'hydrogène, un groupe alkyle en C₁ à C₃₀, un groupe cycloalkyle
en C₃ à C₁₂, un groupe alkényle en C₂ à C₃₀, un groupe cycloalkényle en C₃ à C₁₂, un groupe alkinyle en C₂ à C₃₀, un groupe aryle ou hétéoroaryle, les 7 derniers radicaux cités pouvant porter un ou plusieurs radicaux halogènes et/ou de 1 à 3 radicaux choisis dans un groupe alkyle en C₁ à C₆, un groupe aryle, hétéoroaryle, un groupe cycloalkyle en C₃ à C₇, halogène, OR^{c}, SR^{c}, NR^{c}R^{d}, COR^{c}, COOR^{c}, CO-NR^{c}R^{d}, R^{c} et R^{d} représentant l'hydrogène, un groupe alkyle en C¹ à C₆, un groupe alkyle halogéné en C₁ à C₆, un groupe cyclopentyle, cyclohexyle, phényle, tolyle ou benzyle;
R^{1'}, R^{2'} représentant, indépendamment l'un de l'autre un groupe alkyle en C¹ à
C₃₀, un groupe cycloalkyle en C₃ à C₁₂, une groupe alkényle en C₂ à C₃₀, un groupe cycloalkényle en C₃ à C₁₂, un groupe alkinyle en C² à C³⁰, un groupe aryle ou hétéoroaryle, les 7 derniers radicaux cités pouvant porter un ou plusieurs radicaux halogènes et/ou de 1 à 3 radicaux choisis dans un groupe alkyle en C₁ à C₆, aryle, hétéoroaryle, un groupe cycloalkyle en C₃ à C₇, halogène, OR^{c}, SR^{c}, NR^{c}R^{d}, COR^{c}, COOR^{c}, CO-NR^{c}R^{d}, R^{c} et R^{d} représentant l'hydrogène, un groupe alkyle en C₁ à C₆, un groupe alkyle halogéné en C¹ à C₆, un groupe cyclopentyle, cyclohexyle, phényle, tolyle ou benzyle; ou
R^{1'} et R^{2'} formant en commun une chaîne -CHR^{a}-CHR^{b}-, une chaîne -CHR^{a}-CH₂-CHR^{b} ou un radical 1-2-di-yle C₆H₄ substitué le cas échéant, R^{a} et R^{b} représentant, indépendamment l'un de l'autre l'hydrogène ou un groupe alkyle en C₁ à C₆.

4. Procédé selon l'une quelconque des revendications 1 à 3, **caractérisé en ce qu'**on produit un carbonate quaternaire de la formule I,
R¹, R², R³ représentant, indépendamment l'un de l'autre l'hydrogène, un groupe
alkyle en C¹ à C₃₀, un groupe cycloalkyle en C3 à C¹², un groupe alkényle en C₂ à C₃₀, un groupe cycloalkényle en C³ à C₁₂, un groupe aryle ou hétéoroaryle, les 6 derniers radicaux cités pouvant porter un ou plusieurs radicaux halogènes et/ou de 1 à 3 radicaux choisis dans un groupe alkyle en C₁ à C₆, un groupe aryle, hétéoroaryle, un groupe cycloalkyle en C3 à C7, l'halogène, OR^{c}, SR^{c}, NRcR^{d}, COR^{c}, COOR^{c}, CO-NR^{c}R^{d}, et R^{c} et R^{d} représentant l'hydrogène, un groupe alkyle en C₁ à C₆, un groupe alkyle halogéné en C₁ à C₆, un groupe cyclopentyle, cyclohexyle, phényle, tolyle ou benzyle;
l'hydrogène étant représenté de préférence par;
un groupe alkyle en C₁ à C₃₀, notamment le méthyle, l'éthyle, le 1-propyle,
le 2-propyle, le 1-butyle, le 2-butyle, le 2-méthyle-1-propyle (isobutyle), le 2-méthyle-2-propyle (tert.butyle), le 1-pentyle, le 2-pentyle, le 3-pentyle, le 2-méthyle-1-butyle, le 3-méthyle-1-butyle, le 2-méthyle-2-butyle, le 3-méthyle-2-butyle, le-2,2-diméthyle-1-propyle, le 1-hexyle, le 2-hexyle, le 3-hexyle, le 2-méthyle-1-pentyle, le 3-méthyle-1pentyle, le 4-méthyle-1-pentyle, le 2-méthyle-2-pentyle, le 3-méthyle-2-pentyle, le 4-méthyle-2-pentyle, le 2-méthyle-3-pentyle, le 3-méthyle-3-pentyle, le 2,2-diméthyle-1-butyle, le 2,3-diméthyle-1-butyle, le 3,3-diméthyle-1-butyle, le 2-éthyle-1-butyle, le 2,3-diméthyle-2-butyle, le 3,3-diméthyle-2-butyle, l'heptyle, l'octyle, le nonyle, le décyle, l'etécyle, le dodécyle, le tridécyle, le tétradécyle, le pentadécyle, l'hexadécyle, l'heptadécyle, l'octadécyle, le nonadécyle, l'icosyle, l'hénicosyle, le docosyle, le tricosyle, le tétracosyle, le pentacosyle, l'hexacosyle, l'heptacosyle, l'octacosyle, le nonacosyle ou le triacontyle;
un groupe alkyle en C₁ à C₆, qui est substitué par du phényle ou un groupe cycloalkyle en C₅ à C₇, notamment le phénylméthyle (benzyle), le diphénylméthyle, le triphénylméthyle, le 2- phényléthyle, le 3-phénylpropyle, le cyclopentylméthyle, le 2-cyclopentyléthyle, le 3-cyclopentylpropyle, le cyclohexylméthyle, le 2-cyclohexyléthyle ou le 3-cyclohexylpropyle;
CₙF_{2(na)(1-b)}H_{2ab} avec n ≤ 30, 0 ≤ a ≤ n et b = 0 ou 1, notamment CF₃, C₂F₅, C₆F₁₉, CₐF₁₇, C₁₀F₂₁ ou C₁₂F₂₅;
un groupe cycloalkyle en C₃ à C₁₂, notamment le cyclopentyle ou le cyclohexyle;
un groupe cycloalkyle en C₃ à C₁₂, lequel est substitué par un groupe alkyle en C₁ à C₆, notamment le 2-méthyle-1-cyclopentyle, le 3-méthyle-1-cyclopentyle, le 2-méthyle-1-cyclohexyle, le 3-méthyle-1- cyclohexyle ou le 4-méthyle-1-cyclohexyle;
CₙF_{2(n-a)-(1-b)}H_{2a-b} avec ≤ 30, 0 ≤ a ≤ n et b = 0 ou 1;
un groupe alkényle en C₂ à C₃₀, notamment le 2-propényle, le 3-butényle, le cis-2-butényle ou le trans-2-butényle;
CₙF_{2(n-a)-(1-b)}H_{2a-b} avec n ≤ 30, ≤ a ≤ n et b = 0 ou 1;
un groupe cycloalkényle en C₃ à C₁₂, notamment le 3-cyclopentényle, le 2-cyclohexényle, le 3-cyclohexényle ou le 2,5-cyclohexadiényle; CₙF_{2(n-a)-(1-b)}H_{2a-b} avec n ≤ 30, 0 ≤ a ≤ n et b = 0 ou 1;
un groupe aryle ou hétéoroaryle avec de 2 à 30 atomes de carbone, notamment le phényle, le 1-naphthyle, le 2-naphthyle, le 1-pyrrolyle, le 2-pyrrolyle, le 3-pyrrolyle, le 2-pyridinyle, le 3-pyridinyle ou le 4-pyridinyle;
un groupe aryle ou hétéoroaryle avec 6 à 30 atomes de carbone, lesquels sont substitués par de un à trois radicaux d'un groupe alkyle en C₁ à C₆ ou phényle, notamment le 2-méthylphényle (2-tolyle), le 3-méthylphényle (3-tolyle), le 4-méthylphényle, le 2-éthylphényle, le 3-éthylphényle, le 4-éthylphényle, le 2,3-diméthylphényle, le 2,4-diméthylphényle, le 2,5-diméthylphényle, le 2,6-diméthylphényle, le 3,4-diméthylphényle, le 3,5-diméthylphényle ou le 4-phénylphényle; ou C₆F₍₅₋ₐ₎Hₐ, avec 0 ≤ a ≤ 5;
ou
deux des radicaux R¹, R², R³ formant en commun avec l'hétéroatome auquel ils
sont liés une chaîne -(CH₂)ₙ, n étant 4, 5, 6 ou 7, et ladite chaîne pouvant être substituée par de 1 à un maximum de trois radicaux du groupe alkyle en C₁ à C₄ et/ou pouvant être interrompue par O, S, NH ou - un groupe N-alkyle en C₁ à C₄;
R⁴, R⁵, R⁶, R⁷, R⁸ représentant, indépendamment l'un de l'autre l'hydrogène,
l'halogène, OR^{c}, COR^{c}, un groupe alkyle en C¹ à C₃₀,, un groupe cycloalkyle en C₃ à C₁₂, un groupe alkényle en C₂ à C₃₀, un groupe cycloalkényle en C₃ à C₁₂, un groupe aryle ou hétéoroaryle, les 6 derniers radicaux cités pouvant porter un ou plusieurs radicaux halogènes et/ou de 1 à 3 radicaux choisis dans un groupe comprenant un groupe alkyle en C₁ à C₆, un groupe aryle, hétéoroaryle, un groupe cycloalkyle en C₃ à C₇, halogène, OR^{c}, SR^{c}, NR^{c}R^{d}, COR^{C}, COOR^{c}, CO-NR^{c}R^{d}, et R^{c} et R^{d} représentant l'hydrogène, un groupe alkyle en C₁ à C₆, un groupe alkyle halogéné en C₁ à C₆, un groupe cyclopentyle, cyclohexyle, phényle, tolyle ou benzyle;
l'hydrogène étant représenté de préférence par
l'halogène,
un groupe alkyle en C₁ à C₃₀, notamment le méthyle, l'éthyle, le 1-propyle, le 2-propyle, le 1-butyle, le 2-butyle, le 2-méthyle-1-propyle (isobutyle), le 2-méthyle-2-propyle (tert.-butyle), le 1-pentyle, le 2-pentyle, le 3-pentyle, le 2-méthyle-1-butyle, le 3-méthyle-1-butyle, le 2-méthyle-2-butyle, le 3-méthyle-2-butyle, 2,2-diméthyle-1-propyle, le 1-hexyle, le 2-hexyle, le 3-hexyle, le 2-méthyle-1-pentyle, le 3-méthyle-1pentyle, le 4-méthyle-1-pentyle, le 2-méthyle-2-pentyle, le 3-méthyle-2-pentyle, le 4-méthyle-2-pentyle, le 2-méthyle-3-pentyle, le 3-méthyle-3-pentyle, 2,2-diméthyle-1-butyle, 2,3-diméthyle-1-butyle, 3,3-diméthyle-1-butyle, le 2-éthyle-1-butyle, 2,3-diméthyle-2-butyle, 3,3-diméthyle-2-butyle, l'heptyle, l'octyle, le nonyle, le décyle, l'etécyle, le dodécyle, le tridécyle, le tétradécyle, le pentadécyle, l'hexadécyle, l'heptadécyle, l'octadécyle, le nonadécyle, l'icosyle, l'hénicosyle, le docosyle, le tricosyle, le tétracosyle, le pentacosyle, l'hexacosyle, l'heptacosyle, l'octacosyle, le nonacosyle ou le triacontyle;
un groupe alkyle en C₁ à C₆, lequel est substitué par le phényle ou un groupe cycloalkyle en C₅ à C₇, notamment le phénylméthyle (benzyle), le diphénylméthyle, le triphénylméthyle, le 2- phényléthyle, le 3-phénylpropyle, le cyclopentylméthyle, le 2-cyclopentyléthyle, 3- cyclopentylpropyle, le cyclohexylméthyle, 2 à cyclohexyléthyle ou le 3-cyclohexylpropyle; CₙF₂(_{n-a)-(1-b)}H_{2a-b} avec n ≤ 30, 0 ≤ a ≤ n et b = 0 ou 1, notamment CF₃, C₂F₅, C₆F₁₃, C₈F₁₇, C₁₀F₂₁ ou C₁₂F₂₅;
OR^{a}, notamment méthoxy, éthoxy; ou
COR^{a}, notamment le formyle, ou l'acétyle;
un groupe cycloalkyle en C₃ à C₁₂, notamment le cyclopentyle ou le cyclohexyle,;
un groupe cycloalkyle en C₃ à C₁₂ lequel est substitué par un groupe alkyle en C₁ à C₆, notamment le 2-méthyle-1-cyclopentyle, le 3-méthyle-1-cyclopentyle, le 2-méthyle-1- cyclohexyle, le 3-méthyle-1-cyclohexyle ou le 4-méthyle-1-cyclohexyle;
CₙF₂(_{n-a)-(1-b)}H_{2a-b} avec n ≤ 30, 0 ≤ a ≤ n et b = 0 ou 1;
un groupe alkényle en C₂ à C₃₀, notamment le 2-propényle, le 3-butényle, le cis-2-butényle ou le trans-2-butényle;
CₙF₂(_{n-a)-(1-b})H_{2a-b} avec n ≤ 30, O ≤ a ≤ n et b = 0 ou 1;
un groupe cycloalkényle en C₃ à C₁₂, notamment le 3-cyclopentényle, le 2-cyclohexényle, le 3-cyclohexényle ou le 2,5-cyclohexadiényle; CₙF₂(_{n-a)-(1-b)}H_{2a-b} avec n ≤ 30, 0 ≤ a ≤ n et b = 0 ou 1;
un groupe aryle ou hétéoroaryle avec de 2 à 30 atomes de carbone, notamment le phényle, le 1-naphthyle, le 2-naphthyle, le 1-pyrrolyle, le 2-pyrrolyle, le 3-pyrrolyle, le 2-pyridinyle, le 3-pyridinyle ou le 4-pyridinyle;
un groupe aryle ou hétéoroaryle avec de 6 à 30 atomes de carbone, lesquels sont substitués par de un à trois radicaux du groupe comprenant un groupe alkyle en C*₁* à C₆ ou phényle, notamment le 2-méthylphényle (2-tolyle), le 3-méthylphényle (3-tolyle), le 4-méthylphényle, le 2-éthylphényle, le 3-éthylphényle, le 4-éthylphényle, 2,3-diméthylphényle, 2,4-diméthylphényle, 2,5-diméthylphényle, 2,6-diméthylphényle, 3,4-diméthylphényle, 3,5-diméthylphényle ou le 4-phénylphényle; ou
C₆F(₅ₐ₎Hₐ avec 0 ≤ a ≤ 5; ou
deux des radicaux R, R⁴, R⁵, R⁶, R⁷, R⁸, lesquels sont voisins, forment en commun
avec l'atome, auquel ils sont liés un système cyclique à 5 ou 6 éléments ou un système cyclique aromatique à 5 ou 6 éléments, lequel est insubstitué, ou substitué par de 1 à 4 radicaux choisis dans un groupe comprenant un groupe alkyle en C₁ à C₆, un groupe aryle, hétéoroaryle, un groupe cycloalkyle en C*₃* à C*₇*, halogène, OR^{a}, SR^{a}, NR^{a}R ^{b} COOR^{a}, CO-NR^{a}R^{b} ou COR^{a}, R^{a} et R^{b} représentant, indépendamment l'un de l'autre l'hydrogène, un groupe alkyle en C*₁* à C*₆* un groupe alkyle halogéné en C₁ à C₆, un groupe cyclopentyle, cyclohexyle, phényle, tolyle ou benzyle;
de préférence
deux des radicaux R, R⁴, R⁵, R⁶, R⁷, R⁸, lesquels sont voisins, forment en
commun, avec l'atome auquel ils sont liés un système cyclique aromatique à 5 ou 6 éléments, lequel est insubstitué, ou substitué par de 1 à 4 radicaux du groupe alkyle en C₁ à C₆, groupe alkyle, du groupe aryle, hétéoroaryle, de groupe cycloalkyle en C₃ à C₇, halogène, OR^{a} SR^{a}, NR^{a}R^{b}, COOR^{a}, CO-NR^{a}R^{b} ou COR^{a}, R^{a}, R^{b} représentant, indépendamment l'un de l'autre l'hydrogène, un groupe alkyle en C₁ à C₆, un groupe alkyle halogéné en C₁ à C⁶, un groupe cyclopentyle, cyclohexyle, phényle, tolyle ou benzyle;
R représentant l'hydrogène, un groupe alkyle en C₁ à C₃₀ un groupe cycloalkyle en
C₃ à C₁₂, un groupe alkényle en C₂ à C₃₀, un groupe cycloalkényle en C₃ à C₁₂, un groupe aryle ou hétéoroaryle, les 6 derniers radicaux cités pouvant porter un ou plusieurs radicaux halogènes et/ou de 1 à 3 radicaux choisis dans un groupe comprenant un groupe alkyle en C₁ à C₆, un groupe aryle, hétéoroaryle, un groupe cycloalkyle en C₃ à C₇, halogène, ORc, SR^{c}, NR^{c}R^{d}, COR^{c}, COOR^{c}, CO-NR^{c}R^{d}, R^{c} et R^{d} représentant l'hydrogène, un groupe alkyle en C₁ à C₆, un groupe alkyle halogéné en C₁ à C₆, un groupe cyclopentyle, cyclohexyle, phényle, tolyle ou benzyle;
L'hydrogène étant représenté de préférence par;
un groupe alkyle en C₁ à C₃₀, notamment le méthyle, l'éthyle, le 1-propyle, le 2-propyle, le 1-butyle, le 2-butyle, le 2-méthyle-1-propyle (isobutyle), le 2-méthyle-2-propyle (tert.-butyle), le 1-pentyle, le 2-pentyle, le 3-pentyle, le 2-méthyle-1-butyle, le 3-méthyle-1-butyle, le 2-méthyle-2-butyle, le 3-méthyle-2-butyle, le 2,2-diméthyle-1-propyle, le 1-hexyle, le 2-hexyle, le 3-hexyle, le 2-méthyle-1-pentyle, le 3-méthyle-1-pentyle, le 4-méthyle-1-pentyle, le 2-méthyle-2-pentyle, le 3-méthyle-2-pentyle, le 4-méthyle-2-pentyle, le 2-méthyle-3-pentyle, le 3-méthyle-3-pentyle, 2,2-diméthyle-1-butyle, 2,3-diméthyle-1-butyle, 3,3-diméthyle-1-butyle, le 2-éthyle-1-butyle, 2,3-diméthyle-2-butyle, 3,3-diméthyle-2-butyle, l'heptyle, l'octyle, le nonyle, le décyle, l'etécyle, le dodécyle, le tridécyle, le tétradécyle, le pentadécyle, l'hexadécyle, l'heptadécyle, l'octadécyle, le nonadécyle, l'icosyle, l'hénicosyle, le docosyle, le tricosyle, le tétracosyle, le pentacosyle, l'hexacosyle, l'heptacosyle, l'octacosyle, le nonacosyle ou le triacontyle;
un groupe alkyle en C₁ à C₆, lequel est substitué par un groupe phényle ou un groupe cycloalkyle en C₅ à C₇, notamment le phénylméthyle (benzyle), le diphénylméthyle, le triphénylméthyle, le 2- phényléthyle, le 3-phénylpropyle, le cyclopentylméthyle, 2-cyclopentyléthyle, 3-cyclopentylpropyle, le cyclohexylméthyle, le 2- cyclohexyléthyle ou le 3-cyclohexylpropyle;
CₙF₂(_{n-a)-(1-b)}H_{2a-b} avec n ≤ 30, 0 ≤ a ≤ n et b = 0 ou 1, notamment CF₃, C₂F₅, C₆F₁₃, C₈F₁₇, C₁₀F₂₁ ou C₁₂F₂₅;
un groupe cycloalkyle en C₃ à C₁₂, notamment un groupe cyclopentyle ou cyclohexyle;
un groupe cycloalkyle en C₃ à C₁₂, lequel est substitué par un groupe alkyle en C₁ à C₆, notamment le 2-méthyle-1-cyclopentyle, le 3-méthyle-1-cyclopentyle, le 2-méthyle-1-cyclohexyle, le 3-méthyle-1-cyclohexyle ou le 4-méthyle-1-cyclohexyle;
CₙF₂(_{n-a)-(1-b)}H_{2a-b} avec n ≤ 30, 0 ≤ a ≤ n et b = 0 ou 1;
un groupe alkényle en C₂ à C₃₀, notamment le 2-propényle, le 3-butényle, le cis-2-butényle ou le trans-2-butényle;
CₙF₂(_{n-a)-(1-b)}H_{2a-b} avec n ≤ 30, 0 ≤ a ≤ n et b = 0 ou 1;
C₃ à C₁₂ cycloalkényle, notamment le 3-cyclopentényle, le 2-cyclohexényle, le 3-cyclohexényle ou le 2,5-cyclohexadiényle; CₙF₂(_{n-a)-(1-b)}H_{2a-b} avec n ≤ 30, 0 ≤ a ≤ n et b = 0 ou 1;
aryle ou hétéoroaryle avec de 2 à 30 atomes de carbone, notamment phényle, le 1-naphthyle, le 2-naphthyle, le 1-pyrrolyle, le 2-pyrrolyle, le 3-pyrrolyle, le 2-pyridinyle, le 3-pyridinyle ou le 4-pyridinyle;
un groupe aryle ou hétéoroaryle avec de 6 à 30 atomes de carbone, lesquels sont substitués par de un à trois radicaux de groupe alkyle en C₁ à C⁶ ou phényle, notamment le 2-méthylphényle (2-tolyle), le 3-méthylphényle (3-tolyle), le 4-méthylphényle, le 2-éthylphényle, le 3-éthylphényle, le 4-éthylphényle, 2,3-diméthylphényle, 2,4-diméthylphényle, 2,5-diméthylphényle, 2,6-diméthylphényle, 3,4-diméthylphényle, 3,5-diméthylphényle ou le 4-phénylphényle; ou
C₆F₍₅₋ₐ)Hₐ avec 0 ≤ a ≤ 5;
R¹', R²' représentant, indépendamment l'un de l'autre un groupe alkyle en C₁ à
C₃₀, un groupe cycloalkyle en C₃ à C₁₂, un groupe alkényle en C₂ à C₃₀, un groupe cycloalkényle en C₃ à C₁₂, un groupe alinyle en C₂ à C₃₀, un groupe aryle ou hétéoroaryle, les 7 derniers radicaux cités pouvant porter un ou plusieurs radicaux halogènes et/ou de 1 à 3 radicaux choisis dans le groupe alkyle en C₁ à C₆, aryle, hétéoroaryle, cycloalkyle en C₃ à C₇, halogène, OR^{c}, SR^{C}, NR^{C}R^{d}, COR^{c}, COOR^{c}, CO-NR^{c}R^{d}, R^{c} et R^{d} représentant l'hydrogène, un groupe alkyle en C₁ à C₆, un groupe alkyle halogène en C₁ à C₆, un groupe cyclopentyle, cyclohexyle, phényle, tolyle ou benzyle;
de préférence
un groupe alkyle en C₁ à C₃₀, notamment le méthyle, l'éthyle, le 1-propyle, le 2-propyle, le 1-butyle, le 2-butyle, le 2-méthyle-1-propyle (isobutyle), le 2-méthyle-2-propyle (tert.-butyle), le 1-pentyle, le 2-pentyle, le 3-pentyle, le 2-méthyle-1-butyle, le 3-méthyle-1-butyle, le 2-méthyle-2-butyle, le 3-méthyle-2-butyle, 2,2-diméthyle-1-propyle, le 1-hexyle, le 2-hexyle, le 3-hexyle, le 2-méthyle-1-pentyle, le 3-méthyle-1-pentyle, le 4-méthyle-1 -pentyle, le 2-méthyle-2-pentyle, le 3-méthyle-2-pentyle, le 4-méthyle-2-pentyle, le 2-méthyle-3-pentyle, le 3-méthyle-3-pentyle, 2,2-diméthyle-1-butyle, 2,3-diméthyle-1-butyle, 3,3-diméthyle-1-butyle, le 2-éthyle-1-butyle, 2,3-diméthyle-2-butyle, 3,3-diméthyle-2-butyle, l'heptyle, l'octyle, le nonyle, le décyle, l'etécyle, le dodécyle, le tridécyle, le tétradécyle, le pentadécyle, l'hexadécyle, l'heptadécyle, l'octadécyle, le nonadécyle, l'icosyle, l'hénicosyle, le docosyle, le tricosyle, le tétracosyle, le pentacosyle, l'hexacosyle, l'heptacosyle, l'octacosyle, le nonacosyle ou le triacontyle;
un groupe alkyle en C₁ à C₈ lequel est substitué par le phényle ou un groupe cycloalkyle en C₅ à C₇, notamment le phénylméthyle (benzyle), le diphénylméthyle, le triphénylméthyle, le 2- phényléthyle, le 3-phénylpropyle, le cyclopentylméthyle, 2-cyclopentyléthyle, le 3- cyclopentylpropyle, le cyclohexylméthyle, le 2- cyclohexyléthyle ou le 3-cyclohexylpropyle; CₙF₂(_{n-a)-(1-b)}H_{2a-b} avec n ≤ 30, 0 ≤ a ≤ n et b = 0 ou 1, notamment CF₃, C₂F₅, C₆F₁₃, C₈F₁₇, C₈F₂, ou C₁₂F₂₅;
un groupe cycloalkyle en C₃ à C₁₂, notamment le cyclopentyle ou cyclohexyle; un groupe cycloalkyle en C₃ à C₁₂, lequel est substitué par un groupe alkyle en C₁ à C₆, notamment le 2-méthyle-1-cyclopentyle, le 3-méthyle-1-cyclopentyle, le 2-méthyle-1-cyclohexyle, le 3-méthyle-1-cyclohexyle ou le 4-méthyle-1-cyclohexyle;
CₙF₂(_{n-a)-(1-b)}H_{2a-b} avec n ≤ 30, 0 ≤ a ≤ n et b = 0 ou 1;
un groupe alkényle en C₂ à C₃₀, notamment le 2-propényle, le 3-butényle, le cis-2-butényle ou le trans-2-butényle;
CₙF₂(_{n-a)-(1-b)}H_{2a-b} avec n ≤ 30, 0 ≤ a ≤ n et b = 0 ou 1;
un groupe cycloalkényle en C₃ à C₁₂, notamment 3- cyclopentényle, 2-cyclohexényle, 3-cyclohexényle ou 2,5-cyclohexadiényle; CₙF₂(_{n-a)-(1-b)}H_{2a-b} avec n ≤ 30, 0 ≤ a ≤ n et b = 0 ou 1;
un groupe aryle ou hétéoroaryle avec de 2 à 30 atomes de carbone, notamment le phényle, le 1-naphthyle, le 2-naphthyle, le 1-pyrrolyle, le 2-pyrrolyle, le 3-pyrrolyle, le 2-pyridinyle, le 3-pyridinyle ou le 4-pyridinyle;
un groupe aryle ou hétéoroaryle avec de 6 à 30 atomes de carbone, lesquels sont substitués par de un à trois radicaux d'un groupe alkyle en C₁ à C₆ ou d'un groupe phényle, notamment le 2-méthylphényle (2-tolyle), le 3-méthylphényle (3-tolyle), le 4-méthylphényle, le 2-éthylphényle, le 3-éthylphényle, le 4-éthylphényle, le 2,3-diméthylphényle, le 2,4-diméthylphényle, le 2,5-diméthylphényle, le 2,6-diméthylphényle, le 3,4-diméthylphényle, le 3,5-diméthylphényle ou le 4-phénylphényle; ou C₆F₍₅₋ₐ₎Hₐ avec 0 ≤ a ≤ 5; ou
R^{1'} et R^{2'} formant en commun une chaîne -CHR^{a} -CHR^{b}-, une chaîne -CHR^{a}- CH₂-CHR^{b}-- ou un radical -1,2-di-yle-C₆H₄, R^{a} et R^{b} représentant, indépendamment l'un de l'autre l'hydrogène ou un groupe alkyle en C₁ à C₆;
représentant de préférence -CH₂ CH₂-, -CH₂-CH₂-CH₂-, -CH(CH₃)-CH₂-, -CH₂-CH(CH3-, -CH(CH₃)-CH(CH₃) ou -C₆H₄-1,2-diyle-;

5. Procédé selon l'une quelconque des revendications 1 à 4, **caractérisé en ce qu'**on obtient un carbonate quaternaire de la formule I,
R^{1'}, R^{2'} représentant, indépendamment l'un de l'autre un groupe alkyle en C₁ à
C³⁰, un groupe cycloalkyle en C₃ à C₁₂, un groupe alkényle en C₂ à C₃₀-alkényle, un groupe cycloalkényle en C₃ à C₁₂, un groupe alkinyle en C₂ à C₃₀, un groupe aryle ou hétéoroaryle, les 7 derniers radicaux cités pouvant porter un ou plusieurs radicaux halogènes et/ou de 1 à 3 radicaux choisis dans le groupe alkyle de C₁ à C₆, aryle, hétéoroaryle, groupe cycloalkyle de C₃ à C₇, halogène, OR^{c}, SR^{c}, NR^{c}R^{d}, COR^{c}, COOR^{c}, CO-NR^{c}R^{d}, R^{c} et R^{d} représentant l'hydrogène, un groupe alkyle en C₁ à C₆, un groupe alkyle halogène en C₁ à C₆, un groupe cyclopentyle, cyclohexyle, phényle, tolyle ou benzyle ;
représentant de préférence
un groupe alkyle en C₁ à C₃₀, notamment le méthyle, l'éthyle, le 1-propyle, le 2-propyle, le 1-butyle, le 2-butyle, le 2-méthyle-1-propyle (isobutyle), le 2-méthyle-2-propyle (tert.-butyle), 1-pentyle, le 2-pentyle, le 3-pentyle, le 2-méthyle-1-butyle, le 3-méthyle-1-butyle, le 2-méthyle-2-butyle, le 3-méthyle-2-butyle, 2,2-diméthyle-1-propyle, le 1-hexyle, le 2-hexyle, le 3-hexyle, le 2-méthyle-1-pentyle, le 3-méthyle-1 pentyle, le 4-méthyle-1-pentyle, le 2-méthyle-2-pentyle, le 3-méthyle-2-pentyle, le 4-méthyle-2-pentyle, le 2-méthyle-3-pentyle, le 3-méthyle-3-pentyle, 2,2-diméthyle-1-butyle, 2,3-diméthyle-1-butyle, 3,3-diméthyle-1-butyle, le 2-éthyle-1-butyle, 2,3-diméthyle-2-butyle, 3,3-diméthyle-2-butyle, l'heptyle, l'octyle, le nonyle, le décyle, l'etécyle, le dodécyle, le tridécyle, le tétradécyle, le pentadécyle, l'hexadécyle, l'heptadécyle, l'octadécyle, le nonadécyle, l'icosyle, l'hénicosyle, le docosyle, le tricosyle, le tétracosyle, le pentacosyle, l'hexacosyle, l'heptacosyle, l'octacosyle, le nonacosyle ou le triacontyle;
un groupe alkyle de C₁ à C₆, lequel est substitué par un groupe phényle ou un groupe cycloalkyle en C₅ à C₇, notamment le phénylméthyle (benzyle), le diphénylméthyle, le triphénylméthyle, le 2- phényléthyle, le 3-phénylpropyle, le cyclopentylméthyle, le 2-cyclopentyléthyle, le 3-cyclopentylpropyle, le cyclohexylméthyle, le 2-cyclohexyléthyle ou le 3-cyclohexylpropyle;
CₙF₂(_{n-a)-(1-b)}H_{2a-b} avec n ≤ 30, 0 ≤ a ≤ n et b = 0 ou 1, notamment CF₃, C₂F₅, C₆F₁₃, C₈F₁₇, C₁₀F₂₁ ou C₁₂F₂₅;
un groupe cycloalkyle en C₃ à C₁₂, notamment le cyclopentyle ou cyclohexyle;
un groupe cycloalkyle en C₃ à C₁₂, lequel est substitué par un groupe alkyle en C¹ à C₆, notamment le 2-méthyle-1-cyclopentyle, le 3-méthyle-1-cyclopentyle, le 2-méthyle-1-cyclohexyle, le 3-méthyle-1-cyclohexyle ou le 4-méthyle-1-cyclohexyle;
CₙF₂(_{n-a)-(1-b)}H_{2a-b} avec n ≤ 30, 0 ≤ a ≤ n et b = 0 ou 1;
un groupe alkényle en C₂ à C₃₀-, notamment le 2-propényle, le 3-butényle, le cis-2-butényle ou le trans-2-butényle;
CₙF₂(_{n-a)-(1-b)}H_{2a-b} avec n ≤ 30, 0 ≤ a ≤ n et b = 0 ou 1;
Un groupe cycloalkényle en C₃ à C₁₂, notamment le 3-cyclopentényle, le 2-cyclohexényle, le 3-cyclohexényle ou le 2,5-cyclohexadiényle; CₙF₂(_{n-a)-(1-b)}H_{2a-b} avec n ≤ 30, 0 ≤ a ≤ n et b = 0 ou 1;
un groupe aryle ou hétéoroaryle avec de 2 à 30 atomes de carbone, notamment le phényle, le 1 naphthyle, le 2-naphthyle, le 1-pyrrolyle, le 2-pyrrolyle, le 3-pyrrolyle, le 2-pyridinyle, le 3-pyridinyle ou le 4-pyridinyle; un groupe aryle ou hétéoroaryle avec de 6 à 30 atomes de carbone, lesquels sont substitués par de un à trois radicaux du groupe alkyle en C₁ à C₆ ou phényle, notamment le 2-méthylphényle (2-tolyle), le 3-méthylphényle (3-tolyle), le 4-méthylphényle, le 2-éthylphényle, le 3-éthylphényle, le 4-éthylphényle, le 2,3-diméthylphényle, le 2,4-diméthylphényle, le 2,5-diméthylphényle, le 2,6-diméthylphényle, le 3,4-diméthylphényle, le 3,5-diméthylphényle ou le 4-phénylphényle; ou C₆F₍₅₋ₐ₎Hₐ avec 0 ≤ a ≤ 5.

6. Procédé selon l'une quelconque des revendications 1 à 4, **caractérisé en ce qu'**on obtient un carbonate quaternaire de la formule I,
R¹' et R²' formant en commune une chaîne -CHR^{a}-CHR^{b}-, une chaîne -CHR^{a}-CH₂-CHR^{b}- et un radical -1,2-di-yle-C₆H₄, R^{a} et R^{b} représentant,
indépendamment l'un de l'autre l'hydrogène ou un groupe alkyle en C₁ à C₆; de préférence pour -CH₂- CH₂-, -CH₂-CH₂- CH₂-, -CH(CH₃)-CH₂-, -CH₂-CH(CH₃)-, -CH(CH₃)-CH(CH3) ou -C₆H₄-1,2-diyle-;

7. Procédé selon l'une quelconque des revendications 1 à 6, **caractérisé en ce qu'**on obtient un carbonate quaternaire de la formule I,
[A+] étant un cation hétéroaromatique, choisi dans le groupe 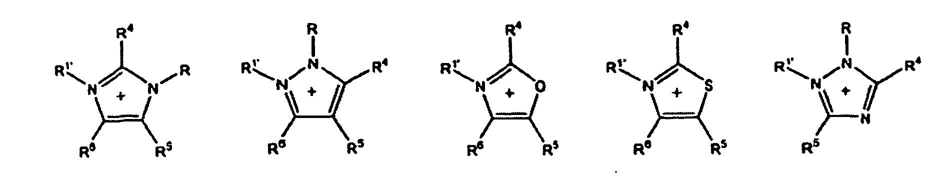 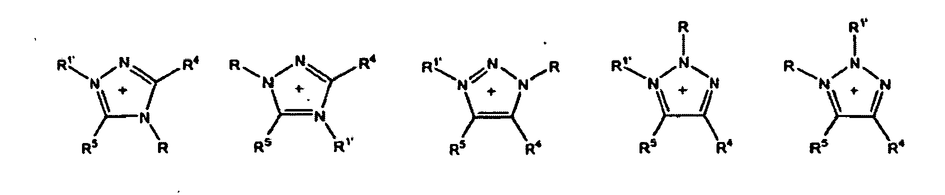 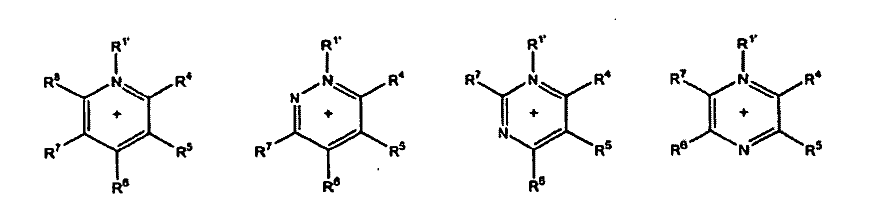 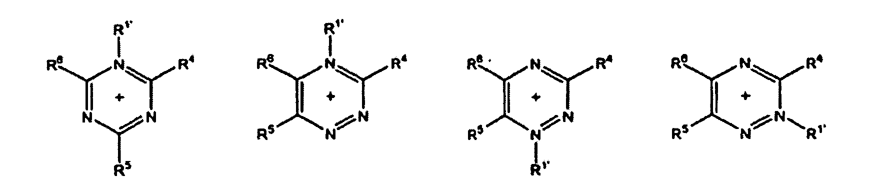 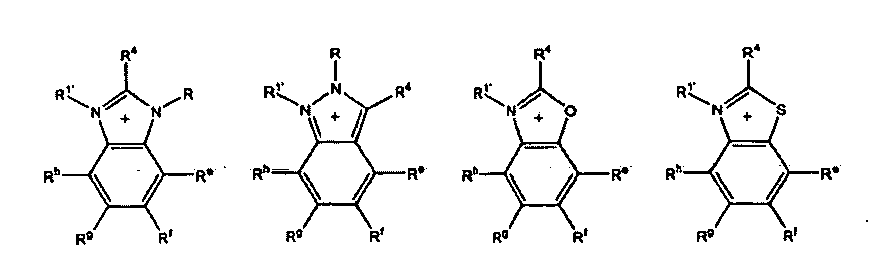 R⁴, R⁵, R⁶, R⁷, R⁸ représentant, indépendamment l'un de l'autre l'hydrogène,
l'halogène, OR^{c}, COR^{c}, un groupe alkyle en C₁ à C₃₀, un groupe cycloalkyle en C₃ à C1₂, un groupe alkényle en C₂ à C₃₀, un groupe cycloalkényle en C³ à C¹², un groupe aryle ou hétéoroaryle, les 6 derniers radicaux cités pouvant porter un ou plusieurs radicaux halogénés et/ou de 1 à 3 radicaux choisis dans le groupe formé par un groupe alkyle en C₁ à C₆, un groupe aryle, hétéoroaryle, un groupe cycloalkyle en C₃ à C₇, halogène, OR^{c}, SR^{c}, NR^{c}R^{d}, COR^{c}, COOR^{c}, CO-NR^{c}R°^{d}, et R^{c} et R^{d} représentant l'hydrogène, un groupe alkyle en C¹ à C₆, un groupe alkyle halogène en C₁ à C₆, un groupe cyclopentyle, cyclohexyle, phényle, tolyle ou benzyle;
l'hydrogène étant représenté de préférence par;
l'halogène,
un groupe alkyle en C₁ à C₃₀, notamment le méthyle, l'éthyle, le 1-propyle, le 2-propyle, le 1-butyle, le 2-butyle, le 2-méthyle-1-propyle (isobutyle), le 2-méthyle-2-propyle (tert.-butyle), le 1-pentyle, le 2-pentyle, le 3-pentyle, le 2-méthyle-1-butyle, le 3-méthyle-1-butyle, le 2-méthyle-2-butyle, le 3-méthyle-2-butyle, 2,2-diméthyle-1-propyle, le 1-hexyle, le 2-hexyle, le 3-hexyle, le 2-méthyle-1-pentyle, le 3-méthyle-1pentyle, le 4-méthyle-1-pentyle, le 2-méthyle-2-pentyle, le 3-méthyle-2-pentyle, le 4-méthyle-2-pentyle, le 2-méthyle-3-pentyle, le 3-méthyle-3-pentyle, le 2,2-diméthyle-1-butyle, le 2,3-diméthyle-1-butyle, le 3,3-diméthyle-1-butyle, le 2-éthyle-1-butyle, le 2,3-diméthyle-2-butyle, le 3,3-diméthyle-2-butyle, l'heptyle, l'octyle, le nonyle, le décyle, l'etécyle, le dodécyle, le tridécyle, le tétradécyle, le pentadécyle, l'hexadécyle, l'heptadécyle, l'octadécyle, le nonadécyle, l'icosyle, l'hénicosyle, le docosyle, le tricosyle, le tétracosyle, le pentacosyle, l'hexacosyle, l'heptacosyle, l'octacosyle, le nonacosyle ou le triacontyle;
un groupe alkyle en C₁ à C₆-groupe alkyle, lequel est substitué par un groupe phényle ou par un groupe cycloalkyle en C₅ à C₇, notamment le phénylméthyle (benzyle), le diphénylméthyle, le triphénylméthyle, le 2-phényléthyle, le 3-phénylpropyle, le cyclopentylméthyle, le 2-cyclopentyléthyle, le 3-cyclopentylpropyle, le cyclohexylméthyle, le 2-cyclohexyléthyle ou le 3-cyclohexylpropyle;
CₙF₂(_{n-a)-(1-b)}H_{2a-b} avec n ≤ 30, 0 ≤ a ≤ n et b = 0 ou 1, notamment CF₃, C₂F₅, C₆F₁₃, C₈F₁₇, C₁₀F₂₁ ou C₁₂F₂₅;
OR^{a}, notamment méthoxy, éthoxy; ou
COR^{a}, notamment le formyle, ou l'acétyle;
un groupe cycloalkyle en C₃ à C₁₂, notamment le cyclopentyle ou
cyclohexyle,;
un groupe cycloalkyle en C₃ à C₁₂, lequel est substitué par un groupe alkyle en C₁ à C₆, notamment le 2-méthyle-1-cyclopentyle, le 3-méthyle-1-cyclopentyle, le 2-méthyle-1-cyclohexyle, le 3-méthyle-1-cyclohexyle ou le 4-méthyle-1-cyclohexyle;
CₙF₂(_{n-a)-(1-b)}H_{2a-b} avec n ≤ 30, 0 ≤ a ≤ n et b = 0 ou 1;
un groupe alkényle en C₂ à C₃₀, notamment le 2-propényle, le 3-butényle, le cis-2-butényle ou le trans-2-butényle;
CₙF₂(_{n-a)-(1-b)}H_{2a-b} avec n ≤ 30, 0 ≤ a ≤ n et b = 0 ou 1;
un groupe cycloalkényle en C₃ à C₁₂, notamment le 3-cyclopentényle, le 2-cyclohexényle, le 3-cyclohexényle ou le 2,5-cyclohexadiényle; CₙF₂(_{n-a)-(1-b)}H_{2a-b} avec n ≤ 30,0 ≤ a ≤ n et b = 0 ou 1;
un groupe aryle ou hétéoroaryle avec de 2 à 30 atomes de carbone, notamment le phényle, le 1-naphthyle, le 2-naphthyle, le 1-pyrrolyle, le 2-pyrrolyle, le 3-pyrrolyle, le 2-pyridinyle, le 3-pyridinyle ou le 4-pyridinyle; un groupe aryle ou hétéoroaryle avec de 6 à 30 atomes de carbone, lesquels sont substitués par de un à trois radicaux d'un groupe alkyle en C₁ à C₆ ou phényle, notamment le 2-méthylphényle (2-tolyle), le 3-méthylphényle (3-tolyle), le 4-méthylphényle, le 2-éthylphényle, le 3-éthylphényle, le 4-éthylphényle, le 2,3-diméthylphényle, le 2,4-diméthylphényle, le 2,5-diméthylphényle, le 2,6-diméthylphényle, le 3,4-diméthylphényle, le 3,5-diméthylphényle ou le 4-phénylphényle; ou C₆F₍₅₋ₐ₎Hₐ avec 0 ≤ a ≤ 5;
R^{e}, R^{f}, R^{g}, R^{h} étant substitués, indépendamment l'un de l'autre pour l'hydrogène, un
groupe alkyle en C₁ à C₆, un groupe aryle, hétéoroaryle, cycloalkyle en C₃ à C₇, halogène, OR^{c}, SR^{c}, NRcR^{d}, COOR^{c}, CO-NR^{c}R^{d} ou COR^{c}, R^{c}, R^{d} représentant, indépendamment l'un de l'autre l'hydrogène, un groupe alkyle en C¹ à C₆, un groupe alkyle halogène en C₁ à C₆, un groupe cyclopentyle, cyclohexyle, phényle, tolyle ou benzyle;
représentant de préférence
l'hydrogène, halogène ou un groupe alkyle en C₁ à C₆, notamment l'hydrogène ou un groupe alkyle en C₁ à C₆;
R représentant l'hydrogène, un groupe alkyle en C₁ à C₃₀, un groupe cycloalkyle
en C₃ à C₁₂, un groupe alkényle en C₂ à C₃₀, un groupe cycloalkényle en C₃ à C₁₂, un groupe aryle ou hétéoroaryle, les 6 derniers radicaux cités pouvant porter un ou plusieurs radicaux halogénés et /ou de 1 à 3 radicaux choisis dans le groupe des alkyles en C₁ à C₆, un groupe, aryle, hétéoroaryle, un groupe cycloalkyle en C₃ à C₇, halogène, OR^{c}, SR^{c}, NR^{c}R^{d}, COR^{c}, COOR^{c}, CO-NR^{c}R^{d}, R^{c} et R^{d} représentant l'hydrogène, un groupe alkyle en C₁ à C₆, un groupe alkyle halogène en C₁ à C₆, un groupe cyclopentyle, cyclohexyle, phényle, tolyle ou benzyle.
représentant de préférence
un groupe alkyle en C₁ à C₃₀, notamment le méthyle, l'éthyle, le 1-propyle, le 2-propyle, le 1-butyle, le 2-butyle, le 2-méthyle-1-propyle (isobutyle), le 2-méthyle-2-propyle (tert.-butyle), le 1-pentyle, le 2-pentyle, le 3-pentyle, le 2-méthyle-1-butyle, le 3-méthyle-1-butyle, le 2-méthyle-2-butyle, le 3-méthyle-2-butyle, le 2,2-diméthyle-1-propyle, le 1-hexyle, le 2-hexyle, le 3-hexyle, le 2-méthyle-1-pentyle, le 3-méthyle-1pentyle, le 4-méthyle-1-pentyle, le 2-méthyle-2-pentyle, le 3-méthyle-2-pentyle, le 4-méthyle-2-pentyle, le 2-méthyle-3-pentyle, le 3-méthyle-3-pentyle, le 2,2-diméthyle-1-butyle, le 2,3-diméthyle-1-butyle, 3le 3-diméthyle-1-butyle, le 2-éthyle-1-butyle, le 2,3-diméthyle-2-butyle, le 3,3-diméthyle-2-butyle, l'heptyle, l'octyle, le nonyle, le décyle, l'etécyle, le dodécyle, le tridécyle, le tétradécyle, le pentadécyle, l'hexadécyle, l'heptadécyle, l'octadécyle, le nonadécyle, l'icosyle, l'hénicosyle, le docosyle, le tricosyle, le tétracosyle, le pentacosyle, l'hexacosyle, l'heptacosyle, l'octacosyle, le nonacosyle ou le triacontyle;
un groupe alkyle en C₁ à C₆, lequel est substitué par un groupe phényle ou par un groupe cycloalkyle en C₅ à C₇, notamment le phénylméthyle (benzyle), le diphénylméthyle, le triphénylméthyle, le 2- phényléthyle, le 3-phénylpropyle, le cyclopentylméthyle, le 2-cyclopentyléthyle, le 3-cyclopentylpropyle, le cyclohexylméthyle, le 2- cyclohexyléthyle ou le 3-cyclohexylpropyle;
CₙF₂(_{n-a)-(1-b)}H_{2a-b} avec n ≤ 30, 0 ≤ a ≤ n et b = 0 ou 1, notamment CF₃, C₂F₅, C₆F₁₃, C₈F₁₇, C₁₀F₂₁ ou C₁₂F₂₅;
un groupe cycloalkyle en C₃ à C₁₂, notamment un groupe cyclopentyle ou cyclohexyle;
un groupe cycloalkyle en C₃ à C₁₂, lequel est substitué par un groupe alkyle en C₁ à C₆, notamment le 2-méthyle-1-cyclopentyle, le 3-méthyle-1-cyclopentyle, le 2-méthyle-1-cyclohexyle, le 3-méthyle-1-cyclohexyle ou le 4-méthyle-1-cyclohexyle;
CₙF₂(_{n-a)-(1-b)}H_{2a-b} avec n ≤ 30, 0 ≤ a ≤ n et b = 0 ou 1;
un groupe alkényle en C₂ à C₃₀, notamment le 2-propényle, le 3-butényle, le cis-2-butényle ou le trans-2-butényle; CₙF₂(_{n-a)-(1-b)}H_{2a-b} avec n ≤ 30, 0 ≤ a ≤ n et b = 0 ou 1;
un groupe cycloalkényle en C₃ à C₁₂, notamment le 3-cyclopentényle, le 2-cyclohexényle, le 3-cyclohexényle ou le 2,5-cyclohexadiényle;
CₙF₂(_{n-a)-(1-b)}H_{2a-b} avec n ≤ 30, 0 ≤ a ≤ n et b = 0 ou 1;
un groupe aryle ou hétéoroaryle avec de 2 à 30 atomes de carbone, notamment un groupe phényle, le 1-naphthyle, le 2-naphthyle, le 1-pyrrolyle, le 2-pyrrolyle, le 3-pyrrolyle, le 2-pyridinyle, le 3-pyridinyle ou le 4-pyridinyle;
un groupe aryle ou hétéoroaryle avec de 6 à 30 atomes de carbone, lesquels sont substitués par de un à trois radicaux de groupe alkyle en C₁ à C₆ ou phényle, notamment le 2-méthylphényle (2-tolyle), le 3-méthylphényle (3-tolyle), le 4-méthylphényle, le 2-éthylphényle, le 3-éthylphényle, le 4-éthylphényle, le 2,3-diméthylphényle, le 2,4-diméthylphényle, le 2,5-diméthylphényle, le 2,6-diméthylphényle, le 3,4-diméthylphényle, le 3,5-diméthylphényle ou le 4-phénylphényle; ou C₆F₍₅₋ₐ₎Hₐ avec 0 ≤ a ≤ 5.

8. Procédé selon la revendication 7, **caractérisé en ce qu'**on obtient un carbonate quaternaire de la formule I,
R⁴, R⁵, R^{e}, R⁷, R⁶, R^{e}, R^{f}, R^{g}, R^{h} représentant, indépendamment l'un de l'autre
l'hydrogène, halogène ou un groupe alkyle en C₁ à C₆, représentant de préférence l'hydrogène, halogène ou un groupe alkyle en C₁ à C₆ ;
R représentant un groupe alkyle en C₁ à C₆.

9. Procédé selon l'une quelconque des revendications 1 à 6 ou 8, **caractérisé en ce qu'**on obtient un carbonate quaternaire de la formule I,
[A⁺] étant un cation d'ammonium quaternaire [R^{1'}R¹R²R³N]⁺ ou un cation de phosphonium [R^{1'}R¹R²R³P]⁺.

10. Procédé selon l'une quelconque des revendications 1 à 9, **caractérisé en ce qu'**on met en oeuvre un acide de Lewis, lequel contient au moins un élément issu du 1^{er} au 13^{ème} groupe du système périodique ou du groupe des lanthanides (atomes n° 57 à 71), de préférence un acide de Lewis contenant au moins un élément choisi dans le groupe lithium, titane, zircon, vanadium, niobate, tantale, chrome, manganèse, fer, cobalt, nickel, cuivre, argent, or, aluminium, cérium, lanthane, néodyme, samarium, gadolinium, erbium et lutétium, notamment du groupe comprenant le lithium, le cuivre, l'argent, le samarium, le cérium, le titane et l'aluminium.

11. Procédé selon la revendication 10, **caractérisé en ce qu'**on met en oeuvre, en tant qu'acide de Lewis au moins un oxyde, carbonate, halogénure ou silicate de lithium, de cuivre, d'argent, de samarium, de cérium, de titane o d'aluminium ou des mélanges et ces derniers et un zéolithe.

12. Procédé selon la revendication 10 ou 11, **caractérisé en ce qu'**on met en oeuvre l'acide de Lewis à raison de 0,001 % en moles à 90 % en moles, de préférence de 0,1 % en moles à 10 % en moles, de façon particulièrement préférée de 0,5 % en moles à 5 % en moles par rapport à l'amine R¹R²R³N de la formule IIa, la phosphine R¹R²R³P de la formule IIb, le sulfure R₁R²S de la formule IIc ou les hétéroaromes de la formule IId.

13. Procédé selon l'une quelconque des revendications 1 à 12, **caractérisé en ce qu'**on met en oeuvre le carbonate d'alkyle et/ou le carbonate d'aryle de la formule III dans une proportion de 0,5 à 10 équivalents molaires, de préférence de 1,0 à 5,0 équivalents molaires, de façon particulièrement préférée de 1,1 à 2,0 équivalents molaires, par rapport à l'amine R¹R²R³N de la formule IIa, à la phosphine R¹R²R³P de la formule IIb, au sulfure R¹R²S de la formule IIc ou aux hétéroaromes de la formule IId.

14. Procédé selon l'une quelconque des revendications 1 à 13, **caractérisé en ce qu'**on procède à la réaction à une température de 30°C à 350°C, de préférence de 50°C à 150°C, de façon particulièrement préférée de 80°C à 120°C.

15. Procédé selon l'une quelconque des revendications 1 à 14, **caractérisé en ce que** le temps de réaction est de l'ordre de 0,1 heure à 14 jours, de préférence de 0,5 heures à 5 jours, de façon particulièrement préférée de 1 heures 24 heures.

16. Procédé selon l'une quelconque des revendications 1 à 15, **caractérisé en ce qu'**on procède à la transformation en présence d'un agent de réduction, de préférence d'un hydrure d'aluminium, d'un hydrure de bore ou en présence d'hydrogène et d'un catalyseur choisi dans le groupe des métaux du groupe du platine; notamment le NaAlH₄, le LiAlH₄, le NaBH₄, le LiBH₄ ou le H₂ en présence d'un catalyseur du groupe des platines, comme le Pt, le Pd, le Rh, le Ru ou l'Ir

17. Procédé selon l'une quelconque des revendications 1 à 16, **caractérisé en ce qu'**on procède à la transformation sous air atmosphérique ou sous atmosphère de vapeur d'eau ou sous une atmosphère de gaz inerte, notamment sous azote ou argon.

18. Procédé selon l'une quelconque des revendications 1 à 17, **caractérisé en ce qu'**on procède à la transformation, soit par lot, en mode semi-continu ou continu.

19. Procédé selon l'une quelconque des revendications 1 à 18, **caractérisé en ce qu'**on procède à la transformation dans une substance ou en présence d'un solvant.

20. Procédé selon la revendication 19, **caractérisé en ce qu'**on procède à la transformation dans une substance.

21. Procédé selon la revendication 19, **caractérisé en ce qu'**on procède à la transformation en présent d'un solvant, de préférence d'un alcool, d'un éther, d'un formamide de dialkyle, de cétone, d'un oxyde sulfonique, de nitrile ou d'un mélange de ces derniers, notamment d'un alcool, de préférence de méthanol, d'éthanol, d'isopropanol, de n-propanol, de n-butanol, de sec-butanol, de tert-butanol, de pentanol, d'hexanol, d'heptanol, d'octanol ou de phénol ou d'un mélange de ces derniers, la part de solvant se situant à de 1 à 90%, de préférence à de 10 à 50%, de façon préférée à de 20 à 30%, par rapport à la masse totale du mélange réactif.

22. Procédé selon la revendication 21, **caractérisé en ce qu'**on met en oeuvre en tant que solvant l'alcool lequel correspond au moins à l'un des radicaux du carbonate d'alkyle et/o d'aryle de la formule III.

23. Procédé selon l'une quelconque des revendications 1 à 22, **caractérisé en ce qu'**on malaxe, qu'on agite ou qu'on mélange intimement le mélange réactif d'une manière quelconque.

24. Procédé selon les revendications 1 à 23, **caractérisé en ce qu'**on obtient le carbonate d'alkyle et/ou d'aryle de la formule III par transformation de CO₂, de CO ou de CO/O₂ ou d'un mélange de ces derniers en présence d'un acide de Lewis, à une pression de 1 à 300 bar, de préférence de 5 à 100 bar, de façon particulièrement préférée de 10 à 50 bar.

25. Procédé selon la revendication 24, **caractérisé en ce qu'**on créé le carbonate d'alkyle ou d'aryle de la formule III en conditions in situ.

26. Procédé d'obtention de carbonates quaternaires de la formule I, selon la description figurant dans les revendications 1 à 9, **caractérisé en ce qu'**on transforme une amine R¹R²R³N de la formule IIa, la phosphine R¹R²R³P de la formule IIb, le sulfure R'R²S de la formule IIc ou l'hétéroarome de la formule IId avec un alcool R^{1'}OH ou R^{2'}OH ou un mélange de ces derniers et du CO₂, du CO/O₂ ou du CO en association avec un oxydant, ou des mélanges de ces derniers, en présence d'au moins un acide de Lewis à une pression de 1 à 300 bar, de préférence de 5 à 100 bar, de façon particulièrement préférée de 10 à 50 bar.

27. Procédé selon l'une quelconque des revendications 24 à 26, **caractérisé en ce qu'**on met en oeuvre l'acide de Lewis dans un proportion de 0,001 % en moles à 90 % en moles, de préférence de 0,1 % en moles à 10 % en moles, de façon particulièrement préférée de 0,5 % en moles à 5 % en moles, par rapport à l'amine R¹R²R³N de la formule IIa, à la phosphine R¹R²R³P de la formule IIb, au sulfure R¹R²S de la formule IIc ou aux hétéroaromes de la formule IId.

28. Procédé selon l'une quelconque des revendications 24 à 27 , **caractérisé en ce qu'**on met en oeuvre l'alcool R^{1'}OH ou R^{2'}OH, ou un mélange de ces derniers dans une proportion 0,5 à 20 équivalents molaires, de préférence de 1,0 à 5 équivalents molaires, de façon particulièrement préférée de 1,5 à 2,5 équivalents molaires, par rapport à l'amine R'R²R³N de la formule IIa, à la phosphine R¹R²R³P de la formule IIb, du sulfure R¹R²S de la formule IIc ou de l'hétéroarome de la formule IId.

29. Procédé selon l'une quelconque des revendications 24 à 28, **caractérisé en ce qu'**on génère de dioxyde de carbone nécessaire pour produire le carbonate d'alkyle et/ou d'aryle de la formule III par transformation d'un carbonate quaternaire de la formule I et/ou d'un composé quaternaire de la formule IVa et/ou IVb 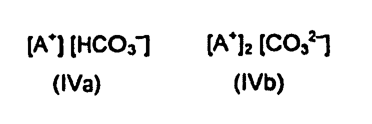 [A⁺] ayant la signification citée dans les revendications 1 à 27 avec un acide HZ de Bronsted.

30. Procédé selon l'une quelconque des revendications 24 à 28, **caractérisé en ce qu'**on génère l'alcool R^{2'}OH nécessaire pour la production du carbonate d'alkyle et/ou d'aryle de la formule III par la transformation d'un carbonate quaternaire de la formule I avec un acide HZ de Bronsted.

31. Procédé selon l'une quelconque des revendications 24 à 30, **caractérisé en ce que** l'acide de Lewis pour l'alkylation ou arylation de l'amine R¹R²R³N de la formule IIa, de la phosphine R'R²R³P de la formule IIb, du sulfure R¹R²S de la formule IIc ou de l'hétéroarome de la formule IId avec le carbonate d'alkyle ou d'aryle de la formule III est identique à l'acide de Lewis pour la production in situ du carbonate d'alkyle et/ou d'aryle de la formule III.

32. Procédé selon l'une quelconque des revendications 1 à 31, **caractérisé en ce que** la réaction se déroule en phase liquide ou en phase gazeuse.

33. Procédé selon l'une quelconque des revendications 1 à 32, **caractérisé en ce que** l'utilisation à d'autres fins des mélanges réactifs s'effectue par distillation, par distillation sous vide, par évaporation à couche mince, par évaporation à court trajet, par évaporation sous rotation, par séchage par pulvérisation, par osmose, par pervaporation, par stripage au gaz ou à la vapeur d'eau, par séparation par congélation, par lyophilisation, par adsorption chimique ou physique ou par d'autres procédés adaptés.

34. Procédé de production d'un composé de la formule V,
[A⁺]ₙ [Zⁿ⁻] (V)
[A⁺] ayant la signification citée dans les revendications 1 à 33, et
[Zⁿ⁻] représentant un anion monovalent, bivalent, trivalent ou quadrivalent; **caractérisé en ce qu'**on produit un carbonate quaternaire de la formule I selon l'un quelconque des procédés 1 à 33 et **en ce qu'**on transforme le carbonate ainsi obtenu de la formule 1 avec un acide de Bronsted [H⁺]ₙ[Zⁿ⁻], dont l'anhydride ou un sel transforme [cation ^{m+}]ₙ[Zⁿ⁻]ₘ.
